# EUROPEAN PATENT APPLICATION

(11) **EP 3 045 180 A1**
(43) Date of publication of application: **20.07.2016**
(21) Application number: 14844286.6
(22) Date of filing: 15.09.2014
(51) Int. Cl.: A61K 38/17, C07K 16/18, A61K 39/395, A61P 25/16

(54) **COMBINATIONS OF AGGREGATING PROTEINS AND MOLECULAR CHAPERONE PROTEINS FOR THE TREATMENT OF PROTEINOPATHIES OR CONFORMATIONAL DISEASES**

(30) Priority: 13.09.2013 ES 201331334
(71) Applicant: Fundación Pública Andaluza Progreso Y Salud, 41092 Sevilla (ES); Universidad de Sevilla, 41013 Sevilla (ES)
(72) Inventor: ROODVELDT CATELLANI, Cintia, E-41092 Sevilla (ES); LABRADOR GARRIDO, Adahir, E-41092 Sevilla (ES); POZO PÉREZ, David, E-41092 Sevilla (ES)
(74) Representative: Fuster, Gustavo
(86) International application number: PCT/ES2014/070704
(87) International publication number: WO 2015/036646

(57) **Abstract**

HSPs (chaperones or heat-shock proteins) have two very valuable characteristics that can be productively exploited in a specific manner in order to "intelligently" manipulate the immune response so as to treat "conformational" disorders or "proteinopathies":
- the classic chaperone functions thereof; and
- the more recently discovered immunoactive properties thereof.

On the basis thereof, the authors of the present invention have combined a peptide, a protein or a polypeptide associated with a "conformational disease" with a biologically active or functional HSP, resulting in a different immunoactive complex which is advantageous for treating the "conformational disease" in question associated with the peptide, protein or polypeptide used.

## Description

The present invention is comprised within the field of medicine, molecular biology and pharmacy, and relates specifically to a vaccine comprising an aggregating peptide or polypeptide and a molecular chaperone in the preparation of a medicinal product or vaccine for the treatment and/or prevention of an aggregating protein-related disease. It also relates to the nucleotide sequences, expression vectors, host cells, antibodies or immunotherapeutic cellular composition related with the composition of the invention, as well as to a method for the diagnosis of aggregating protein-related diseases by means of using the antibodies of the invention.

### PRIOR STATE OF THE ART

Parkinson's disease (PD) is the second most common neurodegenerative disease after Alzheimer's disease, with a higher and increasing prevalence in industrialized countries. This disorder and other proteinopathies or conformational diseases are characterized by the poor folding and aggregation of specific proteins in highly ordered fibrillary structures that accumulate and give rise to the formation of protein deposits in certain cells and areas of the brain (Knowles TP et al. 2014. Nat Rev Mol Cell Biol. 15:384-96.). PD is pathologically characterized by the presence of α-synuclein (αSyn) aggregate deposits in intracellular inclusions known as Lewy bodies (LBs) in the substantia nigra of the brain and by the loss of dopamine-producing neurons. Despite the fact that the causes and the underlying pathogenic mechanism have not yet been completely clarified, it has been established that αSyn plays a fundamental role in the onset and progression of the disease (Marques & Outeiro, 2012. Cell Death Dis. 3: e350). Today, there is no effective treatment against this devastating disease the normal process of which ultimately leads to major loss of neuron cells and dementia.

The αSyn protein, the physiological functions of which are unknown today, is expressed fundamentally in neurons so it is mainly found in the brain. Despite the fact that αSyn has been conventionally considered as an exclusively intracellular protein, evidence showing the presence of αSyn, both in the aggregated and non-aggregated form, in the cerebrospinal fluid (CSF) and blood has been amassed. In fact, it has been found that disequilibrium in the oligomer content or αSyn total protein content in the CSF are associated with PD, amyotrophic lateral sclerosis (ALS), Lewy body dementia (LBD) and Alzheimer's disease (AD). Furthermore, there is new evidence suggesting that these species of aberrant proteins can be transferred from cell to cell and promote the propagation of neurodegeneration in a manner similar to prion. While it has been known for some time that robust, activated microglia-mediated neuroinflammation accompanies the neurodegenerative process in PD, recent studies indicate that extracellular αSyn, particularly the oligomeric forms, plays a key role in inflammatory response, as well as in other neurodegeneration-linked neurotoxic mechanisms.

Amyotrophic lateral sclerosis (ALS) is an incurable neurodegenerative disease that leads to death in a period of between 3 and 5 years after diagnosis. Today, there are no effective treatments for ALS, obtaining preclinical data that support clinical studies for establishing therapies modifying the progression of the disease being a priority for health systems. Today, there is only one approved treatment (Riluzole) for ALS which only achieves rather moderate effects prolonging the survival of the patients between 2-3 months. Of all ALS cases, 90% are sporadic and 10% are familial. Although motor neuron death is responsible for this disease, it has been proven in the last three years that ALS is not a phenomenon exclusively linked to motor neurons since various cell types, among which microglia activation, T-cell infiltration, and acquired immune deficiency response stand out, also influence this disease.

Today, it is known that 20% of familial ALS cases are associated with mutations in the gene encoding the Cu-Zn superoxide dismutase 1 (SOD1) enzyme, one of the main components of the cell defense mechanism against oxidative damage. Furthermore, it is believed that intracellular aggregation and accumulation of SOD1 in motor neurons are crucial for the onset and progression of ALS, although the underlining pathological mechanisms are still unknown.

In recent years, vaccination or immunization has emerged as a potentially powerful therapeutic strategy for the treatment of some neurodegenerative proteinopathies. Despite the fact that some advancements have been made for Alzheimer's disease (AD), this method has been largely unexplored for other diseases such as PD and ALS, today there being only one vaccine under phase I study for PD and none for ALS or other 'conformational diseases'. In fact, the antibodies produced by immunization with αSyn in a mouse model of PD and passive vaccination with anti- αSyn antibodies in a mouse model of Lewy body disease or α-synuclein transgenic mice have shown encouraging, although modest, results. On the other hand, it has been demonstrated that immunization with highly aggregated α-Syn species in mice with PD aggravates neuroinflammation and neurodegeneration through a dysfunction in regulatory T-cells (Treg) which, as is known, are instrumental in modulating immune response. Generally, although immunization with α-synuclein is currently rated as a very appealing treatment option for PD and other synucleinopathies, current immunotherapy protocols have demonstrated up until now mixed/modestly successful results, and it is clear that new approaches for optimizing vaccination schedules are necessary.

### BRIEF DESCRIPTION OF THE INVENTION

HSPs (chaperones or heat-shock proteins) have two very valuable characteristics that can be productively exploited in a specific manner in order to "intelligently" manipulate the immune response so as to treat 'conformational' disorders or 'proteinopathies':
- the classic chaperone functions thereof; and
- the more recently discovered immunoactive properties thereof.

On the basis thereof, the authors of the present invention have combined a peptide, a protein or a polypeptide associated with a 'conformational disease' with a biologically active or functional HSP, resulting in a different immunoactive complex which is advantageous for treating the "conformational disease" in question associated with the peptide, protein or polypeptide used.

Therefore, a first aspect of the invention relates to a composition, hereinafter composition of the invention, comprising:
a) a mixture of peptides/polypeptides/proteins comprising i) at least one peptide/polypeptide/protein the amino acid sequence of which corresponds with an aggregating peptide/polypeptide/protein (hereinafter "sequence A of the invention") or any of the biologically active variants and fragments of said sequence, and ii) a peptide/polypeptide/protein the amino acid sequence of which corresponds with a molecular chaperone (hereinafter "sequence B of the invention") or with any of the biologically active variants and fragments of said sequence; and/or
b) a fusion peptide/polypeptide/protein, hereinafter "fusion amino acid sequence of the invention", comprising the amino acid sequences of at least one aggregating peptide/polypeptide/protein ("sequence A of the invention") or any of the biologically active variants and fragments thereof, and of a peptide/polypeptide/protein the amino acid sequence of which corresponds with a chaperone ("sequence B of the invention") or with any of the biologically active variants and fragments thereof.

In a preferred embodiment of this aspect of the invention, at least one of the sequences A and/or B are recombinant sequences. In another preferred embodiment, sequence A corresponds with the amino acid sequence of α-synuclein or with any of the biologically active variants and fragments thereof. In another preferred embodiment, sequence B corresponds with the amino acid sequence of the chaperone Grp94 or with any of the biologically active variants and fragments thereof. In another even more preferred embodiment, sequence A corresponds with the amino acid sequence of α-synuclein or with any of the biologically active variants and fragments thereof, and sequence B corresponds with the amino acid sequence of the chaperone Grp94 or with any of the biologically active variants and fragments thereof.

In another preferred embodiment, sequence A corresponds with the amino acid sequence of SOD1 or with any of the biologically active variants and fragments thereof. In another preferred embodiment, peptide B corresponds with the amino acid sequence of the chaperone Hsp70 or with any of the biologically active variants and fragments thereof. In another even more preferred embodiment, sequence A corresponds with the amino acid sequence of SOD1 or with any of the biologically active variants and fragments thereof, and peptide B corresponds with the amino acid sequence of the chaperone Hsp70 or with any of the biologically active variants and fragments thereof

Another aspect of the invention relates to an isolated nucleotide sequence, hereinafter "nucleotide sequence of the invention", comprising one or more nucleotide sequences encoding a whole fusion protein of the invention or a fraction thereof.

Another aspect of the invention relates to an isolated expression vector (hereinafter "expression vector of the invention"), comprising the nucleotide sequence of the invention.

Another aspect of the invention relates to an isolated host cell (hereinafter "host cell of the invention"), comprising an expression vector of the invention or a nucleotide sequence of the invention.

Another aspect of the invention relates to an antibody or a fragment thereof, hereinafter "antibody of the invention" or "antibody fragment of the invention", capable of binding to one of sequences A or B of the invention or to the fusion amino acid sequence of the invention. In a preferred embodiment of this aspect, the antibody or the antibody fragment of the invention is obtained after immunization of an animal (in the presence or absence of an adjuvant) or the cells of an animal with a sequence A and B of the invention or with the fusion amino acid sequence of the invention. In a more preferred embodiment, the mammal is human.

Another aspect of the invention relates to an immunotherapeutic cellular composition comprising at least one isolated, activated antigen presenting cell (APC), in which said APC is obtained from a patient diagnosed with a disease caused by an aggregating protein of the invention, and wherein said APC is stimulated by *ex vivo* exposure to a composition comprising sequence A and sequence B of the invention, or the fusion amino acid sequence of the invention or any of the biologically active variants and fragments thereof.

Another aspect of the invention relates to a pharmaceutical composition, hereinafter "pharmaceutical composition of the invention", comprising the composition of the invention, the nucleotide sequence of the invention, the expression vector of the invention, the host cell of the invention, the antibody or a fragment of the antibody of the invention and/or the immunotherapeutic cellular composition of the invention. In a preferred embodiment of this aspect, the pharmaceutical composition further comprises a pharmaceutically acceptable vehicle. In another preferred embodiment, the pharmaceutical composition further comprises an adjuvant.

Another aspect of the invention relates to a combined preparation, hereinafter "combined preparation of the invention", comprising sequence A of the invention and sequence B of the invention.

Another aspect of the invention relates to the use of sequence B of the invention, the composition of the invention, the nucleotide sequence of the invention, the expression vector of the invention, the host cell of the invention, the pharmaceutical composition of the invention, the combined preparation of the invention, or the antibody of the invention, in the preparation of a medicinal product. In a preferred embodiment, the medicinal product is a vaccine.

Another aspect of the invention relates to the use of sequence B of the invention, the composition of the invention, the nucleotide sequence of the invention, the expression vector of the invention, the host cell of the invention, the antibody or a fragment of the antibody of the invention, the immunotherapeutic cellular composition of the invention, the pharmaceutical composition of the invention, or the combined preparation of the invention, in the preparation of a medicinal product for the treatment or prevention of aggregating protein-related diseases or disorders. In a preferred embodiment of this aspect, the aggregating protein-related disease or disorder is selected from the list consisting of: Parkinson's disease (PD), Lewy body dementia (LBD), the Lewy body variant of Alzheimer's disease, multiple system atrophy (MSA), Alzheimer's disease, pure autonomic failure (PAF), amyotrophic lateral sclerosis (ALS), frontotemporal dementia, Gaucher disease, Down syndrome, Huntington's disease, prion disease, Creutzfeldt-Jakob disease and other transmissible spongiform encephalopathies, multiple sclerosis, Gerstmann-Straussler-Scheinker syndrome, Kuru, fatal familial insomnia, type II diabetes, cerebrovascular amyloidosis, glaucoma, age-related macular degeneration, psychiatric syndromes, schizophrenia and/or schizophrenia-like disorders, and neurodegeneration due to age-related protein aggregation. More preferably, it is Parkinson's disease (PD) and amyotrophic lateral sclerosis (ALS).

Another aspect of the invention relates to the use of sequences A and B of the invention, the composition of the invention, the nucleotide sequence of the invention, the expression vector of the invention, the host cell of the invention, the pharmaceutical composition of the invention, the combined preparation of the invention, or the antibody of the invention, in the preparation of a medicinal product. In a preferred embodiment, the medicinal product is a vaccine.

Another aspect of the invention relates to the use of sequences A and B of the invention, the composition of the invention, the nucleotide sequence of the invention, the expression vector of the invention, the host cell of the invention, the antibody or a fragment of the antibody of the invention, the immunotherapeutic cellular composition of the invention, the pharmaceutical composition of the invention, or the combined preparation of the invention, in the preparation of a medicinal product for the treatment or prevention of aggregating protein-related diseases or disorders. Preferably, the aggregating protein is α-synuclein. In a preferred embodiment of this aspect, the α-synuclein-related disease or disorder or the disease or disorder related with another aggregating protein is selected from the list consisting of: Parkinson's disease (PD), Lewy body dementia (LBD), the Lewy body variant of Alzheimer's disease, multiple system atrophy (MSA), Alzheimer's disease, pure autonomic failure (PAF), amyotrophic lateral sclerosis (ALS), frontotemporal dementia, Gaucher disease, Down syndrome, Huntington's disease, prion disease, Creutzfeldt-Jakob disease and other transmissible spongiform encephalopathies, multiple sclerosis, Gerstmann-Straussler-Scheinker syndrome, Kuru, fatal familial insomnia, type II diabetes, cerebrovascular amyloidosis, glaucoma, age-related macular degeneration, psychiatric syndromes, schizophrenia and/or schizophrenia-like disorders, and neurodegeneration due to age-related protein aggregation. More preferably, it is Parkinson's disease (PD) and amyotrophic lateral sclerosis (ALS).

In another preferred embodiment, the α-synuclein-related disease or disorder is selected from the list consisting of: Parkinson's disease (PD), Lewy body dementia, multiple system atrophy (MSA), Gaucher disease, pure autonomic failure (PAF) and Alzheimer's disease.

Another even more preferred embodiment relates to the use of a composition of the invention comprising as sequence A, the amino acid sequence of α-synuclein, and as sequence B, the amino acid sequence of the chaperone Grp94, for the preparation of a medicinal product for the treatment or prevention of an α-synuclein-related disease or disorder. In a preferred embodiment, the α-synuclein-related disease or disorder is selected from the list consisting of: Parkinson's disease (PD), Lewy body dementia, multiple system atrophy (MSA), Gaucher disease, pure autonomic failure (PAF) and Alzheimer's disease.

Another even more preferred embodiment relates to the use of a composition of the invention comprising as sequence A, the amino acid sequence of α-synuclein, and as sequence B, the amino acid sequence of the chaperone Grp94.

Another aspect of the invention relates to the use of the combined preparation of the invention, comprising sequence A and sequence B of the invention, in the preparation of a medicinal product for combined, simultaneous or sequential administration, for the treatment or prevention of aggregating protein-related diseases or disorders. More preferably, the aggregating protein is α-synuclein or α-synucleinopathies. In a preferred embodiment of this aspect, the α-synuclein-related disease or disorder is selected from the list consisting of: Parkinson's disease (PD), Lewy body dementia (LBD), the Lewy body variant of Alzheimer's disease, multiple system atrophy (MSA), Alzheimer's disease, amyotrophic lateral sclerosis (ALS), Down syndrome, frontotemporal dementia, Gaucher disease, Huntington's disease, prion disease, Creutzfeldt-Jakob disease and other transmissible spongiform encephalopathies, multiple sclerosis, Gerstmann-Straussler-Scheinker syndrome, Kuru, fatal familial insomnia, type II diabetes, cerebrovascular amyloidosis, glaucoma, age-related macular degeneration, psychiatric syndromes, schizophrenia and/or schizophrenia-like disorders, and neurodegeneration due to age-related protein aggregation. More preferably, it is Parkinson's disease.

Another even more preferred embodiment relates to the use of the combined preparation of the invention comprising as sequence A, the amino acid sequence of α-synuclein, and as sequence B, the amino acid sequence of the chaperone Grp94, for the preparation of a medicinal product for the treatment or prevention of an α-synuclein-related disease or disorder. In a preferred embodiment, the α-synuclein-related disease or disorder is selected from the list consisting of: Parkinson's disease (PD), Lewy body dementia (LBD), the Lewy body variant of Alzheimer's disease, multiple system atrophy (MSA), Alzheimer's disease, amyotrophic lateral sclerosis (ALS), frontotemporal dementia, Gaucher disease, Down syndrome, Huntington's disease, prion disease, Creutzfeldt-Jakob disease and other transmissible spongiform encephalopathies, multiple sclerosis, Gerstmann-Straussler-Scheinker syndrome, Kuru, fatal familial insomnia, type II diabetes, cerebrovascular amyloidosis, glaucoma, age-related macular degeneration, psychiatric syndromes, schizophrenia and/or schizophrenia-like disorders, and neurodegeneration due to age-related protein aggregation. More preferably, it is Parkinson's disease (PD) and amyotrophic lateral sclerosis (ALS).

### DESCRIPTION OF THE DRAWINGS

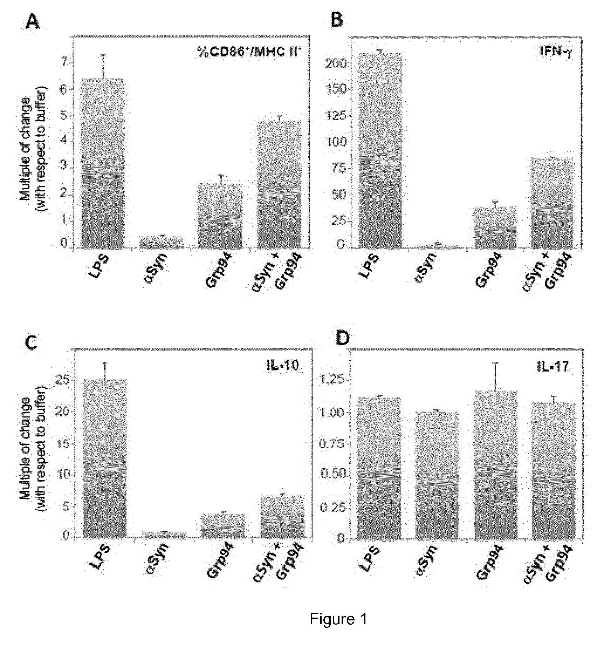

Figure 1 shows the evaluation of the effect on murine splenocytes stimulated *in vitro* with an αSyn and Grp94 combination. A. Activation profile flow cytometry analysis of murine splenocytes in cultures originating from healthy C57BL/6 mice, incubated *in vitro* for 24 hrs with LPS (0.5 µg/ml), αSyn (14.3 nM or 0.2 µg/ml), Grp94 (14.3 nM or 1.3 µg/ml), an αSyn and Grp94 combination (0.2 µg/ml and 1.3 µg/ml, respectively), or with buffer solution as reference. B-D. Levels of IFN-γ (B), IL-10 (C) and IL-17 (D) in the supernatants of the cultures mentioned in (A), measured using ELISA. The graphs are representative of two independent experiments, and the values correspond to the average ± S.D. of duplicates.

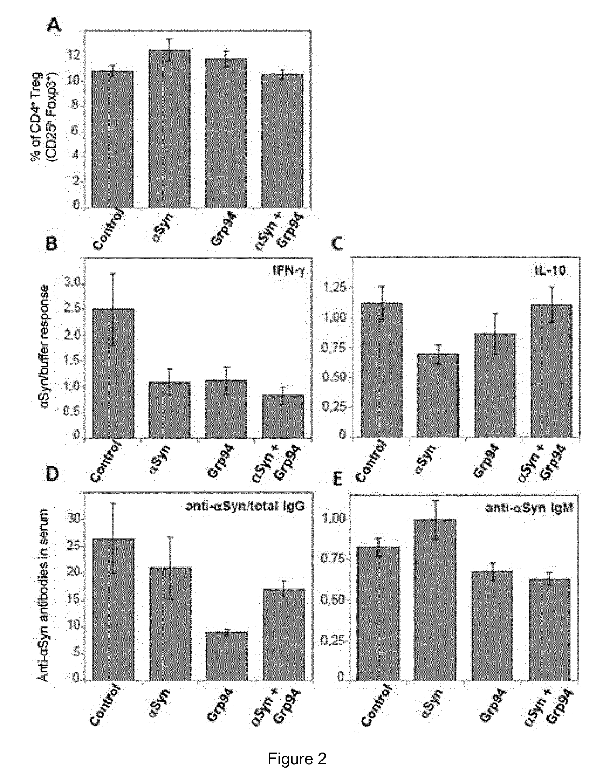

Figure 2 shows the Immune response generated in C57BL/6 mice immunized with an αSyn and Grp94 combination according to protocol 1. A. Flow cytometry analysis of the CD4⁺/CD25^{h}/Foxp3⁺ (Treg) splenocyte population with respect to the total CD4⁺ splenocytes. B and C. Levels of IFN-γ (B) and IL-10 (C) in the supernatants of splenocyte cultures incubated *in vitro* for 24 h with αSyn (15 µg/ml) or buffer solution. D. Relative levels of anti-αSyn IgG antibodies with respect to the total IgG in serum, measured using ELISA. The values correspond to the average ± S.D. with samples from 5 mice per group (N=5).

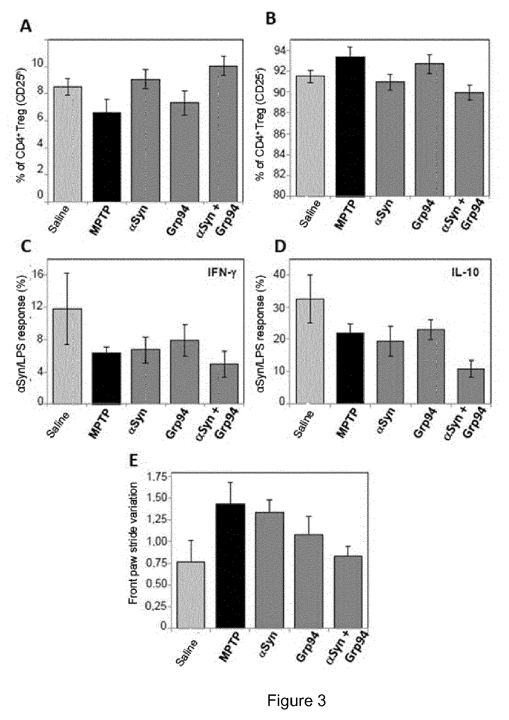

Figure 3 shows the immune response and behavioral test in C57BL/6 splenocyte receptor mice (obtained after the immunization of mutant mice according to protocol 1) with subsequent Parkinson's disease modeling by means of administration of MPTP. A and B. Flow cytometry analysis of the CD4⁺/CD25^{h} (Treg) splenocyte population with respect to the total CD4⁺ splenocytes (A), and of the CD4⁺/CD25⁻ (Teff) population with respect to the total CD4⁺ splenocytes (B). C and D. Levels of IFN-g (B) and IL-10 (C) in the supernatants of splenocyte cultures incubated *in vitro* for 24 hrs with αSyn (15 µg/ml) or LPS (0.5 µg /ml). E. Footprinting behavioral test (on front paws) after the adoptive transfer and subsequent administration of MPTP for 3 months, a recovery period of 1 month and administration of amphetamines (see Materials and Methods). The 'Saline' and 'MPTP' groups correspond to the 'healthy' and 'sick' animal controls which have not adopted splenocytes. The values correspond to the average ± S.E.M. of each group (N=7, with the exception of 'Saline' with N=4, and 'MPTP' with N=5).

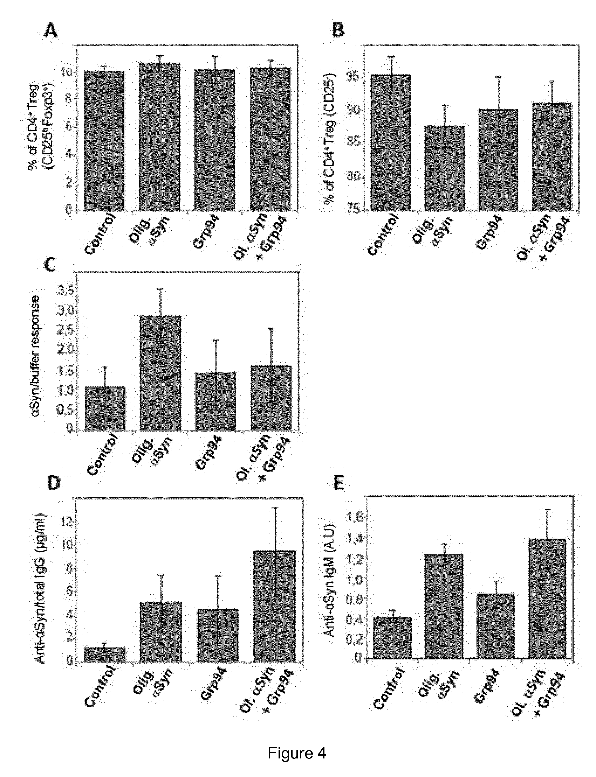

Figure 4 shows the immune response generated in C57BL/6 mice immunized with an αSyn and Grp94 combination, according to protocol 2. A and B. Flow cytometry analysis of the CD4⁺/CD25h (Treg) splenocyte population with respect to the total CD4⁺ splenocytes (A), and of the CD4⁺/CD25⁻ (Teff) population with respect to the total CD4⁺ splenocytes (B). C. Levels of IFN-γ in the supernatants of splenocyte cultures incubated *in vitro* for 24 hrs with αSyn (15 µg/ml) or buffer solution. D. Relative levels of anti-αSyn IgG antibodies with respect to the total IgG in serum, measured using ELISA. E. Levels of anti-αSyn IgM antibodies in serum, measured using ELISA. The values correspond to the average ± S.E.M. with samples from 5 mice per group (N=5).

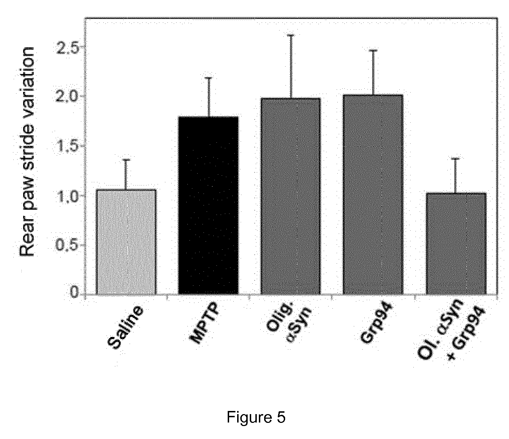

Figure 5 shows the behavioral test in C57BL/6 splenocyte receptor mice (obtained after immunization of the donor mice according to protocol 2) with subsequent Parkinson's disease modeling by means of administration of MPTP. *Footprinting* behavioral test (on rear paws) after the adoptive transfer of splenocytes, and subsequent administration of MPTP for 3 months, a recovery period of 1 month and administration of amphetamines (see Materials and Methods). The 'Saline' and 'MPTP' groups correspond to the 'healthy' and 'sick' animal controls which have not adopted splenocytes. The values correspond to the average ± S.E.M. of each group (N=5).

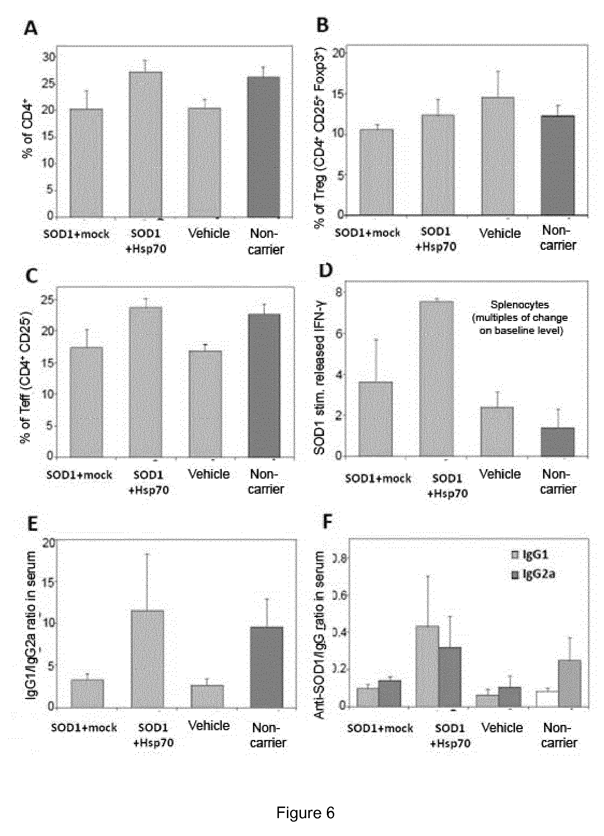

Figure 6 shows the immune response generated in mice model of ALS (Tg SOD1) vaccinated with a SOD1 and Hsp70 combination according to protocol 4. Preliminary results. Flow cytometry analysis of the CD4⁺ splenocyte population (A), CD4⁺/CD25^{h}/Foxp³⁺ (Treg) (B), and CD4⁺CD25⁻ (Teff) (C), with respect to the total CD4⁺ splenocytes. D. Levels of IFN-γ in the supernatants of splenocyte cultures incubated *in vitro* for 24 hrs with SOD1 (20 µg/ml) or buffer solution. E and F. Paired ratio per mouse of total IgG1/IgG2a antibody level (E) or anti-SOD1 level (F) in serum, measured using ELISA. Non-vehicle: non-transgenic animal (healthy control). The values correspond to the average ± S.E.M. with samples from 3 mice per group (N=3).

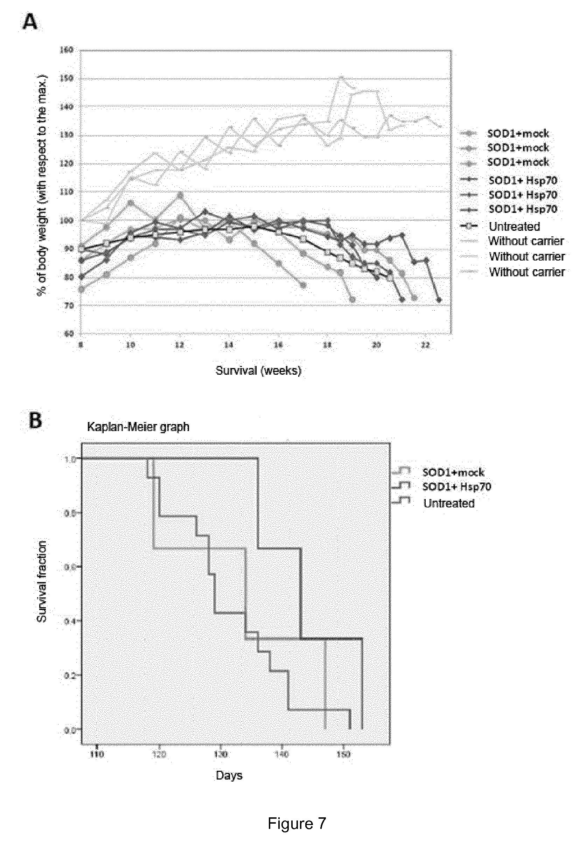

Figure 7 shows the evaluation of the degree of protection in the murine model of ALS (Tg SOD1) by means of measuring the weight and survival of the 'animals vaccinated according to protocol 4. Preliminary results. A. Representation of the progression of the body weight over time with respect to the maximum weight reached for each mouse. B. Representation of the survival with respect to time (Kaplan-Meier graph). Non-vehicle: non-transgenic animal (healthy control). Number of animals per group: 3 (N=3).

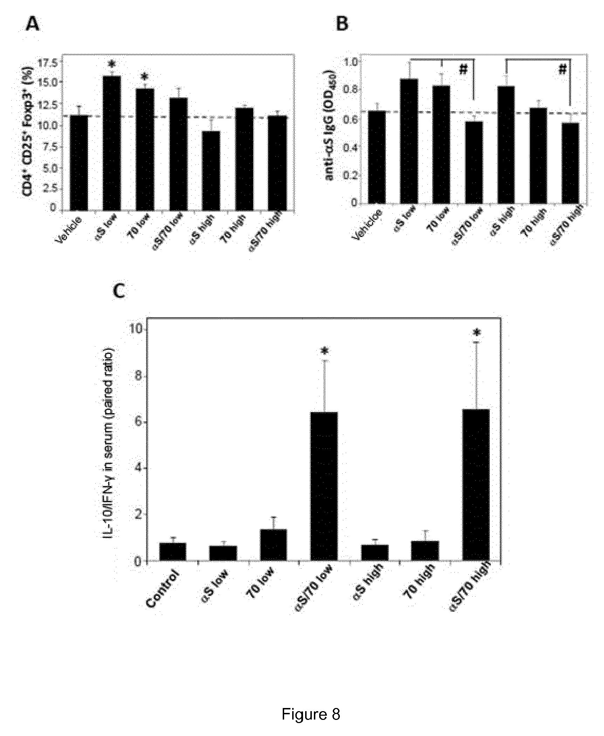

Figure 8 shows the characterization of the immune response generated by immunization of C57BL/6 mice with a αSyn and Hsp70 combination/complex in the absence of adjuvant. A. Flow cytometry analysis of the CD4⁺/CD25⁺/Foxp3⁺ (Treg) splenocyte population. B. Anti-αSyn IgG antibody level measured in serum using ELISA. C. Representation of the paired ratio per mouse of the levels of IL-10/IFN-γ measured in serum, using ELISA. αS: α-synuclein (aSyn); '70': Hsp70; 'low': low dose; 'high': high dose (see Materials and Methods). The values correspond to the average ± S.E.M. with samples from 6-7 mice per group (N=6-7).

### DETAILED DESCRIPTION OF THE INVENTION

The authors of the present invention have found that immunization with mixtures of α-synuclein (αSyn) and molecular chaperone are capable of promoting, modulating and redirecting immune response, being able to be very beneficial for the development of new therapeutic and prophylactic strategies against synucleinopathies. Additionally, the authors of the present invention have also proven how the use of other aggregating proteins in combination with molecular chaperones are shown to be excellent therapeutic tools for immunization against various "proteinopathies", such as Alzheimer's disease (AD), Huntington's disease, prion diseases, and type II diabetes.

There are provided, therefore, antibodies and cells (passive immunization) and vaccines (active immunization) for use in several methods for diagnosing and combating (which includes delaying the onset, the treatment and/or the prevention of) aggregating protein-related disorders, and more preferably α-synuclein-related disorders or *α-synucleinopathies,* such as Parkinson's disease (PD), Lewy body dementia (LBD), the Lewy body variant of Alzheimer's disease, multiple system atrophy (MSA) and other α-synuclein-related neurodegenerative disorders. Furthermore, the antibodies, cells and vaccines can be used for delaying the onset, the treatment and/or prevention of other neurodegenerative disorders such as Alzheimer's disease, Down syndrome, amyotrophic lateral sclerosis (ALS), frontotemporal dementia, Gaucher disease, Huntington's disease, prion disease, Creutzfeldt-Jakob disease, multiple sclerosis, Gerstmann-Straussler-Scheinker syndrome, Kuru, fatal familial insomnia, cerebrovascular amyloidosis, glaucoma, age-related macular degeneration, psychiatric syndromes, schizophrenia and/or schizophrenia-like disorders, neurodegeneration due to age-related protein aggregation. The antibodies, cells and vaccines can also be used in detection methods for the purpose of diagnostic therapy or follow up of said diseases, among others.

### COMPOSITIONS OF THE INVENTION

Therefore, a first aspect of the invention relates to a composition, hereinafter composition of the invention, comprising:
a) a mixture of peptides/polypeptides/proteins comprising i) at least one peptide/polypeptide/protein the amino acid sequence of which corresponds with an aggregating peptide/polypeptide/protein (hereinafter "sequence A of the invention") or any of the biologically active variants and fragments of said sequence, and ii) a peptide/polypeptide/protein the amino acid sequence of which corresponds with a molecular chaperone (hereinafter "sequence B of the invention") or with any of the biologically active variants and fragments of said sequence; and/or
b) a fusion peptide/polypeptide/protein, hereinafter "fusion amino acid sequence of the invention", comprising the amino acid sequences of at least one aggregating peptide/polypeptide/protein ("sequence A of the invention) or any of the biologically active variants and fragments thereof, and of a peptide/polypeptide/protein the amino acid sequence of which corresponds with a chaperone ("sequence B of the invention") or with any of the biologically active variants and fragments thereof.

"Sequence A" or "aggregating peptide" and/or "aggregating protein" is understood herein as peptides, polypeptides or proteins commonly found forming aggregates in 'conformational' diseases, particularly in neurodegenerative diseases (Knowles TP et al. 2014. Nat Rev Mol Cell Biol. 15:384-96.). These proteins include, but are not limited to, α-synuclein (SEQ ID NO: 1), huntingtin (SEQ ID NO: 2), beta-amyloid peptide (SEQ ID NO: 3), TDP-43 (SEQ ID NO: 4), FUS (SEQ ID NO: 5), tau (SEQ ID NO: 6), prion protein (SEQ ID NO: 7), fibronectin (SEQ ID NO: 8), and superoxide dismutase or SOD1, (SEQ ID NO: 9).

Therefore, in a preferred embodiment of this aspect of the invention the aggregating protein or sequence A of the composition of the invention is selected from the list consisting of α-synuclein, huntingtin, beta-amyloid peptide, TDP-43, FUS, tau, prion protein, fibronectin, SOD1 or any of the combinations thereof.

More preferably, the aggregating protein is α-synuclein or any of the biologically active variants and fragments thereof.

In another preferred embodiment, the aggregating protein is SOD1 or any of the biologically active variants and fragments thereof.

α-synuclein (SNCA, NACP, PARK1, PARK4, PD1, alpha-synuclein; non A-beta component of AD amyloid; synuclein alpha-140) is understood herein as a member of the synuclein family which also includes beta- and gamma-synuclein. Synucleins are expressed abundantly in the brain and alpha- and beta-synuclein D2 selectively inhibit phospholipase. α-synuclein can be used for integrating the signaling and the traffic of the presynaptic membrane. α-synuclein defects have been involved in the pathogenesis of Parkinson's disease. α-synuclein is a main component of amyloid plaques in the brain of patients with Alzheimer's disease. Two different isoforms have been identified for this gene. Its amino acid sequence (the amino acid sequence of α-synuclein) corresponds with SEQ ID NO: 1.

In the context of the present invention, the α-synuclein gene is also defined by a nucleotide or polynucleotide sequence, which constitutes the coding sequence of the protein included in SEQ ID NO: 1 and would comprise different variants originating from:
a) nucleic acid molecules encoding a polypeptide comprising the amino acid sequence of SEQ ID NO: 1,
b) nucleic acid molecules the complementary strand of which hybridizes with the polynucleotide sequence of a),
c) nucleic acid molecules the sequence of which differs from a) and/or b) due to genetic code degeneration,
d) nucleic acid molecules encoding a polypeptide comprising the amino acid sequence with an identity of at least 80%, 90%, 95%, 98% or 99% with SEQ ID NO: 1, and in which the polypeptide encoded by said nucleic acids has the activity and structural characteristics of the α-synuclein protein. Immunogenic activity is also included within this activity.

In addition to using the recombinant protein or synthetic peptides, α-synuclein can be derived directly from Lewy bodies present in the post mortem autopsy of human brain tissue for cases with α-synucleinopathies. Both soluble (i.e., preparations soluble in Tris-buffered saline solution) and insoluble (i.e., preparations insoluble in Tris-buffered saline solution) α-synuclein fractions are purified. These sample preparations can be injected into animals as such, or fractioned using chromatographic methods before injection. The generated antibodies can be used for the treatment of patients in a passive vaccination schedule or in diagnostic immunoassays.

In the present invention, α-synuclein refers both to the soluble, unfolded native form and to that with conformational and post-translational modifications. Furthermore, native α-synuclein can be modified by means of, for example, but without limitation, substitution of Ser/Ala with Cys, for example, through site-directed mutagenesis. An example of this type is described in the following patent application by Chang, US2006/0018918 A1.

SOD1 (also referred to as ALS; SOD; ALS1; IPOA; hSod1; HEL-S-44; homodimer) is understood herein as a protein that binds copper and zinc ions and is one of two isozymes responsible for destroying free superoxide radicals in the body. The encoded isozyme is a soluble cytoplasmic protein acting as a homodimer to convert natural but damaging superoxide radicals into molecular oxygen and hydrogen peroxide. The other isozyme is a mitochondrial protein. Mutations in this gene were involved as causes of familial amyotrophic lateral sclerosis. Its amino acid sequence (the amino acid sequence of SOD1) is included in GenBank sequence NP_000445.1, MM_000454.4.

In the context of the present invention, the *SOD1* gene is also defined by a nucleotide or polynucleotide sequence which constitutes the coding sequence of the protein listed in sequence NP_000445.1 and would comprise different variants originating from:
a) nucleic acid molecules encoding a polypeptide comprising the amino acid sequence Nip_000445.1,
b) nucleic acid molecules the complementary strand of which hybridizes with the polynucleotide sequence of a),
c) nucleic acid molecules the sequence of which differs from a) and/or b) due to genetic code degeneration,
d) nucleic acid molecules encoding a polypeptide comprising the amino acid sequence with an identity of at least 80%, 90%, 95%, 98% or 99% with NP_000445.1, and in which the polypeptide encoded by said nucleic acids has the activity and structural characteristics of the SOD1 protein. Immunogenic activity is also included within this activity.

In another preferred embodiment of this aspect of the invention, the chaperone or sequence B of the invention belongs to the Hsp70 family, Hsp90 family, immunophilin family, peptidase C56 family, PPlase family, 14-3-3 family, the small heat-shock protein group, small GTPase family, Hsp40 (DnaJ) family, or any of the combinations thereof. More preferably, the chaperones of the Hsp90 family are selected from Grp94 (SEQ ID NO: 10) and Hsp90 (SEQ ID NO: 11) or any of the combinations thereof. In another preferred embodiment, the chaperones of the Hsp70 family are selected from Hsp70 (SEQ ID NO: 12), Hsc70 (SEQ ID NO: 13) and Grp75 (SEQ ID NO: 14) or any of the combinations thereof. In another preferred embodiment, the chaperones of the immunophilin family are selected from FKBP12 (SEQ ID NO: 15) and FKBP4/FKBP52 (SEQ ID NO: 16) or the combination thereof. In another preferred embodiment, the chaperone of the peptidase C56 family is DJ1/PARK7 (SEQ ID NO: 17). In another preferred embodiment, the chaperones of the PPlase family are selected from Pin1 (SEQ ID NO: 18), CypA (SEQ ID NO: 19), Cyp40 (SEQ ID NO: 20) or any of the combinations thereof. In another preferred embodiment, the chaperones of the 14-3-3 family are selected from 14-3-3 gamma (SEQ ID NO: 21), 14-3-3 epsilon (SEQ ID NO: 22), 14-3-3 tau (SEQ ID NO: 23) or any of the combinations thereof. In another preferred embodiment, the chaperone of the small heat-shock protein group is Hsp27 (SEQ ID NO: 24). In another preferred embodiment, the chaperone of the small GTPase family is Rab11A (SEQ ID NO: 25). In another preferred embodiment, the chaperones of the Hsp40 (DnaJ) family are selected from Hsp40 (SEQ ID NO: 26) and CSP (SEQ ID NO: 27) or the combination thereof. In another preferred embodiment, the chaperone is clusterin (SEQ ID NO: 28), Grp170 (SEQ ID NO: 29), calreticulin (SEQ ID NO: 30), Hsp105 (SEQ ID NO: 31), CHIP (SEQ ID NO: 32), alpha-crystallin (SEQ ID NO: 33) or any of the combinations thereof. In the composition of the invention, the combination of different chaperones, even from different group or family, can also be possible.

In a more preferred embodiment, the chaperone is Grp94 or any of its biologically active variants, and combinations thereof. Even more preferably, the chaperone is Grp94.

Grp94 (heat shock protein 90 kDa beta (Grp94), member 1, ECGP; GP96; TRA1; GRP94) is a gene encoding a member of family of adenosine triphosphate (ATP)-metabolizing molecular chaperones with roles in stabilizing and folding other proteins. The encoded protein is localized to melanosomes and the endoplasmic reticulum. Expression of this protein is associated with a variety of pathogenic states, including tumor formation. There is a microRNA gene located within the 5' exon of this gene. There are pseudogenes for this gene on chromosomes 1 and 15.

In the context of the present invention, the Grp94 gene is also defined by a nucleotide or polynucleotide sequence, which constitutes the coding sequence of the protein included in SEQ ID NO: 10 and would comprise different variants originating from:
a) nucleic acid molecules encoding a polypeptide comprising the amino acid sequence of SEQ ID NO: 10,
b) nucleic acid molecules the complementary strand of which hybridizes with the polynucleotide sequence of a),
c) nucleic acid molecules the sequence of which differs from a) and/or b) due to genetic code degeneration,
d) nucleic acid molecules encoding a polypeptide comprising the amino acid sequence with an identity of at least 80%, 90%, 95%, 98% or 99% with SEQ ID NO: 10, and in which the polypeptide encoded by said nucleic acids has the activity and the structural characteristics of the Grp94 protein.

Several amino acid sequences are available in the databases for the peptides and proteins of the invention, differing in one or more amino acids of the following amino acid sequences: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33. These peptide and protein variants are comprised in the present invention, provided that they have an activity and a structural behavior similar to the amino acid sequence from which it is derived. As it is used herein, the protein variants of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33 are proteins with an amino acid sequence differing from said amino acid sequence due to one or more substitutions, additions or deletions of amino acids. Generally, any amino acid residue of the sequence shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33 can be substituted with another amino acid, provided that the resulting protein variant exhibits an activity and a structural behavior similar to the amino acid sequence from which it is derived. The activity and structural behavior of said proteins can be determined using methods known in the state of the art. In particular, protein variants differing from the amino acid sequences that have been shown in up to 5, 10, 15, 20 or even 25 amino acid positions are included.

Preferably, the substitutions are conservative substitutions, i.e., substitutions of an amino acid residue with another amino acid having a similar polarity, acting as a functional equivalent. Preferably, the amino acid residue used as a substitute is selected from the same amino acid group as the amino acid residue to be substituted. For example, a hydrophobic residue can be substituted with another hydrophobic residue, or a polar residue can be substituted with another polar residue having the same charge. Functionally homologous amino acids which can be used for a conservative substitution comprise, for example, non-polar amino acids such as glycine, valine, alanine, isoleucine, leucine, methionine, proline, phenylalanine and tryptophan. The examples of uncharged polar amino acids include serine, threonine, glutamine, asparagine, tyrosine and cysteine. The examples of charged polar amino acids (basic) include histidine, arginine and lysine. The examples of charged polar amino acids (acidic) include aspartic acid and glutamic acid.

The term "variant" also comprises amino acid sequences including more amino acids than the sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33, i.e., amino acid sequences in which one or more amino acids have been inserted. Such insertions can in principle take place in any position of the amino acid sequences of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33. The insertions can be contiguous amino acid segments comprising, for example, 2, 3, 4, 5, 6, 7, 8, 9 or 10 amino acids. Similarly, the term "variant" also includes amino acid sequences in which one or more amino acids are deleted compared with the amino acid sequences of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33. Deletion can involve several contiguous amino acid residues of the mentioned sequences for example, 2, 3, 4, 5, 6, 7, 8, 9 or 10 amino acids. This deletion does not affect the activity and structural behavior of said variants. The protein variants can also include structural modifications, for example, modified amino acids, such as amino acids which have been altered by phosphorylation, nitration, glycosylation, acetylation, thiolation, branching and/or cyclization.

The protein variants of sequence SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33, which have been modified by substitution, addition or deletion of amino acids, usually show a considerable degree of amino acid sequence identity or of identity with the protein of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33. Preferably, the homology or identity at the amino acid level is at least 70%, 75%, 80%, 85%, 90%, 91 %, 92%, 93%, 94%, 95%, 96%, 97%, 98% or 99% if the sequence of the variant is optimally aligned with that of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33, respectively. The methods and computer programs for determining amino acid homology are well known in the art.

The context of the invention also contemplates using immunologically active fragments either derived from the protein of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33, or from variants thereof. Immunologically active fragments of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33 or variants thereof include peptides or polypeptides differing from the amino acid sequence shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33, or of variants thereof due to the absence of one or more amino acids at the N-terminal end and/or the C-terminal end of the protein. The person having average skill will be capable of determining other active fragments of the sequence represented in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and/or SEQ ID NO: 33 or variants thereof by means of performing the routine methods described in great detail in the state of the art.

Within biologically active variants, it is understood, however, that a protein activity loss of 20%, 30%, 40%, 50%, 60%, 70%, or even up to 80% can be tolerated if a sequence (A and/or B) or a particular fusion amino acid sequence exhibits other favorable characteristics, such as greater stability or shelf life or improved pharmacological properties. A person skilled in the art will be capable of easily determining if a given fusion amino acid sequence of the invention is suitable for being used in a desired therapeutic context.

In a more preferred embodiment of the invention, the aggregating protein and/or the chaperone were obtained in a recombinant manner. Alternatively, both the aggregating protein and the chaperone can be obtained from cells or tissues. In turn, both the aggregating protein/proteins and the chaperone or chaperones forming part of the composition of the invention can be of a human or animal origin, being able to be any known ortholog of said protein or proteins. Preferably, it is of human origin (or in the case of having been obtained in a recombinant manner, the amino acid sequence has a high percentage of identity with the known amino acid sequence of the human protein, preferably of at least 70%, more preferably 80%, 90%, 95%, 98%, or much more preferably 90%, with the known human amino acid sequence of said protein).

As it is used herein, the term "identity" refers to the proportion of identical amino acids (or nucleotides) between two amino acid (or nucleotide) sequences that are compared.

When comparing the sequence of an aggregating protein or a human chaperone with the sequence of the same protein in other species, it is evident that there are certain regions that are more conserved than others. This information can be indicative of the fact that those zones are crucial for maintaining protein structure or function. Both the active regions determining the activity of said proteins and the conserved cysteines between which disulfide bridges are established, important for protein formation, will most probably be conserved between both proteins, whereas divergence is more apparent in other regions.

As it is used herein, the term "homology" refers to the similarity between two structures due to a common evolutionary lineage, and more specifically, to the similarity between the amino acids (or nucleotides) of two or more proteins (polynucleotides) or amino acid (or nucleotide) sequences.

Given that two proteins are considered homologous if they have the same evolutionary origin or if they have similar function and structure, it is generally assumed that values of similarity or identity greater than 30% indicate homologous structures. Therefore, it can be considered that percentages of identity of at least 80% will include the active regions conserved between the different species capable of generating immunogenicity or having chaperone activity and also immunological activity, where appropriate.

Sequence A and sequence B of the invention can be found bound in a fusion protein, or alternatively chemically bound to one another, for example, but without limitation, by means of cross-linking.

"Cross-linking" is understood herein as a chemical technique binding two or more molecules by means of a covalent bond; they contain substances having ends reactive with respect to specific functional groups of proteins or other molecules. These can be divided into homobifunctional (the same reactive groups) and heterobifunctional (different reactive groups), having chemical cross-linkers which may or may not be inverted. Homobifunctional cross-linkers have a potential drawback connecting two neighboring groups, either on the surface of nanoparticles or in the induction of undesired cross-linking proteins. Heterobifunctional cross-linkers allow sequential conjugations, minimizing polymerization. For example, sulfosuccinimidyl 4-(N-maleimidomethyl)cyclohexane-1-carboxylate (sulfo-SMCC) can be used for coupling the content of thiol biomolecules with amine-coated nanoparticles, or vice versa, considering that the heterobifunctional cross-linker 1-ethyl-3-(3-dimethylaminopropyl) carbodiimide (EDC) is commonly used for connecting NH₂ and -COOH groups (Jameson and Wong, 2009; Hurt et al., 2010 Chem Biol. April 24; 16(4): 372-381).

Additionally, sequences A and B of the invention can be obtained synthetically. The design of synthetic amino acid sequences is known in the state of the art. Therefore, analysis of the data of experimentally determined antigenic sites has revealed that hydrophobic residues, Leu and Val, if they are found on the protein surface, have a great tendency to form part of antigenic determinants. Semi-empirical methods using the physicochemical properties of the amino acid residues and their frequencies and occurrence in segments of experimentally known epitopes for determining the antigenic determinants of a protein have therefore been developed.

Synthetic amino acid sequences have several advantages with respect to specific antibody production and reactivity. The exact sequence of the synthesized peptide can be selected from the amino acid sequence of the protein as determined by the amino acid sequence of the protein or by the predicted amino acid sequence determined from the DNA sequence encoding the protein.

The use of synthetic specific peptides eliminates the need for a full-length protein in vaccination and production of an antibody assay. Furthermore, Merrifield solid-phase peptide synthesis techniques allow chemically producing unlimited amounts of peptides, essentially from the synthetic peptide of interest. Said techniques have progressed to the automated peptide synthesis capacity.

### NUCLEOTIDES, GENE CONSTRUCTS, EXPRESSION VECTORS AND HOST CELLS OF THE INVENTION

Synthetic amino acid sequences can also be prepared through expression in a host cell containing a recombinant DNA molecule comprising a nucleotide sequence that is transcribed to the peptides or proteins of the invention, operatively bound to an expression control sequence, or a vehicle or recombinant DNA cloning vector containing such recombinant DNA molecule. Alternatively, the peptides can be expressed by direct injection of a single DNA molecule into a host cell. Generally, the synthetic peptides produced according to the invention have protective antigenic sequences. As it is used herein, the expression "protective antigen" defines those antigens capable of generating a protective immune (immunogenic) response in the host, i.e., a host response, leading to the generation of immune effector molecules, antibodies or cells sterilizing, suppressing or reducing the activity of the aggregating proteins which are causing a disease, thereby "protecting" the host from a clinical or sub-clinical disease and/or from loss of productivity. Such protective immune response can usually take place through the generation of an immunocompetent cell-mediated protective response and of antibodies which are capable of inhibiting the function, cytotoxicity mechanism or cell-to-cell propagation of aggregating proteins, leading to their elimination or to a loss of their negative effect on the host. Said host is preferably a mammal, and even much more preferably, a human.

Therefore another aspect of the invention relates to a nucleotide sequence, hereinafter nucleotide sequence of the invention, comprising one or more nucleotide sequences, encoding the entire or a fraction of sequence A, sequence B, or the fusion amino acid sequence of the invention.

Providing a nucleic acid molecule or a nucleotide sequence according to the invention therefore makes it possible to obtain sequences A and B, and/or the fusion amino acid sequence/sequences of the invention, as well as their biologically active variants or immunogenic fragments, in amounts not available up until now, thereby allowing the development of compositions, preferably pharmaceutical compositions.

This aspect of the invention provides a method for preparing a recombinant protein or peptide encoded by a nucleic acid molecule or nucleotide sequence of the invention which comprises culturing a eukaryotic or prokaryotic cell containing a nucleic acid molecule or nucleotide sequence of the invention, in conditions in which said protein or peptide is expressed, and recovering said peptide thus produced.

This method includes the cloning and expression vectors comprising the nucleic acid molecules or nucleotide sequences of the invention. Such expression vectors include suitable control sequences, such as, for example, translation control elements (such as start and stop codes) and transcription control elements (for example, promoter-operator regions, binding sites, etc.). The vectors according to the invention can include plasmids and viruses (comprising bacteriophages and eukaryotic viruses), according to methods well known and documented in the art, and can be expressed in a variety of different expression systems, also well known and documented in the art. Suitable viral vectors include baculoviruses and also adenoviruses and vaccine viruses. Many other viral and non-viral vectors are described and known in the art.

Therefore, another aspect of the invention relates to an isolated expression vector (hereinafter expression vector of the invention), comprising the nucleotide sequence of the invention.

A variety of techniques which can be used for introducing such vectors into prokaryotic or eukaryotic cells for expression, or into a germline or into somatic cells, to form transgenic animals, is also known. Suitable transformation or transfection techniques are well described in the bibliography.

Transformed or transfected eukaryotic or prokaryotic host cells containing a nucleic acid molecule or the nucleotide sequence according to the invention, as defined above, also form part of this aspect of the invention.

Therefore, another aspect of the invention relates to an isolated host cell, (hereinafter host cell of the invention), comprising an expression vector of the invention or a nucleotide sequence of the invention.

### ANTIBODIES OF THE INVENTION

The compositions of the invention are formulated for use as immunogens. These immunogens can also be used as vaccines in animals, and more particularly in mammals, including humans, or for producing a response in the production of antibodies in animals. For the formulation of such compositions, an immunologically effective amount of each of the sequences A and B of the composition of the invention together with the use of a suitable physiologically acceptable vehicle for administration to mammals including humans, are necessary. Sequences A and B can be covalently bound to one another, to other peptides, to a vehicle protein or to other vehicles, incorporated in nanoparticles, liposomes or other similar vesicles, and/or mixed with an adjuvant or absorbent as is well known in the field of vaccines. For example, sequences A and B can be mixed with immunostimulating complexes. Alternatively, sequences A and B are not coupled to and are merely mixed with a physiologically acceptable vehicle such as a normal saline or buffer compound suitable for administration to mammals including humans.

Therefore, and as described above, synthetic sequences A and B produced according to the invention have protective antigenic sequences. These protective antigens are capable of generating a protective immune (immunogenic) response in the host, i.e., a host response, leading to the generation of immune effector molecules, antibodies or cells inactivating or reducing the activity or effectiveness of the aggregating proteins causing a disease, thereby "protecting" the host from a clinical or sub-clinical disease and from loss of productivity. Such protective immune response can usually take place through the generation of immunocompetent cell populations and/or antibodies which are capable of inhibiting the function of the aggregating protein causing the disease.

As with all immunogenic compositions for producing a response in antibodies, the immunologically effective amounts of sequences A and B of the invention must be determined empirically. Considered factors include the immunogenicity of the natural peptide, whether or not the peptide is complexed through a covalent bond with an adjuvant or a vehicle protein or another vehicle, and the route of administration for the composition, for example, and without limitation, the intravenous route, intramuscular route, subcutaneous route, and as in a particular embodiment of the invention, the intranasal route or oral route, as well as the number of immunization doses that would be administered. Such factors are known in the field of vaccines and it is in accordance with the skill of the immunologist who has performed such determinations without undue experimentation. The antibodies will be capable of binding to sequences A or B of the invention or to the fusion amino acid sequence of the invention.

Therefore, another aspect of the invention relates to an antibody or a fragment thereof, hereinafter antibody of the invention or antibody fragment of the invention, capable of binding to one of sequences A or B of the invention or to the fusion amino acid sequence of the invention. In a preferred embodiment of this aspect, the antibody or the antibody fragment of the invention is obtained after immunization of an animal or of the cells of an animal with amino acid sequences A and B of the invention or with the fusion amino acid sequence of the invention. In a particular embodiment of this aspect of the invention, the animal which is used for immunization is a mammal, including human beings.

In a preferred embodiment, the animal which is used for immunization is a mammal, not including human beings.

These antibodies can be monoclonal or polyclonal. The antibody of the invention can be also recombinant, chimeric, humanized, synthetic or a combination of any of the foregoing.

In another particular embodiment of this aspect of the invention, the antibodies are used as a medicinal product.

The antibodies of the present invention can be formulated for administration to an animal, and more preferably to a mammal, including human beings, in a variety of forms. Therefore, the antibodies can be in a sterile aqueous solution or in biological fluids, such as serum. The aqueous solutions may or may not be buffered and have additional active or inactive components. The additional components include salts for modulating ionic strength, preservatives including, but not limited to, antimicrobial agents, antioxidants, chelating agents and the like, and nutrients including glucose, dextrose, nucleotides, vitamins and minerals. Alternatively, the antibodies can be prepared for administration in solid form. The antibodies can be combined with several inert excipients or vehicles, including but not limited to, binders such as microcrystalline cellulose, tragacanth gum or gelatin; excipients such as starch or lactose; dispersing agents such as alginic acid or cornstarch; lubricants such as magnesium stearate, glidants such as colloidal silicon dioxide; sweetening agents such as sucrose or saccharine; or favoring agents such as mint or methyl salicylate.

The antibodies or their formulations can be administered to an animal, including a mammal and, therefore, human beings, in a many ways. Such ways include, but are not limited to, intraperitoneally, intravenously, intramuscularly, subcutaneously, intrathecally, intraventricularly, orally, enterally, parenterally, intranasally or cutaneously.

The antibody dosage for obtaining a pharmaceutically effective amount depends on a variety of factors, such as, for example, the age, weight, gender, tolerance, etc... of the animal.

The term "antigen" refers herein to a cell surface molecule (generally a protein or a polysaccharide) which can induce antibody formation. There are many different types of molecules which can act as antigens, such as proteins or peptides, polysaccharides and, more rarely, other molecules such as nucleic acids. Specifically, the term antigen would refer herein to amino acid sequences A and B of the invention, as well as to the fusion amino acid sequence of the invention, i.e., both to aggregating proteins and to chaperones.

A preferred embodiment of this aspect of the invention relates to fragments of the antibodies of the invention for being used in the same way and manner as that described for the antibodies of the present invention. Some examples of the fragments of the antibodies of the invention are shown below:
- "Fab" fragments resulting from digestion of a whole antibody with papain and comprising only one antigen-binding site and a CL region and CH1,
- "F(ab')2" fragments resulting from digestion of a whole antibody with pepsin and containing two antigen-binding sites,
- "Fab"" fragments containing the light-chain constant domain and the first heavy-chain constant domain (CH1) and has only one antigen-binding site. The Fab' fragments differ from the Fab fragments by the addition of certain residues at the carboxyl terminal end of the heavy-chain domain CH1 including one or more cysteines of the hinge region of the antibody,
- "Fv" is the minimum antibody fragment containing a complete antigen-recognition and -binding site. This region consists of a dimer of one heavy- and one light-chain variable domain in close, non-covalent association. In this configuration, the three hypervariable regions (CDRs) of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the capacity to recognize and bind to the antigen, although at a lower affinity than the entire binding site.
- Single-chain FV or "scFv" antibody fragments comprise the VL and VH antibody domains, these domains being present in a single polypeptide chain. Preferably, the VL and VH regions are connected by a polypeptide linker which allows scFv to form desired structures for antigen-binding.
- "Diabodies" comprise a heavy-chain variable domain (VH) connected to a light-chain variable domain (VL) in the same polypeptide chain (VH-VL) connected by means of a peptide linker which is too short to allow pairing between the two domains on the same chain. This forces pairing with the complementary domains of another chain and promotes the assembly of a dimeric molecule with two functional antigen-binding sites.
- "Bispecific antibodies" (BAb) are individual, divalent antibodies (or fragments thereof effective in immunotherapy) having two specific antigen-binding sites for binding to the antigen in a different manner. The two antigen sites can be coupled to one another chemically or by means of genetic engineering methods known in the art.

All these antibody fragments can additionally be modified using conventional techniques known in the art, for example, using deletion/deletions, insertion/insertions, substitution/substitutions, addition/additions of amino acids and/or recombination/recombinations and/or any other modification/modifications (for example, post-translational or chemical modifications, such as glycosylation and phosphorylation) known in the art, alone or in combination.

### IMMUNOTHERAPEUTIC CELLULAR COMPOSITION OF THE INVENTION

Another aspect of the invention relates to an immunotherapeutic cell composition, hereinafter immunotherapeutic cellular composition of the invention, comprising at least one activated antigen presenting cell (APC), hereinafter activated antigen presenting cell of the invention, in which said APC are obtained from a patient diagnosed with a disease caused by an aggregating protein of the invention and wherein said APC is stimulated by *ex vivo* exposure to the composition of the invention. In a preferred embodiment of this aspect of the invention the isolated, activated antigen presenting cell or APC is a dendritic cell (DC).

As it is used herein, the term "antigen presenting cells" or "APCs" refers to cells which are capable of activating T cells, and include, but are not limited to, certain macrophages, B cells, and more preferably, dendritic cells (DCs). "Potent antigen presenting cells" are cells which, after being put in contact with an antigen, can activate CD8+ cytotoxic T lymphocytes (naive CTL) in a primary immune response.

"Dendritic cells" or "DCS" are members of a diverse population of morphologically similar cell types found in lymphoid or non-lymphoid tissues.

These cells are characterized by their distinctive morphology and high levels of MHC-class II expression surface. Steinman et al., Ann. Rev. Immunol. 9: 271 (1991). APCs and DCs can be isolated from a series of tissue sources, and suitably from peripheral blood, as described, for example, in WO2004026238. Preferred immunotherapeutic compositions of the present invention use APCs or DCSs which are isolated from a patient diagnosed with a disease caused by an aggregating protein, or preferably with α-synucleinopathy.

APCs and DCs can be isolated by means of routine methodologies which are available in the art. A suitable example of a methodology for isolating DCs is disclosed in US patent No. 5976,546, No. 6,080,409, and No. 6,210,662.

### PHARMACEUTICAL COMPOSITION OF THE INVENTION

Another aspect of the invention relates to a pharmaceutical composition, hereinafter pharmaceutical composition of the invention, comprising the composition of the invention, the nucleotide sequence of the invention, the expression vector of the invention, the host cell of the invention, the antibody of the invention, or the immunotherapeutic cellular composition of the invention. In a preferred embodiment of this aspect, the pharmaceutical composition further comprises a pharmaceutically acceptable vehicle. In another preferred embodiment, the pharmaceutical composition further comprises an adjuvant. In another preferred embodiment, the composition of the invention does not comprise an adjuvant.

The term "adjuvant" refers herein to an agent, as long as it does not have an antigenic effect itself, which can stimulate the immune system increasing its response to the vaccine. Although not being limited thereto, aluminum salts "aluminum phosphate" and "aluminum hydroxide" are the two most commonly used adjuvants in vaccines. Other substances, such as, for example, squalene can also be used as adjuvants.

Another aspect of the invention relates to a dosage form, hereinafter dosage form of the invention, comprising the composition of the invention.

The pharmaceutical compositions provided herein contain therapeutically effective amounts of each of the sequences A and B provided herein which are useful in the treatment of or in improving one or more of the symptoms of aggregating protein toxicity-associated diseases or disorders.

Suitable pharmaceutical vehicles for the administration of the compositions provided herein include any of the vehicles known by the persons skilled in the art which are considered suitable for the particular mode of administration.

Furthermore, the compositions of the invention can be combined with other active ingredients.

The pharmaceutical compositions of the invention can be formulated in suitable pharmaceutical preparations such as solutions, suspensions, tablets, dispersible tablets, pills, capsules, powders or sustained release formulations or elixirs, for oral administration, or in sterile solutions or suspensions for parenteral administration, as well as in preparations of transdermal patches and dry powder inhalers. In a preferred embodiment, the pharmaceutical compositions of the invention are formulated using techniques and methods well known in this field (see, for example, Ansel Introduction to Pharmaceutical Dosage Forms, Fourth Edition 1985, 126).

The active ingredients can be administered at one go, or can be divided into several smaller doses to be administered at time intervals. It is understood that the dosage and the precise duration of treatment depend on the disease being treated and can be determined empirically using known test protocols or by extrapolation from *in vivo* or *in vitro* assay data. It must be indicated that the concentrations and values of dosage also can change with the severity of the condition to be alleviated. Furthermore, it must be understood that for any particular subject, the specific dosage regimens must be adjusted over time according to individual need and the professional criterion of the person administering or supervising the administration of the compositions, and that the concentration ranges described herein are merely illustrative and do not seek to limit the scope or practice of the claimed compositions.

As it is used herein, the term "active ingredient", "active substance", "pharmaceutically active substance", or "pharmaceutically active ingredient" means any component which potentially provides a pharmacological activity or another different effect in diagnosing, curing, mitigating, treating or preventing a disease, or which affects the structure or function of the human body or the body of other animals. The term includes those components promoting a chemical change in the preparation of a drug and are present therein in an envisaged modified form providing the specific activity or the effect. In the present invention it primarily refers, but is not limited, to the combination of sequences A and B of the invention, as well as to the fusion amino acid sequence of the invention, the antibodies of the invention and the activated antigen presenting cell of the invention.

In another preferred embodiment of the invention, the pharmaceutical composition of the invention is a vaccine.

### COMBINED PREPARATION OF THE INVENTION

Sequences A and B of the invention can be found bound to one another in one and the same composition, or juxtaposed for simultaneous, combined or sequential administration to a mammal, including human beings.

Therefore, another aspect of the invention relates to a combined preparation, hereinafter combined preparation of the invention, comprising sequences A and B of the invention or any of their biologically active fragments and/or variables.

It must be emphasized that the term "combined preparation", or also referred to as "juxtaposition", herein means that the components of the combined preparation do not need to be bound to one another, for example, in a true composition, so that they will be available for combined, separate or sequential application. The expression "juxtaposed" therefore means that a true combination is not necessary in view of the physical separation of the components.

In another preferred embodiment of the invention, the combined preparation of the invention is a vaccine.

### USES OF THE INVENTION

Another aspect of the invention relates to the use of sequences A and B of the invention, the composition of the invention, the nucleotide sequence of the invention, the expression vector of the invention, the host cell of the invention, the antibody of the invention, the immunotherapeutic cellular composition of the invention, the pharmaceutical composition of the invention, or the combined preparation of the invention, in the preparation of a medicinal product. In a preferred embodiment, the medicinal product is a vaccine. Therefore, another aspect of the invention relates to a vaccine, hereinafter vaccine of the invention, comprising sequences A and B of the invention, the composition of the invention, the nucleotide sequence of the invention, the expression vector of the invention, the host cell of the invention, the antibody of the invention, the immunotherapeutic cellular composition of the invention, the pharmaceutical composition of the invention, or the combined preparation of the invention.

In the context of the present invention, the term "vaccine" refers to an antigenic preparation used for establishing the response of the immune system to a disease. They are antigen preparations which cause, once in the body, the immune system response, by means of antibody production, and generate immunological memory producing permanent or temporary immunity.

The vaccine can contain a single type of peptide or a variety of peptides covering different or similar epitopes. Furthermore, or as an alternative, a single polypeptide with multiples epitopes can be provided. This last type of vaccine is referred to polyvalent vaccine. A multiple epitope includes two or more repetitive epitopes.

Another aspect of the invention relates to the use of sequences A and B of the invention, the composition of the invention, the nucleotide sequence of the invention, the expression vector of the invention, the host cell of the invention, the antibody of the invention, the immunotherapeutic cellular composition of the invention, the pharmaceutical composition of the invention, or the combined preparation of the invention, in the preparation of a medicinal product for the treatment or prevention of aggregating protein-related diseases or disorders.

In a preferred embodiment of this aspect of the invention, the aggregating protein is α-synuclein or α-synucleinopathies. In a preferred embodiment of this aspect, the α-synuclein-related disease or disorder is selected from the list consisting of: Parkinson's disease (PD), Lewy body dementia (LBD), the Lewy body variant of Alzheimer's disease, multiple system atrophy (MSA), Alzheimer's disease, Down syndrome, amyotrophic lateral sclerosis (ALS), frontotemporal dementia, Gaucher disease, Huntington's disease, prion disease, Creutzfeldt-Jakob disease, multiple sclerosis, Gerstmann-Sträussler-Scheinker syndrome, Kuru, fatal familial insomnia, cerebrovascular amyloidosis, glaucoma, age-related macular degeneration, neurodegeneration due to age-related protein aggregation, psychiatric syndromes, schizophrenia and/or schizophrenia-like disorders. More preferably, it is Parkinson's disease (PD).

In another preferred embodiment, the aggregating protein is SOD1, and even more preferably the disease is amyotrophic lateral sclerosis (ALS).

Another aspect of the invention relates to the use of the combined preparation of the invention, comprising sequences A and B of the invention, in the preparation of a medicinal product for combined, simultaneous or sequential administration, for the treatment or prevention of aggregating protein-related diseases or disorders of the invention. In a preferred embodiment, the aggregating protein is α-synuclein or synucleinopathies.

As it is used herein, the term "medicinal product" refers to any substance used for preventing, diagnosis, alleviating, treating or curing diseases in human beings and animals. In the context of the present invention it also refers to a composition capable of generating an immune response against an aggregating protein given that it is causing a disease in human beings or animals. It therefore includes what is known as vaccine, as defined above in this specification.

### DIAGNOSTIC METHOD OF THE INVENTION

Another aspect of the invention relates to a method for the *in vitro* detection of an aggregating protein of the invention which comprises adding the antibody of the invention to a biological sample comprising or suspected of comprising said aggregating protein, and detecting and measuring a concentration of any complex formed between the antibody and the aggregating protein. In a preferred embodiment, said aggregating protein is α-synuclein. More preferably, the antibody is labeled with a detectable marker, and the presence of the complex formed between the antibody and α-synuclein is highlighted through detection of the marker. In another preferred embodiment, detection of the aggregating protein can be used for the diagnosis, prognosis or follow-up of a disease related with said aggregating protein. Said proteins and diseases have been described above in a non-limiting manner in the specification.

The terms "polynucleotide" and "nucleic acid" are used herein interchangeably, referring to polymeric forms of nucleotides of any length, both ribonucleotides (RNA) and deoxyribonucleotides (DNA).

The terms "amino acid sequence", "peptide", "oligopeptide", "polypeptide" and "protein" are used herein interchangeably, and refer to a polymeric form of amino acids of any length, which can be chemically or biochemically modified, coding or non-coding forms.

Throughout the description and the claims the word "comprises" and variants thereof do not intend to exclude other technical features, supplements, components or steps. For persons skilled in the art, other objects, advantages and features of the invention will be inferred in part from the description and in part from the practice of the invention. The following examples and drawings are provided by way of illustration and they are not meant to limit the present invention.

### EXAMPLES OF THE INVENTION

### Overexpression and purification of α-synuclein

Human wild-type α-synuclein (αSyn) was overexpressed in *E. coli* BL21 (DE3) cells using the plasmid pT7-7 and purified as previously described (Roodveldt et al., 2010. PLoS One 5(10): e13481; Roodveldt et al., 2013. PLoS One. 8(11):e79160). The purity (>95%) and monomeric state of the preparation was evaluated using 15% SDS-PAGE, mass spectrometry, native electrophoresis in 4-10% gradient (Lonza, Basel, Switzerland), as described (Roodveldt et al., PLoS One 2010). Before use and for the elimination of any possible endotoxin content, the protein in solution was subjected to filtration through centrifugation with Amicon Ultra-100 kDa (Merck Millipore Ltd., Carrigtwohill, IRL). The endotoxin content in the protein preparation was measured with the Oxisensor Chromogenic LAL Assay Kit (GenScript, Piscataway, USA), and the values obtained were <1 EU/mg protein. The αSyn protein concentration was determined with Micro BCA Reagent Kit (Pierce, Rockford, IL, USA).

### Preparation and characterization of α-synuclein oligomers

The preparation and characterization of αSyn soluble oligomers was carried out as described (Roodveldt et al., 2012. Biochemistry 51(44):8771-8) and the purified oligomeric fraction was stored at 4°C up to a maximum of 24 hrs. The protein concentration was determined as described above.

### Grp94 protein

Canine full-length recombinant Grp94 (Gp96) expressed in baculovirus (endotoxin-free) from Abcam (Cambridge, United Kingdom; #ab92290) was used.

### Hsp70 protein

Full-length human cytosolic recombinant Hsp70 (HSPA1A) protein overexpressed in *E. coli* and purified as described Was used (Roodveldt et al., 2009. EMBO J. 28(23):3758-70). The purity (>95%) of the preparation was evaluated using 15% SDS-PAGE. Before use and for the elimination of any possible endotoxin content, the protein in solution was subjected to filtration through centrifugation with Amicon Ultra-100 kDa (Merck Millipore Ltd., Carrigtwohill, Ireland). The endotoxin content in the protein preparation was measured with the Oxisensor Chromogenic LAL Assay Kit (GenScript, Piscataway, USA), and the values obtained were <1 EU/mg protein. The αSyn protein concentration was determined with Micro BCA Reagent Kit (Pierce, Rockford, IL, USA).

### SOD1 protein

Full-length human recombinant superoxide dismutase 1 (SOD1) protein overexpressed in *E. coli* purified by chromatography and with a purity >95% (ProSpec, Rehovot, Israel) was used.

### Mouse immunization protocols

### Protocol 1:

The mice were immunized with 5 µg of Grp94 and/or with 0.8 µg of non-aggregated αSyn. Preparation of protein solution for immunization: after incubating Grp94 and αSyn with a chaperone:αSyn molar ratio of 1:1 (in 20 mM HEPES, pH 7.5) in combination or alone (in the cases of controls) for 2 hours at 42°C, the solution was brought to a final volume of 150 µl of DPBS per mouse. It was then mixed with another 150 µl of complete Freund's adjuvant and the mixture was stirred vigorously until obtaining an emulsion. Each mouse was administered 300 µl of emulsion, distributed in three subcutaneous injections: one in the upper part of each rear paw and another in the cervical area. The same process was performed after 7 days but in this case using incomplete Freund's adjuvant. The mice were sacrificed 7 days after the second immunization.

### Protocol 2:

The mice were inoculated with 8 µg of Grp94 and/or 13 µg of αSyn oligomers, Preparation of protein solution for immunization: after incubating Grp94 and oligomeric αSyn with a chaperone:αSyn protomer molar ratio of 1:10 (in 20 mM HEPES, pH 7.5) in combination or alone (in the cases of controls) for 2 hours at 42°C, the solution was brought to the total volume of 100 µl per mouse. This solution, in the absence of adjuvant, was inoculated in a single subcutaneous injection applied in the lumbar area. The same process was repeated after 7 days and 21 days following the first immunization. Seven days after the last immunization, i.e., 28 days after the start of the process, the animals were sacrificed.

In both cases, between 500 and 700 µl of blood were drawn from the mice to obtain serum and analyze the presence of antibodies, and the spleen was extracted for extracting the splenocytes which were used for analyzing the regulatory T lymphocyte (Treg) population and the immune response against αSyn.

### Protocol 3:

The mice were immunized with a combination of 11 µg of Hsp70 and 2.5 µg of non-aggregated SOD1, or alternatively with 11 µg of Hsp70, or 2.5 µg of SOD1, or a vehicle, as controls. Preparation of protein solution for immunization: after incubating Hsp70 and SOD1 with a chaperone:SOD1 molar ratio of 1:1 (in 20 mM Tris, pH 7.4) in combination or alone (in the cases of controls) for 2 hours at room temperature, the solution was brought to a final volume of 150 µl of PBS per mouse. It was then mixed with another 150 µl of complete Freund's adjuvant and the mixture was emulsified transferring the mixture between two syringes for 5 minutes. Each mouse was administered 300 µl of emulsion, distributed in three subcutaneous injections: one in the upper part of each rear paw and another in the cervical area. The same process but in this case using incomplete Freund's adjuvant was performed after 14 and 28 days following the first injection in order to enhance the response.

### Protocol 4: immunization with aSyn and Hsp70 without adjuvant (manuscript)

The mice were immunized with 5 µg or 50 µg of Hsp70 (low and high doses, respectively) and/or with 1.06 µg or 10.6 µg of non-aggregated αSyn (low and high doses, respectively).

Preparation of protein solution for immunization: the proteins Hsp70 and αSyn were incubated with a chaperone:αSyn molar ratio of 1:1 (Tris/HCl 50 mM, pH 7.4; 150 mM KCI, 2 mM MgCl₂) in combination or alone (in the cases of controls) at 37°C for 2 hours in the presence of 4 mM ATP after which 2.5 mM ADP was added and incubated 2 more hours. The solution was brought to a final volume of 200 µl of PBS per mouse and the mice were administered a single subcutaneous injection in the lumbar area. The same process was performed after 7 days. The mice were sacrificed 7 days after the second immunization.

### Adoptive splenocytes transfer

After the immunization process according to protocols 1 and 2 and sacrificing the immunized mice, an equal number of splenocytes originating from each mouse of one and the same group was mixed and 10 million splenocytes were transferred to each recipient mouse by means of a single intraperitoneal injection. After the transfer, the mice were given a leeway of 10 days before starting the treatment with MPTP in order to allow the splenocytes to establish themselves in the host.

### Treatment of mice with MPTP

The animals receiving the adoptive transfer were chronically treated with MPTP as described above (Villadiego J. *et al.*, 2005. 25, 4091 - 4098). After treatment, the animals were left to recover for one month before proceeding with the behavioral tests.

### Behavioral evaluations of the animals treated with MPTP

Behavioral analysis (footprinting) was carried out after leaving the mice to recover for one month after chronic treatment with MPTP and amphetamines were then administered following the protocol previously described in Muñoz-Manchado A.B. et al, 2013. Neurobiol Aging 34(3):902-15.

### Sacrificing treated animals and extraction of samples

After treatment with MPTP and subsequent recovery and behavioral evaluation, the mice were left for 10 days to assure the complete elimination of amphetamines and to dismiss any undesired interaction. The mice were sacrificed and their blood was drawn to obtain serum, and the spleen was extracted for isolating splenocytes.

### Evaluation of the immune response in immunized and recipient animals

### - Determination of Treg cells using flow cytometry:

Splenocytes were isolated from the spleen using a standard protocol by means of perfusion described above. 10⁶ cells were labeled following the manufacturer's recommendations using the Mouse Foxp3 Buffer Set (BD Pharmigen^{™}) and anti-CD4 FITC, anti-CD25 APC and anti-Foxp3 PE antibodies, all from BD Pharmigen^{™}. They were analyzed using a FACS Calibur flow cytometer and the Cell Quest Pro software (BD Pharmigen^{™}).

### - In vitro stimulation with antigen and determination of cytokines (IFN-γ, IL-10 and IL-17):

3x10⁶ cells were divided into three wells and stimulated, depending on the case, with culture medium alone, αSyn (15 µg per well), SOD1 (20 µg per well), or LPS (0.5 µg per well) in 1 ml of culture medium. After 36 hours, the supernatants were collected and centrifuged to eliminate cell debris and cells and kept at -80°C for subsequent cytokine analysis.

To measure IFN-γ, IL-10 and IL-17, specific ELISA kits, *BD OptEIA^{™} Set Mouse IFN-gamma, BD OptEIA^{™} Set Mouse IL-10* (BD Pharmigen^{™}) and *BD OptEIA^{™} Set Mouse IL17*(BD Pharmigen^{™}) were used and ELISAs were performed following the manufacturer's instructions.

### - Determination of total IgG and anti-αSyn or anti-SOD1 in serum:

The blood drawn while sacrificing the animals was left at room temperature for one hour and at 4°C for another hour, thereby allowing it to clot. It was then centrifuged at 21,000 g for 15 minutes in order to obtain cell-free serum which was frozen at -80°C for subsequent analysis. To measure the IgG1 and IgG2a levels as well as total IgM and IgG levels, the sera were diluted 1:240000 in PBS, 100 µl per well were transferred to a MaxiSorp 96-well plate (NUNC) and incubated for 1 hour at 37°C, they were then washed with PBST (0.05% Tween 20), blocked for 1 hour at 37°C with Assay Diluent (BD Pharmigen^{™}). After washing the wells again, a specific anti-IgG antibody (Promega), IgG1 (AbCam), IgG2a (AbCAm) or IgM (Miltenyi) conjugated to a HRP diluted 1:4000 in Assay Diluent was used and incubated for 1 hour at 37°C. After washing the wells, TMB Substrate Reagent (BD Pharmigen^{™}) was used to determine the amount of IgG, IgG1, IgG2a or IgM present in the sera.

A similar protocol but with variations was used to measure the amount of specific anti-αSyn antibodies. Briefly, the plate was pretreated either with αSyn diluted in PBS or an anti-αSyn antibody (N19, Santa Cruz) at 1 µg per well in 100 µl and incubated 2 hours at 37°C, the wells were then washed and blocked like in the preceding protocol. In the case of having pretreated with anti-αSyn antibody, the wells were incubated with 10 µg per well of αSyn for 1 hour at 37°C and washed with PBST. In both cases, 100 µl per well were incubated with sera diluted 1:50 in Assay Diluent for an hour at 37°C, then washing, detection and development were carried out in the same manner as in the preceding protocol.

A similar protocol but with variations was used to measure the amount of specific anti-SOD1 antibodies. Briefly, the plate was pretreated with SOD1 diluted in PBS at 0.5 µg per well in 100 µl and incubated 2 hours at 37°C, the wells were then washed and blocked like in the preceding protocol and 100 µl per well were incubated with sera diluted 1:50 in Assay Diluent for an hour at 37°C, then washing, detection and development were carried out in the same manner as in the preceding protocol.

### Results

### In vitro stimulation of splenocytes with a Grp94/Gp96 and α-synuclein combination to evaluate their immune response-inducing potential

In order to promote an immune response against α-synuclein (αSyn) which may potentially act as a neuroprotector in the context of a synucleinopathy, the effect of an αSyn and Grp94 combination was first evaluated *in vitro.* To that end, a culture of murine splenocytes with a Grp94 and αSyn combination (1:1 molar ratio), or the equivalent amounts of Grp94 or aSyn as controls, were incubated for 24 hrs. After separating the cells, they were analyzed using flow cytometry quantifying the expression of cell activation surface markers, particularly MHC II and CD86, observing a 50% reduction and a 2-fold increase in the expression of these markers in the case of stimulation with αSyn and Grp94, respectively, with respect to the stimulation control with buffer solution (Figure 1A). On the other hand, the stimulation of splenocytes with an αSyn and Grp94 combination (αSyn+Grp94) caused a 4.5-fold increase in the expression of these markers with respect to the control (Figure 1 A). Furthermore, the levels of cytokines IFN-γ, IL-10, and IL-17 were measured in the supernatants using ELISA (Figures 1B, 1C, 1D), also detecting relative increases for IFN-γ and IL-10 in the case of stimulation with the combination of proteins (Figures 1B and 1C).

### Immunization of mice with a Grp94/Gp96 and α-synuclein combination, adoptive transfer of their splenocytes to recipient mice and subsequent administration of MPTP (model of Parkinson's disease) to evaluate its PD protection potential

Having observed a different response of the splenocytes in culture as a result of stimulation with the combination of proteins, healthy C57BL/6 male mice 5-7 weeks of age (5 mice per group) were immunized with said preparations (αSyn, Grp94, an αSyn and Grp94 combination, and buffer as control) to evaluate the *in vivo* response. To that end, two vaccination protocols were designed (protocol 1 and 2). Seven days after finish applying immunization protocol 1, the mice were sacrificed for analyzing the splenocytes and serum. First, no changes were observed in the regulatory T-cell (Treg) content in relation to the total CD4⁺ cells (Figure 2A). Next, the splenocytes were cultured and alternatively incubated with αSyn or with buffer solution as a reference, after which the supernatants were separated for analyzing the levels of certain cytokines (Figures 2B and 2C). In this case, a different response was also observed for the αSyn and Grp94 combination, proving a minimum reduction in the levels of IFN-γ (Figure 2B) and equivalent values of IL-10 (Figure 2C) for αSyn+Grp94, as a result of the *in vitro* stimulation of splenocytes with αSyn. Finally, the levels and proportion of anti-αSyn antibodies in serum were measured. First, and surprisingly, a significant reduction in the anti-αSyn IgG antibody content was observed in the case of mice immunized with αSyn alone (Figure 2D), whereas immunization with αSyn+Grp94 produced intermediate levels of anti-αSyn IgG antibodies in relation to immunizations with αSyn and Grp94 (Figure 2D).

On the other hand, as a strategy to evaluate the synucleinopathy therapeutic potential, splenocytes originating from the mice immunized according to vaccination protocol 1 were transferred to healthy C57BL/6 mice, chronic treatment with MPTP was applied to such mice for 3 months as a model of Parkinson's disease (see Materials and Methods). Forty-three days after the administration of the last dose of MPTP for all the groups (except for the 'saline control') and after having performed the behavioral tests, the mice were sacrificed and the spleens and serum extracted for analysis. The isolated splenocytes were analyzed using flow cytometry and cultured and stimulated *in vitro* with αSyn or buffer for analyzing the cytokine-mediated response (Figure 3 A-D). According to the results, the animals treated with MPTP which have not received splenocytes (MPTP control) had lower levels of Treg cells than animals not treated with MPTP or with splenocytes ('healthy' or 'saline' control) (Figure 3A). The recipient animals treated with splenocytes originating from animals immunized with αSyn or Grp94 showed values somewhat greater and less than 'saline', respectively, whereas the recipients of splenocytes from mice immunized with αSyn+Grp94 showed the highest percentages of Treg (Figure 3A). The same but opposite profile was observed for effector T cells (Teff, Figure 3B), with coinciding levels for 'αSyn+Grp94' and 'saline' mice.

On the other hand, the response of the cultured splenocytes with respect to the stimulation with αSyn or LPS was evaluated *in vitro.* In this case, a reduction was observed in the supernatant levels of IFN-γ and IL-10 for the splenocytes from the recipients of cells of animals immunized with αSyn+Grp94, in relation to the equivalents of αSyn and Grp94 alone (Figures 3B and 3C), demonstrating a different effect of vaccination with the αSyn and Grp94 combination.

Finally, the results of the behavioral test (footprinting) in recipient animals showed minimum and maximum values of the measured parameter (stride variation) of average ± S.E.M. of 1.43 ± 0.25 (N=5) and 0.76 ± 0.26 (N=4) for 'MPTP' and 'saline' controls, respectively, and a value close to the 'healthy' control of 0.83±0.17 (N=7) for treatment with 'αSyn+Grp94', less than that obtained with treatment with 'αSyn' or 'Grp94' alone (Figure 3E).

A second immunization protocol (protocol 2) was then tested in healthy C57BL/6 male mice 5-7 weeks of age (5 mice per group), in which 3 immunizations were performed in the absence of adjuvant, and oligomeric/aggregated αSyn was used. In this case, no differences were observed in the Treg content with respect to the total CD4⁺ cells (Figure 4A), although a certain tendency towards a higher Teff cell content in mice immunized with 'Ol. αSyn+Grp94' with respect to those immunized with Ol. αSyn and Grp94 separately was detected (Figure 4B). Like before, the levels of IFN-γ in the supernatants of splenocytes cultured and stimulated *in vitro* with αSyn or buffer solution as a reference were measured, observing an increase in the samples originating from immunization with Ol. αSyn, and values similar to the control for 'Grp94' and 'Ol. αSyn+Grp94' (Figure 4C). Furthermore, the results of serum anti-αSyn IgG antibody content in relation to total IgG, and of anti-αSyn IgM antibody content showed higher levels in the case of mice immunized with 'Ol. αSyn+Grp94' than those immunized with Ol. αSyn or Grp94 separately (Figures 4D and 4E).

Like before, splenocytes from the mice immunized according to protocol 2 were transferred to healthy male C57BL/6 mice, MPTP was then administered to said mice for 3 months as a model of Parkinson's diseases. The results of the behavioral test (footprinting) in the recipient animals (Figure 5) showed minimum and maximum values of the measured parameter (stride variation) of average ± S.E.M. of 1.79 ± 0.26 (N=5) and 1.06 ± 0.16 (N=5) for 'MPTP' and 'saline' controls, respectively, and a value similar to that of the 'healthy' control of 1.02±0.23 (N=5) for treatment with 'αSyn+Grp94', differing from that obtained with the treatment with 'αSyn' or 'Grp94' alone (1.98 ± 0.39, N=5; 2.01 ± 0.55, N=5) (Figure 5).

These results demonstrate that immunization with a combination of αSyn (both in the non-aggregated and oligomeric forms) and the chaperone Grp94 produces different effects on the immune response of the immunized mice and that the adoptive transfer of splenocytes originating from said immunization is capable of preventing or counteracting the development of symptoms related to Parkinson's disease in the recipient mice.

### Vaccination of a transgenic mice model of ALS (SOD1 G93A) with an Hsp70 and SOD1 combination to evaluate their ALS therapeutic/prophylactic capacity

A preliminary experiment was conducted to evaluate the therapeutic potential of immunization with a combination of SOD1 and the chaperone Hsp70. To that end, a transgenic mice model of ALS (Tg SOD1 G93A, Jackson Laboratories Inc., USA) was used with mice 9 weeks of age (3 mice per group; N=3) that were immunized according to protocol 3. The mice were then carefully monitored by means of measuring various standard parameters of murine models of ALS, starting before the onset of the disease (typically in week 12-13), and until the termination of the disease according to the 'end point' criterion (typically in week 18-19, when the animal's weight drops up to <80% with respect to its maximum weight). After being sacrificed at the 'end point', the spleen and blood were extracted for analyzing the splenocytes and serum.

The isolated splenocytes were analyzed using flow cytometry to analyze the CD4⁺ cell, regulatory T cell (Treg) and effector T cell (Teff) populations (Figure 6). According to this analysis, immunization with SOD1+Hsp70 produces an increase in the CD4⁺ population and the Teff cell content with respect to animals treated with a vehicle, whereas no changes are observed in the Treg cell content. On the other hand, the isolated splenocytes were stimulated *in vitro* with SOD1 for quantifying IFN-γ secretion, as an indicator of the type of antigen-specific response (Figure 6). The data obtained shows a 3-fold increase of the levels of IFN-γ secreted upon stimulation with SOD1 with respect to the control. Finally, the levels of IgG antibodies in serum were measured to analyze the IgGl/IgG2a ratio in the different groups. The results of this quantification indicate an increase of said quotient in the case of the SOD1+Hsp70 group both for total IgG antibodies, and for the SOD1-specific antibody population (Figure 6), suggesting the importance of Th2-type or 'neuroprotective' response in the context of neurodegenerative diseases.

On the other hand, analysis of the weight evolution and survival of each mouse allows observing a certain protection against weight loss in mice immunized with SOD1+Hsp70 with respect to controls (Figure 7), as well as an increase in survival (Figure 7). These preliminary results indicate a different immune response induced by SOD1+Hsp70 and a potential beneficial effect of this vaccination strategy against ALS.

### Immunization of C57BL/6 mice with an adjuvant-free αSyn and Hsp70 complex/combination and characterization of the immune response generated

First, the formation of a complex between αSyn and Hsp70 was demonstrated by means of PAGE-Western blot and Biacore after pre-incubating the proteins in buffer. Healthy C57BL/6 mice (N=6-7) were immunized with two different doses ('low' and 'high' doses) of said preparation or with the corresponding controls prepared in the same manner, in the absence of adjuvant, according to protocol 4. Seven days after the end of the immunization protocol, the mice were sacrificed and the spleen and blood were extracted for isolating splenocytes and obtaining serum. The analysis of splenocytes using flow cytometry showed that, while immunization with αSyn or Hsp70 alone causes an increase in the Treg population, the αSyn+Hsp70 group is capable of suppressing said effect, keeping that population within the 'normal' levels with respect to the control (Figure 8). On the other hand, a similar different effect for αSyn+Hsp70 was observed in the levels of anti-αSyn IgG antibodies, indicating that the response induced by the complex may be capable of suppressing an antigen-specific humoral response for the autoantigen αSyn (Figure 8). Finally, the levels of IL-10 and IFN-γ in serum were quantified, and the IL-10/IFN-γ paired quotients for each mouse (Figure 8) were analyzed. The results obtained clearly demonstrate a different increase of said quotient for animals immunized with the αSyn+Hsp70 combination, indicating that the response induced is a Th2-type or 'neuroprotective' response in the context of neurodegenerative diseases.

## Claims

1. A composition comprising:
a) a mixture of peptides/polypeptides/proteins comprising i) at least one peptide/polypeptide/protein the amino acid sequence of which corresponds with an aggregating peptide/polypeptide/protein ("sequence A of the invention") or with any of the biologically active variants and fragments of said sequence, and ii) a peptide/polypeptide/protein the amino acid sequence of which corresponds with a molecular chaperone ("sequence B of the invention") or with any of the biologically active variants and fragments of said sequence; and/or
b) a fusion peptide/polypeptide/protein, "fusion amino acid sequence of the invention", comprising i) the amino acid sequences of at least one aggregating peptide/polypeptide/protein ("sequence A of the invention) or any of the biologically active variants and fragments thereof, and ii) the sequences of at least one peptide/polypeptide/protein the amino acid sequence of which corresponds with a chaperone ("sequence B of the invention") or with any of the biologically active variants and fragments thereof.

2. The composition according to the preceding claim, where the aggregating peptide/polypeptide/protein is selected from the list consisting of α-synuclein, huntingtin, beta-amyloid peptide, TDP-43, FUS, tau, prion protein, fibronectin and SOD1.

3. The composition according to any of claims 1-2, where the aggregating peptide/polypeptide/protein is α-synuclein or any of the biologically active variants and fragments thereof.

4. The composition according to any of claims 1-3, where the chaperone is selected from the list consisting of the Hsp90 family, Hsp70 family, immunophilin family, peptidase C56 family, PPlase family, 14-3-3 family, small heat-shock protein group, small GTPase family, Hsp40 (DnaJ) family, and clusterin, Grp170, calreticulin, Hsp105, CHIP, alpha-crystallin or any of the combinations thereof.

5. The composition according to any of claims 1-4, where the chaperone belongs to the Hsp90 family and is selected from the list consisting of Grp94, Grp96 and Hsp90 or any of the combinations thereof.

6. The composition according to any of claims 1-4, where the chaperone belongs to the Hsp70 family and is selected from the list consisting of Hsp70, Hsc70 and Grp75 or any of the combinations thereof.

7. The composition according to any of claims 1-4, where the chaperone belongs to the immunophilin family and is selected from the list consisting of FKBP12 and FKBP4/FKBP52 or the combination thereof.

8. The composition according to any of claims 1-4, where the chaperone is DJ1/PARK7.

9. The composition according to any of claims 1-4, where the chaperone belongs to the PPlase family and is selected from the list consisting of Pin1, CypA, Cyp40 or any of the combinations thereof.

10. The composition according to any of claims 1-4, where the chaperone belongs to the 14-3-3 family and is selected from the list consisting of 14-3-3 gamma, 14-3-3 epsilon, 14-3-3 tau or any of the combinations thereof.

11. The composition according to any of claims 1-4, where the chaperone is Hsp27.

12. The composition according to any of claims 1-4, where the chaperone is Rab11A.

13. The composition according to any of claims 1-4, where the chaperone of the Hsp40 (DnaJ) family is selected from Hsp40 and CSP or the combination thereof.

14. The composition according to any of claims 1-4, where the chaperone is selected from the list consisting of clusterin, Grp170, calreticulin, Hsp105, CHIP, alpha-crystallin or any of the combinations thereof.

15. The composition according to claim 1, where the aggregating peptide/polypeptide/protein is α-synuclein or SOD1 and the chaperone is selected from any of those defined in claims 4-14.

16. The composition according to any of claims 1-4, where the chaperone is Grp94 or any of its biologically active variants, and combinations thereof.

17. The composition according to claim 1, where the aggregating peptide/polypeptide/protein is α-synuclein and the chaperone is Grp94, Grp96 or Hsp70, or where the aggregating peptide/polypeptide/protein is SOD1 and the chaperone is Hsp70.

18. The composition according to any of claims 1-17, where at least one of the sequences A and/or B are recombinant sequences.

19. A nucleotide sequence comprising one or more nucleotide sequences encoding the fusion protein as defined in any of claims 1-18.

20. An expression vector comprising the nucleotide sequence of the preceding claim.

21. A host cell comprising the expression vector according to claim 20 or the nucleotide sequence of claim 19.

22. An antibody or a fragment thereof capable of binding to one of sequences A or B, or to the fusion amino acid sequence according to any of the preceding claims.

23. An antibody or a fragment thereof obtained or obtainable after the immunization of an animal, or of the cells of an animal, with the composition as defined in any of claims 1-18.

24. The antibody according to the preceding claim, where the animal used for the immunization is a mammal.

25. An immunotherapeutic cellular composition comprising at least one isolated, activated antigen presenting cell (APC), wherein said APC is obtained from a patient diagnosed with a disease caused by an aggregating protein of the invention, and wherein said APC is stimulated by *ex vivo* exposure to a composition as defined in any of claims 1-18.

26. The immunotherapeutic cellular composition according to the preceding claim, where the isolated, activated antigen presenting cell or APC is a dendritic cell (DC).

27. A pharmaceutical composition comprising the composition according to any of claims 1-18, the nucleotide sequence according to claim 19, the expression vector according to claim 20, the host cell according to claim 21, the antibody according to any of claims 22-24, or the immunotherapeutic cellular composition according to any of claims 25-26.

28. The pharmaceutical composition according to the preceding claim further comprising a pharmaceutically acceptable vehicle.

29. The pharmaceutical composition according to any of claims 27-28, where said composition is a vaccine optionally comprising an adjuvant.

30. A combined preparation comprising sequences A and B as they are described in any of claims 1-18.

31. Use of the composition according to any of claims 1-18, the nucleotide sequence according to claim 19, the expression vector according to claim 20, the host cell according to claim 21, the antibody according to any of claims 22-24, or the immunotherapeutic cellular composition according to any of claims 25-26, in the preparation of a medicinal product.

32. Use of the composition according to any of claims 1-18, the nucleotide sequence according to claim 19, the expression vector according to claim 20, the host cell according to claim 21, the antibody according to any of claims 22-24, or the immunotherapeutic cellular composition according to any of claims 25-26, in the preparation of a medicinal product, where the medicinal product is a vaccine.

33. Use of the composition according to any of claims 1-18, the nucleotide sequence according to claim 19, the expression vector according to claim 20, the host cell according to claim 21, the antibody according to any of claims 22-24, or the immunotherapeutic cellular composition according to any of claims 25-26, in the preparation of a medicinal product for the treatment or prevention of aggregating protein-related diseases or disorders.

34. Use of the combined preparation according to claim 30 comprising sequence A and sequence B in the preparation of a medicinal product for combined, simultaneous or sequential administration, for the treatment or prevention of aggregating protein-related diseases or disorders.

35. Use according to any of claims 33-34, where the aggregating protein is α-synuclein and the chaperone is selected from any of those defined in claims 4-14.

36. Use according to any of claims 33-35, where the aggregating protein-related disease or disorder is selected from the list consisting of: Parkinson's disease (PD), Lewy body dementia (LBD), the Lewy body variant of Alzheimer's disease, multiple system atrophy (MSA), Alzheimer's disease, pure autonomic failure (PAF), Down syndrome, amyotrophic lateral sclerosis (ALS), frontotemporal dementia, Gaucher disease, Huntington's disease, type II diabetes, prion disease, Creutzfeldt-Jakob disease, multiple sclerosis, Gerstmann-Sträussler-Scheinker syndrome, Kuru, fatal familial insomnia, cerebrovascular amyloidosis, glaucoma, age-related macular degeneration, neurodegeneration due to age-related protein aggregation, psychiatric syndromes, schizophrenia and/or schizophrenia-like disorders.

37. Use of the composition according to any of claims 1-18, the nucleotide sequence according to claim 19, the expression vector according to claim 20, the host cell according to claim 21, the antibody according to any of claims 22-24, or the immunotherapeutic cellular composition according to any of claims 25-26, where sequence A is α-synuclein or any of the biologically active variants and fragments thereof, sequence B is the chaperone as defined in any of claims 4-14, and the disease is selected from the list consisting of: Parkinson's disease (PD), Lewy body dementia, multiple system atrophy (MSA), Gaucher disease, pure autonomic failure (PAF), and Alzheimer's disease.

38. Use of the composition according to any of claims 1-18, the nucleotide sequence according to claim 19, the expression vector according to claim 20, the host cell according to claim 21, the antibody according to any of claims 22-24, or the immunotherapeutic cellular composition according to any of claims 25-26, where sequence A is α-synuclein or any of the biologically active variants and fragments thereof, sequence B is the chaperone Grp94 or any of the biologically active variants and fragments thereof, in the preparation of a medicinal product for the treatment or prevention of Parkinson's disease.

39. Use according to any of claims 33-34, where the aggregating protein is SOD1 and the chaperone is selected from any of those defined in claims 4-14, preferably the chaperone is Hsp70.

40. Use according to claim 39, where the SOD1-related disease or disorder is ALS.


SEQUENCE LISTING

<110>  Fundación Pública Andaluza Porgreso y Salud
       Universidad de Sevilla

<120>  Combinaciones de proteínas agregantes y chaperonas moleculares
       para el tratamiento de proteinopatías o enfermedades
       conformacionales.

<130>  CABIMER-13003

<160>  33

<170>  PatentIn version 3.5

<210>  1
<211>  140
<212>  PRT
<213>  Homo sapiens

<400>  1

<210>  2
<211>  3142
<212>  PRT
<213>  Homo sapiens

<400>  2

Met Ala Thr Leu Glu Lys Leu Met Lys Ala Phe Glu Ser Leu Lys Ser
1               5                   10                  15

Phe Gln Gln Gln Gln Gln Gln Gln Gln Gln Gln Gln Gln Gln Gln Gln
            20                  25                  30

Gln Gln Gln Gln Gln Gln Pro Pro Pro Pro Pro Pro Pro Pro Pro Pro
        35                  40                  45

Pro Gln Leu Pro Gln Pro Pro Pro Gln Ala Gln Pro Leu Leu Pro Gln
    50                  55                  60

Pro Gln Pro Pro Pro Pro Pro Pro Pro Pro Pro Pro Gly Pro Ala Val
65                  70                  75                  80

Ala Glu Glu Pro Leu His Arg Pro Lys Lys Glu Leu Ser Ala Thr Lys
                85                  90                  95

Lys Asp Arg Val Asn His Cys Leu Thr Ile Cys Glu Asn Ile Val Ala
            100                 105                 110

Gln Ser Val Arg Asn Ser Pro Glu Phe Gln Lys Leu Leu Gly Ile Ala
        115                 120                 125

Met Glu Leu Phe Leu Leu Cys Ser Asp Asp Ala Glu Ser Asp Val Arg
    130                 135                 140

Met Val Ala Asp Glu Cys Leu Asn Lys Val Ile Lys Ala Leu Met Asp
145                 150                 155                 160

Ser Asn Leu Pro Arg Leu Gln Leu Glu Leu Tyr Lys Glu Ile Lys Lys
                165                 170                 175

Asn Gly Ala Pro Arg Ser Leu Arg Ala Ala Leu Trp Arg Phe Ala Glu
            180                 185                 190

Leu Ala His Leu Val Arg Pro Gln Lys Cys Arg Pro Tyr Leu Val Asn
        195                 200                 205

Leu Leu Pro Cys Leu Thr Arg Thr Ser Lys Arg Pro Glu Glu Ser Val
    210                 215                 220

Gln Glu Thr Leu Ala Ala Ala Val Pro Lys Ile Met Ala Ser Phe Gly
225                 230                 235                 240

Asn Phe Ala Asn Asp Asn Glu Ile Lys Val Leu Leu Lys Ala Phe Ile

245                 250                 255

Ala Asn Leu Lys Ser Ser Ser Pro Thr Ile Arg Arg Thr Ala Ala Gly
            260                 265                 270

Ser Ala Val Ser Ile Cys Gln His Ser Arg Arg Thr Gln Tyr Phe Tyr
        275                 280                 285

Ser Trp Leu Leu Asn Val Leu Leu Gly Leu Leu Val Pro Val Glu Asp
    290                 295                 300

Glu His Ser Thr Leu Leu Ile Leu Gly Val Leu Leu Thr Leu Arg Tyr
305                 310                 315                 320

Leu Val Pro Leu Leu Gln Gln Gln Val Lys Asp Thr Ser Leu Lys Gly
                325                 330                 335

Ser Phe Gly Val Thr Arg Lys Glu Met Glu Val Ser Pro Ser Ala Glu
            340                 345                 350

Gln Leu Val Gln Val Tyr Glu Leu Thr Leu His His Thr Gln His Gln
        355                 360                 365

Asp His Asn Val Val Thr Gly Ala Leu Glu Leu Leu Gln Gln Leu Phe
    370                 375                 380

Arg Thr Pro Pro Pro Glu Leu Leu Gln Thr Leu Thr Ala Val Gly Gly
385                 390                 395                 400

Ile Gly Gln Leu Thr Ala Ala Lys Glu Glu Ser Gly Gly Arg Ser Arg
                405                 410                 415

Ser Gly Ser Ile Val Glu Leu Ile Ala Gly Gly Gly Ser Ser Cys Ser
            420                 425                 430

Pro Val Leu Ser Arg Lys Gln Lys Gly Lys Val Leu Leu Gly Glu Glu
        435                 440                 445

Glu Ala Leu Glu Asp Asp Ser Glu Ser Arg Ser Asp Val Ser Ser Ser
    450                 455                 460

Ala Leu Thr Ala Ser Val Lys Asp Glu Ile Ser Gly Glu Leu Ala Ala
465                 470                 475                 480

Ser Ser Gly Val Ser Thr Pro Gly Ser Ala Gly His Asp Ile Ile Thr
                485                 490                 495

Glu Gln Pro Arg Ser Gln His Thr Leu Gln Ala Asp Ser Val Asp Leu
        500                 505                 510

Ala Ser Cys Asp Leu Thr Ser Ser Ala Thr Asp Gly Asp Glu Glu Asp
    515                 520                 525

Ile Leu Ser His Ser Ser Ser Gln Val Ser Ala Val Pro Ser Asp Pro
    530                 535                 540

Ala Met Asp Leu Asn Asp Gly Thr Gln Ala Ser Ser Pro Ile Ser Asp
545                 550                 555                 560

Ser Ser Gln Thr Thr Thr Glu Gly Pro Asp Ser Ala Val Thr Pro Ser
                565                 570                 575

Asp Ser Ser Glu Ile Val Leu Asp Gly Thr Asp Asn Gln Tyr Leu Gly
            580                 585                 590

Leu Gln Ile Gly Gln Pro Gln Asp Glu Asp Glu Glu Ala Thr Gly Ile
        595                 600                 605

Leu Pro Asp Glu Ala Ser Glu Ala Phe Arg Asn Ser Ser Met Ala Leu
    610                 615                 620

Gln Gln Ala His Leu Leu Lys Asn Met Ser His Cys Arg Gln Pro Ser
625                 630                 635                 640

Asp Ser Ser Val Asp Lys Phe Val Leu Arg Asp Glu Ala Thr Glu Pro
                645                 650                 655

Gly Asp Gln Glu Asn Lys Pro Cys Arg Ile Lys Gly Asp Ile Gly Gln
            660                 665                 670

Ser Thr Asp Asp Asp Ser Ala Pro Leu Val His Cys Val Arg Leu Leu
        675                 680                 685

Ser Ala Ser Phe Leu Leu Thr Gly Gly Lys Asn Val Leu Val Pro Asp
    690                 695                 700

Arg Asp Val Arg Val Ser Val Lys Ala Leu Ala Leu Ser Cys Val Gly
705                 710                 715                 720

Ala Ala Val Ala Leu His Pro Glu Ser Phe Phe Ser Lys Leu Tyr Lys
                725                 730                 735

Val Pro Leu Asp Thr Thr Glu Tyr Pro Glu Glu Gln Tyr Val Ser Asp
            740                 745                 750

Ile Leu Asn Tyr Ile Asp His Gly Asp Pro Gln Val Arg Gly Ala Thr
        755                 760                 765

Ala Ile Leu Cys Gly Thr Leu Ile Cys Ser Ile Leu Ser Arg Ser Arg
    770                 775                 780

Phe His Val Gly Asp Trp Met Gly Thr Ile Arg Thr Leu Thr Gly Asn
785                 790                 795                 800

Thr Phe Ser Leu Ala Asp Cys Ile Pro Leu Leu Arg Lys Thr Leu Lys
                805                 810                 815

Asp Glu Ser Ser Val Thr Cys Lys Leu Ala Cys Thr Ala Val Arg Asn
            820                 825                 830

Cys Val Met Ser Leu Cys Ser Ser Ser Tyr Ser Glu Leu Gly Leu Gln
        835                 840                 845

Leu Ile Ile Asp Val Leu Thr Leu Arg Asn Ser Ser Tyr Trp Leu Val
    850                 855                 860

Arg Thr Glu Leu Leu Glu Thr Leu Ala Glu Ile Asp Phe Arg Leu Val
865                 870                 875                 880

Ser Phe Leu Glu Ala Lys Ala Glu Asn Leu His Arg Gly Ala His His
                885                 890                 895

Tyr Thr Gly Leu Leu Lys Leu Gln Glu Arg Val Leu Asn Asn Val Val
            900                 905                 910

Ile His Leu Leu Gly Asp Glu Asp Pro Arg Val Arg His Val Ala Ala
        915                 920                 925

Ala Ser Leu Ile Arg Leu Val Pro Lys Leu Phe Tyr Lys Cys Asp Gln
    930                 935                 940

Gly Gln Ala Asp Pro Val Val Ala Val Ala Arg Asp Gln Ser Ser Val
945                 950                 955                 960

Tyr Leu Lys Leu Leu Met His Glu Thr Gln Pro Pro Ser His Phe Ser
                965                 970                 975

Val Ser Thr Ile Thr Arg Ile Tyr Arg Gly Tyr Asn Leu Leu Pro Ser
            980                 985                 990

Ile Thr Asp Val Thr Met Glu Asn  Asn Leu Ser Arg Val  Ile Ala Ala
        995                 1000                 1005

Val Ser His Glu Leu Ile Thr Ser Thr Thr Arg Ala Leu Thr Phe
    1010                1015                1020

Gly Cys Cys Glu Ala Leu Cys Leu Leu Ser Thr Ala Phe Pro Val
    1025                1030                1035

Cys Ile Trp Ser Leu Gly Trp His Cys Gly Val Pro Pro Leu Ser
    1040                1045                1050

Ala Ser Asp Glu Ser Arg Lys Ser Cys Thr Val Gly Met Ala Thr
    1055                1060                1065

Met Ile Leu Thr Leu Leu Ser Ser Ala Trp Phe Pro Leu Asp Leu
    1070                1075                1080

Ser Ala His Gln Asp Ala Leu Ile Leu Ala Gly Asn Leu Leu Ala
    1085                1090                1095

Ala Ser Ala Pro Lys Ser Leu Arg Ser Ser Trp Ala Ser Glu Glu
    1100                1105                1110

Glu Ala Asn Pro Ala Ala Thr Lys Gln Glu Glu Val Trp Pro Ala
    1115                1120                1125

Leu Gly Asp Arg Ala Leu Val Pro Met Val Glu Gln Leu Phe Ser
    1130                1135                1140

His Leu Leu Lys Val Ile Asn Ile Cys Ala His Val Leu Asp Asp
    1145                1150                1155

Val Ala Pro Gly Pro Ala Ile Lys Ala Ala Leu Pro Ser Leu Thr
    1160                1165                1170

Asn Pro Pro Ser Leu Ser Pro Ile Arg Arg Lys Gly Lys Glu Lys
    1175                1180                1185

Glu Pro Gly Glu Gln Ala Ser Val Pro Leu Ser Pro Lys Lys Gly
    1190                1195                1200

Ser Glu Ala Ser Ala Ala Ser Arg Gln Ser Asp Thr Ser Gly Pro
    1205                1210                1215

Val Thr Thr Ser Lys Ser Ser Ser Leu Gly Ser Phe Tyr His Leu
    1220                1225                1230

Pro Ser Tyr Leu Lys Leu His Asp Val Leu Lys Ala Thr His Ala

1235                1240                1245

Asn Tyr Lys Val Thr Leu Asp Leu Gln Asn Ser Thr Glu Lys Phe
    1250                1255                1260

Gly Gly Phe Leu Arg Ser Ala Leu Asp Val Leu Ser Gln Ile Leu
    1265                1270                1275

Glu Leu Ala Thr Leu Gln Asp Ile Gly Lys Cys Val Glu Glu Ile
    1280                1285                1290

Leu Gly Tyr Leu Lys Ser Cys Phe Ser Arg Glu Pro Met Met Ala
    1295                1300                1305

Thr Val Cys Val Gln Gln Leu Leu Lys Thr Leu Phe Gly Thr Asn
    1310                1315                1320

Leu Ala Ser Gln Phe Asp Gly Leu Ser Ser Asn Pro Ser Lys Ser
    1325                1330                1335

Gln Gly Arg Ala Gln Arg Leu Gly Ser Ser Ser Val Arg Pro Gly
    1340                1345                1350

Leu Tyr His Tyr Cys Phe Met Ala Pro Tyr Thr His Phe Thr Gln
    1355                1360                1365

Ala Leu Ala Asp Ala Ser Leu Arg Asn Met Val Gln Ala Glu Gln
    1370                1375                1380

Glu Asn Asp Thr Ser Gly Trp Phe Asp Val Leu Gln Lys Val Ser
    1385                1390                1395

Thr Gln Leu Lys Thr Asn Leu Thr Ser Val Thr Lys Asn Arg Ala
    1400                1405                1410

Asp Lys Asn Ala Ile His Asn His Ile Arg Leu Phe Glu Pro Leu
    1415                1420                1425

Val Ile Lys Ala Leu Lys Gln Tyr Thr Thr Thr Thr Cys Val Gln
    1430                1435                1440

Leu Gln Lys Gln Val Leu Asp Leu Leu Ala Gln Leu Val Gln Leu
    1445                1450                1455

Arg Val Asn Tyr Cys Leu Leu Asp Ser Asp Gln Val Phe Ile Gly
    1460                1465                1470

Phe Val Leu Lys Gln Phe Glu Tyr Ile Glu Val Gly Gln Phe Arg
    1475                1480                1485

Glu Ser Glu Ala Ile Ile Pro Asn Ile Phe Phe Phe Leu Val Leu
    1490                1495                1500

Leu Ser Tyr Glu Arg Tyr His Ser Lys Gln Ile Ile Gly Ile Pro
    1505                1510                1515

Lys Ile Ile Gln Leu Cys Asp Gly Ile Met Ala Ser Gly Arg Lys
    1520                1525                1530

Ala Val Thr His Ala Ile Pro Ala Leu Gln Pro Ile Val His Asp
    1535                1540                1545

Leu Phe Val Leu Arg Gly Thr Asn Lys Ala Asp Ala Gly Lys Glu
    1550                1555                1560

Leu Glu Thr Gln Lys Glu Val Val Val Ser Met Leu Leu Arg Leu
    1565                1570                1575

Ile Gln Tyr His Gln Val Leu Glu Met Phe Ile Leu Val Leu Gln
    1580                1585                1590

Gln Cys His Lys Glu Asn Glu Asp Lys Trp Lys Arg Leu Ser Arg
    1595                1600                1605

Gln Ile Ala Asp Ile Ile Leu Pro Met Leu Ala Lys Gln Gln Met
    1610                1615                1620

His Ile Asp Ser His Glu Ala Leu Gly Val Leu Asn Thr Leu Phe
    1625                1630                1635

Glu Ile Leu Ala Pro Ser Ser Leu Arg Pro Val Asp Met Leu Leu
    1640                1645                1650

Arg Ser Met Phe Val Thr Pro Asn Thr Met Ala Ser Val Ser Thr
    1655                1660                1665

Val Gln Leu Trp Ile Ser Gly Ile Leu Ala Ile Leu Arg Val Leu
    1670                1675                1680

Ile Ser Gln Ser Thr Glu Asp Ile Val Leu Ser Arg Ile Gln Glu
    1685                1690                1695

Leu Ser Phe Ser Pro Tyr Leu Ile Ser Cys Thr Val Ile Asn Arg
    1700                1705                1710

Leu Arg Asp Gly Asp Ser Thr Ser Thr Leu Glu Glu His Ser Glu
    1715                1720                1725

Gly Lys Gln Ile Lys Asn Leu Pro Glu Glu Thr Phe Ser Arg Phe
    1730                1735                1740

Leu Leu Gln Leu Val Gly Ile Leu Leu Glu Asp Ile Val Thr Lys
    1745                1750                1755

Gln Leu Lys Val Glu Met Ser Glu Gln Gln His Thr Phe Tyr Cys
    1760                1765                1770

Gln Glu Leu Gly Thr Leu Leu Met Cys Leu Ile His Ile Phe Lys
    1775                1780                1785

Ser Gly Met Phe Arg Arg Ile Thr Ala Ala Ala Thr Arg Leu Phe
    1790                1795                1800

Arg Ser Asp Gly Cys Gly Gly Ser Phe Tyr Thr Leu Asp Ser Leu
    1805                1810                1815

Asn Leu Arg Ala Arg Ser Met Ile Thr Thr His Pro Ala Leu Val
    1820                1825                1830

Leu Leu Trp Cys Gln Ile Leu Leu Leu Val Asn His Thr Asp Tyr
    1835                1840                1845

Arg Trp Trp Ala Glu Val Gln Gln Thr Pro Lys Arg His Ser Leu
    1850                1855                1860

Ser Ser Thr Lys Leu Leu Ser Pro Gln Met Ser Gly Glu Glu Glu
    1865                1870                1875

Asp Ser Asp Leu Ala Ala Lys Leu Gly Met Cys Asn Arg Glu Ile
    1880                1885                1890

Val Arg Arg Gly Ala Leu Ile Leu Phe Cys Asp Tyr Val Cys Gln
    1895                1900                1905

Asn Leu His Asp Ser Glu His Leu Thr Trp Leu Ile Val Asn His
    1910                1915                1920

Ile Gln Asp Leu Ile Ser Leu Ser His Glu Pro Pro Val Gln Asp
    1925                1930                1935

Phe Ile Ser Ala Val His Arg Asn Ser Ala Ala Ser Gly Leu Phe
    1940                1945                1950

Ile Gln Ala Ile Gln Ser Arg Cys Glu Asn Leu Ser Thr Pro Thr
    1955                1960                1965

Met Leu Lys Lys Thr Leu Gln Cys Leu Glu Gly Ile His Leu Ser
    1970                1975                1980

Gln Ser Gly Ala Val Leu Thr Leu Tyr Val Asp Arg Leu Leu Cys
    1985                1990                1995

Thr Pro Phe Arg Val Leu Ala Arg Met Val Asp Ile Leu Ala Cys
    2000                2005                2010

Arg Arg Val Glu Met Leu Leu Ala Ala Asn Leu Gln Ser Ser Met
    2015                2020                2025

Ala Gln Leu Pro Met Glu Glu Leu Asn Arg Ile Gln Glu Tyr Leu
    2030                2035                2040

Gln Ser Ser Gly Leu Ala Gln Arg His Gln Arg Leu Tyr Ser Leu
    2045                2050                2055

Leu Asp Arg Phe Arg Leu Ser Thr Met Gln Asp Ser Leu Ser Pro
    2060                2065                2070

Ser Pro Pro Val Ser Ser His Pro Leu Asp Gly Asp Gly His Val
    2075                2080                2085

Ser Leu Glu Thr Val Ser Pro Asp Lys Asp Trp Tyr Val His Leu
    2090                2095                2100

Val Lys Ser Gln Cys Trp Thr Arg Ser Asp Ser Ala Leu Leu Glu
    2105                2110                2115

Gly Ala Glu Leu Val Asn Arg Ile Pro Ala Glu Asp Met Asn Ala
    2120                2125                2130

Phe Met Met Asn Ser Glu Phe Asn Leu Ser Leu Leu Ala Pro Cys
    2135                2140                2145

Leu Ser Leu Gly Met Ser Glu Ile Ser Gly Gly Gln Lys Ser Ala
    2150                2155                2160

Leu Phe Glu Ala Ala Arg Glu Val Thr Leu Ala Arg Val Ser Gly
    2165                2170                2175

Thr Val Gln Gln Leu Pro Ala Val His His Val Phe Gln Pro Glu

2180                2185                2190

Leu Pro Ala Glu Pro Ala Ala Tyr Trp Ser Lys Leu Asn Asp Leu
    2195                2200                2205

Phe Gly Asp Ala Ala Leu Tyr Gln Ser Leu Pro Thr Leu Ala Arg
    2210                2215                2220

Ala Leu Ala Gln Tyr Leu Val Val Val Ser Lys Leu Pro Ser His
    2225                2230                2235

Leu His Leu Pro Pro Glu Lys Glu Lys Asp Ile Val Lys Phe Val
    2240                2245                2250

Val Ala Thr Leu Glu Ala Leu Ser Trp His Leu Ile His Glu Gln
    2255                2260                2265

Ile Pro Leu Ser Leu Asp Leu Gln Ala Gly Leu Asp Cys Cys Cys
    2270                2275                2280

Leu Ala Leu Gln Leu Pro Gly Leu Trp Ser Val Val Ser Ser Thr
    2285                2290                2295

Glu Phe Val Thr His Ala Cys Ser Leu Ile Tyr Cys Val His Phe
    2300                2305                2310

Ile Leu Glu Ala Val Ala Val Gln Pro Gly Glu Gln Leu Leu Ser
    2315                2320                2325

Pro Glu Arg Arg Thr Asn Thr Pro Lys Ala Ile Ser Glu Glu Glu
    2330                2335                2340

Glu Glu Val Asp Pro Asn Thr Gln Asn Pro Lys Tyr Ile Thr Ala
    2345                2350                2355

Ala Cys Glu Met Val Ala Glu Met Val Glu Ser Leu Gln Ser Val
    2360                2365                2370

Leu Ala Leu Gly His Lys Arg Asn Ser Gly Val Pro Ala Phe Leu
    2375                2380                2385

Thr Pro Leu Leu Arg Asn Ile Ile Ile Ser Leu Ala Arg Leu Pro
    2390                2395                2400

Leu Val Asn Ser Tyr Thr Arg Val Pro Pro Leu Val Trp Lys Leu
    2405                2410                2415

Gly Trp Ser Pro Lys Pro Gly Gly Asp Phe Gly Thr Ala Phe Pro
    2420                2425                2430

Glu Ile Pro Val Glu Phe Leu Gln Glu Lys Glu Val Phe Lys Glu
    2435                2440                2445

Phe Ile Tyr Arg Ile Asn Thr Leu Gly Trp Thr Ser Arg Thr Gln
    2450                2455                2460

Phe Glu Glu Thr Trp Ala Thr Leu Leu Gly Val Leu Val Thr Gln
    2465                2470                2475

Pro Leu Val Met Glu Gln Glu Glu Ser Pro Pro Glu Glu Asp Thr
    2480                2485                2490

Glu Arg Thr Gln Ile Asn Val Leu Ala Val Gln Ala Ile Thr Ser
    2495                2500                2505

Leu Val Leu Ser Ala Met Thr Val Pro Val Ala Gly Asn Pro Ala
    2510                2515                2520

Val Ser Cys Leu Glu Gln Gln Pro Arg Asn Lys Pro Leu Lys Ala
    2525                2530                2535

Leu Asp Thr Arg Phe Gly Arg Lys Leu Ser Ile Ile Arg Gly Ile
    2540                2545                2550

Val Glu Gln Glu Ile Gln Ala Met Val Ser Lys Arg Glu Asn Ile
    2555                2560                2565

Ala Thr His His Leu Tyr Gln Ala Trp Asp Pro Val Pro Ser Leu
    2570                2575                2580

Ser Pro Ala Thr Thr Gly Ala Leu Ile Ser His Glu Lys Leu Leu
    2585                2590                2595

Leu Gln Ile Asn Pro Glu Arg Glu Leu Gly Ser Met Ser Tyr Lys
    2600                2605                2610

Leu Gly Gln Val Ser Ile His Ser Val Trp Leu Gly Asn Ser Ile
    2615                2620                2625

Thr Pro Leu Arg Glu Glu Glu Trp Asp Glu Glu Glu Glu Glu Glu
    2630                2635                2640

Ala Asp Ala Pro Ala Pro Ser Ser Pro Pro Thr Ser Pro Val Asn
    2645                2650                2655

Ser Arg Lys His Arg Ala Gly Val Asp Ile His Ser Cys Ser Gln
    2660                2665                2670

Phe Leu Leu Glu Leu Tyr Ser Arg Trp Ile Leu Pro Ser Ser Ser
    2675                2680                2685

Ala Arg Arg Thr Pro Ala Ile Leu Ile Ser Glu Val Val Arg Ser
    2690                2695                2700

Leu Leu Val Val Ser Asp Leu Phe Thr Glu Arg Asn Gln Phe Glu
    2705                2710                2715

Leu Met Tyr Val Thr Leu Thr Glu Leu Arg Arg Val His Pro Ser
    2720                2725                2730

Glu Asp Glu Ile Leu Ala Gln Tyr Leu Val Pro Ala Thr Cys Lys
    2735                2740                2745

Ala Ala Ala Val Leu Gly Met Asp Lys Ala Val Ala Glu Pro Val
    2750                2755                2760

Ser Arg Leu Leu Glu Ser Thr Leu Arg Ser Ser His Leu Pro Ser
    2765                2770                2775

Arg Val Gly Ala Leu His Gly Val Leu Tyr Val Leu Glu Cys Asp
    2780                2785                2790

Leu Leu Asp Asp Thr Ala Lys Gln Leu Ile Pro Val Ile Ser Asp
    2795                2800                2805

Tyr Leu Leu Ser Asn Leu Lys Gly Ile Ala His Cys Val Asn Ile
    2810                2815                2820

His Ser Gln Gln His Val Leu Val Met Cys Ala Thr Ala Phe Tyr
    2825                2830                2835

Leu Ile Glu Asn Tyr Pro Leu Asp Val Gly Pro Glu Phe Ser Ala
    2840                2845                2850

Ser Ile Ile Gln Met Cys Gly Val Met Leu Ser Gly Ser Glu Glu
    2855                2860                2865

Ser Thr Pro Ser Ile Ile Tyr His Cys Ala Leu Arg Gly Leu Glu
    2870                2875                2880

Arg Leu Leu Leu Ser Glu Gln Leu Ser Arg Leu Asp Ala Glu Ser
    2885                2890                2895

Leu Val Lys Leu Ser Val Asp Arg Val Asn Val His Ser Pro His
    2900                2905                2910

Arg Ala Met Ala Ala Leu Gly Leu Met Leu Thr Cys Met Tyr Thr
    2915                2920                2925

Gly Lys Glu Lys Val Ser Pro Gly Arg Thr Ser Asp Pro Asn Pro
    2930                2935                2940

Ala Ala Pro Asp Ser Glu Ser Val Ile Val Ala Met Glu Arg Val
    2945                2950                2955

Ser Val Leu Phe Asp Arg Ile Arg Lys Gly Phe Pro Cys Glu Ala
    2960                2965                2970

Arg Val Val Ala Arg Ile Leu Pro Gln Phe Leu Asp Asp Phe Phe
    2975                2980                2985

Pro Pro Gln Asp Ile Met Asn Lys Val Ile Gly Glu Phe Leu Ser
    2990                2995                3000

Asn Gln Gln Pro Tyr Pro Gln Phe Met Ala Thr Val Val Tyr Lys
    3005                3010                3015

Val Phe Gln Thr Leu His Ser Thr Gly Gln Ser Ser Met Val Arg
    3020                3025                3030

Asp Trp Val Met Leu Ser Leu Ser Asn Phe Thr Gln Arg Ala Pro
    3035                3040                3045

Val Ala Met Ala Thr Trp Ser Leu Ser Cys Phe Phe Val Ser Ala
    3050                3055                3060

Ser Thr Ser Pro Trp Val Ala Ala Ile Leu Pro His Val Ile Ser
    3065                3070                3075

Arg Met Gly Lys Leu Glu Gln Val Asp Val Asn Leu Phe Cys Leu
    3080                3085                3090

Val Ala Thr Asp Phe Tyr Arg His Gln Ile Glu Glu Glu Leu Asp
    3095                3100                3105

Arg Arg Ala Phe Gln Ser Val Leu Glu Val Val Ala Ala Pro Gly
    3110                3115                3120

Ser Pro Tyr His Arg Leu Leu Thr Cys Leu Arg Asn Val His Lys

3125                3130                3135

Val Thr  Thr Cys
    3140

<210>  3
<211>  770
<212>  PRT
<213>  Homo sapiens

<400>  3

<210>  4
<211>  414
<212>  PRT
<213>  Homo sapiens

<400>  4

<210>  5
<211>  526
<212>  PRT
<213>  Homo sapiens

<400>  5

<210>  6
<211>  758
<212>  PRT
<213>  Homo sapiens

<400>  6

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu
                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Glu Pro Glu Ser
        115                 120                 125

Gly Lys Val Val Gln Glu Gly Phe Leu Arg Glu Pro Gly Pro Pro Gly
    130                 135                 140

Leu Ser His Gln Leu Met Ser Gly Met Pro Gly Ala Pro Leu Leu Pro

145                 150                 155                 160

Glu Gly Pro Arg Glu Ala Thr Arg Gln Pro Ser Gly Thr Gly Pro Glu
                165                 170                 175

Asp Thr Glu Gly Gly Arg His Ala Pro Glu Leu Leu Lys His Gln Leu
            180                 185                 190

Leu Gly Asp Leu His Gln Glu Gly Pro Pro Leu Lys Gly Ala Gly Gly
        195                 200                 205

Lys Glu Arg Pro Gly Ser Lys Glu Glu Val Asp Glu Asp Arg Asp Val
    210                 215                 220

Asp Glu Ser Ser Pro Gln Asp Ser Pro Pro Ser Lys Ala Ser Pro Ala
225                 230                 235                 240

Gln Asp Gly Arg Pro Pro Gln Thr Ala Ala Arg Glu Ala Thr Ser Ile
                245                 250                 255

Pro Gly Phe Pro Ala Glu Gly Ala Ile Pro Leu Pro Val Asp Phe Leu
            260                 265                 270

Ser Lys Val Ser Thr Glu Ile Pro Ala Ser Glu Pro Asp Gly Pro Ser
        275                 280                 285

Val Gly Arg Ala Lys Gly Gln Asp Ala Pro Leu Glu Phe Thr Phe His
    290                 295                 300

Val Glu Ile Thr Pro Asn Val Gln Lys Glu Gln Ala His Ser Glu Glu
305                 310                 315                 320

His Leu Gly Arg Ala Ala Phe Pro Gly Ala Pro Gly Glu Gly Pro Glu
                325                 330                 335

Ala Arg Gly Pro Ser Leu Gly Glu Asp Thr Lys Glu Ala Asp Leu Pro
            340                 345                 350

Glu Pro Ser Glu Lys Gln Pro Ala Ala Ala Pro Arg Gly Lys Pro Val
        355                 360                 365

Ser Arg Val Pro Gln Leu Lys Ala Arg Met Val Ser Lys Ser Lys Asp
    370                 375                 380

Gly Thr Gly Ser Asp Asp Lys Lys Ala Lys Thr Ser Thr Arg Ser Ser
385                 390                 395                 400

Ala Lys Thr Leu Lys Asn Arg Pro Cys Leu Ser Pro Lys His Pro Thr
                405                 410                 415

Pro Gly Ser Ser Asp Pro Leu Ile Gln Pro Ser Ser Pro Ala Val Cys
            420                 425                 430

Pro Glu Pro Pro Ser Ser Pro Lys Tyr Val Ser Ser Val Thr Ser Arg
        435                 440                 445

Thr Gly Ser Ser Gly Ala Lys Glu Met Lys Leu Lys Gly Ala Asp Gly
    450                 455                 460

Lys Thr Lys Ile Ala Thr Pro Arg Gly Ala Ala Pro Pro Gly Gln Lys
465                 470                 475                 480

Gly Gln Ala Asn Ala Thr Arg Ile Pro Ala Lys Thr Pro Pro Ala Pro
                485                 490                 495

Lys Thr Pro Pro Ser Ser Gly Glu Pro Pro Lys Ser Gly Asp Arg Ser
            500                 505                 510

Gly Tyr Ser Ser Pro Gly Ser Pro Gly Thr Pro Gly Ser Arg Ser Arg
        515                 520                 525

Thr Pro Ser Leu Pro Thr Pro Pro Thr Arg Glu Pro Lys Lys Val Ala
    530                 535                 540

Val Val Arg Thr Pro Pro Lys Ser Pro Ser Ser Ala Lys Ser Arg Leu
545                 550                 555                 560

Gln Thr Ala Pro Val Pro Met Pro Asp Leu Lys Asn Val Lys Ser Lys
                565                 570                 575

Ile Gly Ser Thr Glu Asn Leu Lys His Gln Pro Gly Gly Gly Lys Val
            580                 585                 590

Gln Ile Ile Asn Lys Lys Leu Asp Leu Ser Asn Val Gln Ser Lys Cys
        595                 600                 605

Gly Ser Lys Asp Asn Ile Lys His Val Pro Gly Gly Gly Ser Val Gln
    610                 615                 620

Ile Val Tyr Lys Pro Val Asp Leu Ser Lys Val Thr Ser Lys Cys Gly
625                 630                 635                 640

Ser Leu Gly Asn Ile His His Lys Pro Gly Gly Gly Gln Val Glu Val
                645                 650                 655

Lys Ser Glu Lys Leu Asp Phe Lys Asp Arg Val Gln Ser Lys Ile Gly
        660                 665                 670

Ser Leu Asp Asn Ile Thr His Val Pro Gly Gly Gly Asn Lys Lys Ile
        675                 680                 685

Glu Thr His Lys Leu Thr Phe Arg Glu Asn Ala Lys Ala Lys Thr Asp
    690                 695                 700

His Gly Ala Glu Ile Val Tyr Lys Ser Pro Val Val Ser Gly Asp Thr
705                 710                 715                 720

Ser Pro Arg His Leu Ser Asn Val Ser Ser Thr Gly Ser Ile Asp Met
                725                 730                 735

Val Asp Ser Pro Gln Leu Ala Thr Leu Ala Asp Glu Val Ser Ala Ser
            740                 745                 750

Leu Ala Lys Gln Gly Leu
        755

<210>  7
<211>  253
<212>  PRT
<213>  Homo sapiens

<400>  7

<210>  8
<211>  2386
<212>  PRT
<213>  Homo sapiens

<400>  8

Met Leu Arg Gly Pro Gly Pro Gly Leu Leu Leu Leu Ala Val Gln Cys
1               5                   10                  15

Leu Gly Thr Ala Val Pro Ser Thr Gly Ala Ser Lys Ser Lys Arg Gln
            20                  25                  30

Ala Gln Gln Met Val Gln Pro Gln Ser Pro Val Ala Val Ser Gln Ser
        35                  40                  45

Lys Pro Gly Cys Tyr Asp Asn Gly Lys His Tyr Gln Ile Asn Gln Gln
    50                  55                  60

Trp Glu Arg Thr Tyr Leu Gly Asn Ala Leu Val Cys Thr Cys Tyr Gly
65                  70                  75                  80

Gly Ser Arg Gly Phe Asn Cys Glu Ser Lys Pro Glu Ala Glu Glu Thr
                85                  90                  95

Cys Phe Asp Lys Tyr Thr Gly Asn Thr Tyr Arg Val Gly Asp Thr Tyr
            100                 105                 110

Glu Arg Pro Lys Asp Ser Met Ile Trp Asp Cys Thr Cys Ile Gly Ala
        115                 120                 125

Gly Arg Gly Arg Ile Ser Cys Thr Ile Ala Asn Arg Cys His Glu Gly
    130                 135                 140

Gly Gln Ser Tyr Lys Ile Gly Asp Thr Trp Arg Arg Pro His Glu Thr
145                 150                 155                 160

Gly Gly Tyr Met Leu Glu Cys Val Cys Leu Gly Asn Gly Lys Gly Glu
                165                 170                 175

Trp Thr Cys Lys Pro Ile Ala Glu Lys Cys Phe Asp His Ala Ala Gly
            180                 185                 190

Thr Ser Tyr Val Val Gly Glu Thr Trp Glu Lys Pro Tyr Gln Gly Trp
        195                 200                 205

Met Met Val Asp Cys Thr Cys Leu Gly Glu Gly Ser Gly Arg Ile Thr
    210                 215                 220

Cys Thr Ser Arg Asn Arg Cys Asn Asp Gln Asp Thr Arg Thr Ser Tyr
225                 230                 235                 240

Arg Ile Gly Asp Thr Trp Ser Lys Lys Asp Asn Arg Gly Asn Leu Leu
                245                 250                 255

Gln Cys Ile Cys Thr Gly Asn Gly Arg Gly Glu Trp Lys Cys Glu Arg
            260                 265                 270

His Thr Ser Val Gln Thr Thr Ser Ser Gly Ser Gly Pro Phe Thr Asp
        275                 280                 285

Val Arg Ala Ala Val Tyr Gln Pro Gln Pro His Pro Gln Pro Pro Pro
    290                 295                 300

Tyr Gly His Cys Val Thr Asp Ser Gly Val Val Tyr Ser Val Gly Met
305                 310                 315                 320

Gln Trp Leu Lys Thr Gln Gly Asn Lys Gln Met Leu Cys Thr Cys Leu
                325                 330                 335

Gly Asn Gly Val Ser Cys Gln Glu Thr Ala Val Thr Gln Thr Tyr Gly
            340                 345                 350

Gly Asn Ser Asn Gly Glu Pro Cys Val Leu Pro Phe Thr Tyr Asn Gly
        355                 360                 365

Arg Thr Phe Tyr Ser Cys Thr Thr Glu Gly Arg Gln Asp Gly His Leu
    370                 375                 380

Trp Cys Ser Thr Thr Ser Asn Tyr Glu Gln Asp Gln Lys Tyr Ser Phe
385                 390                 395                 400

Cys Thr Asp His Thr Val Leu Val Gln Thr Arg Gly Gly Asn Ser Asn
                405                 410                 415

Gly Ala Leu Cys His Phe Pro Phe Leu Tyr Asn Asn His Asn Tyr Thr
            420                 425                 430

Asp Cys Thr Ser Glu Gly Arg Arg Asp Asn Met Lys Trp Cys Gly Thr
        435                 440                 445

Thr Gln Asn Tyr Asp Ala Asp Gln Lys Phe Gly Phe Cys Pro Met Ala
    450                 455                 460

Ala His Glu Glu Ile Cys Thr Thr Asn Glu Gly Val Met Tyr Arg Ile
465                 470                 475                 480

Gly Asp Gln Trp Asp Lys Gln His Asp Met Gly His Met Met Arg Cys
                485                 490                 495

Thr Cys Val Gly Asn Gly Arg Gly Glu Trp Thr Cys Ile Ala Tyr Ser
            500                 505                 510

Gln Leu Arg Asp Gln Cys Ile Val Asp Asp Ile Thr Tyr Asn Val Asn
        515                 520                 525

Asp Thr Phe His Lys Arg His Glu Glu Gly His Met Leu Asn Cys Thr
    530                 535                 540

Cys Phe Gly Gln Gly Arg Gly Arg Trp Lys Cys Asp Pro Val Asp Gln
545                 550                 555                 560

Cys Gln Asp Ser Glu Thr Gly Thr Phe Tyr Gln Ile Gly Asp Ser Trp
                565                 570                 575

Glu Lys Tyr Val His Gly Val Arg Tyr Gln Cys Tyr Cys Tyr Gly Arg

580                 585                 590

Gly Ile Gly Glu Trp His Cys Gln Pro Leu Gln Thr Tyr Pro Ser Ser
        595                 600                 605

Ser Gly Pro Val Glu Val Phe Ile Thr Glu Thr Pro Ser Gln Pro Asn
    610                 615                 620

Ser His Pro Ile Gln Trp Asn Ala Pro Gln Pro Ser His Ile Ser Lys
625                 630                 635                 640

Tyr Ile Leu Arg Trp Arg Pro Lys Asn Ser Val Gly Arg Trp Lys Glu
                645                 650                 655

Ala Thr Ile Pro Gly His Leu Asn Ser Tyr Thr Ile Lys Gly Leu Lys
            660                 665                 670

Pro Gly Val Val Tyr Glu Gly Gln Leu Ile Ser Ile Gln Gln Tyr Gly
        675                 680                 685

His Gln Glu Val Thr Arg Phe Asp Phe Thr Thr Thr Ser Thr Ser Thr
    690                 695                 700

Pro Val Thr Ser Asn Thr Val Thr Gly Glu Thr Thr Pro Phe Ser Pro
705                 710                 715                 720

Leu Val Ala Thr Ser Glu Ser Val Thr Glu Ile Thr Ala Ser Ser Phe
                725                 730                 735

Val Val Ser Trp Val Ser Ala Ser Asp Thr Val Ser Gly Phe Arg Val
            740                 745                 750

Glu Tyr Glu Leu Ser Glu Glu Gly Asp Glu Pro Gln Tyr Leu Asp Leu
        755                 760                 765

Pro Ser Thr Ala Thr Ser Val Asn Ile Pro Asp Leu Leu Pro Gly Arg
    770                 775                 780

Lys Tyr Ile Val Asn Val Tyr Gln Ile Ser Glu Asp Gly Glu Gln Ser
785                 790                 795                 800

Leu Ile Leu Ser Thr Ser Gln Thr Thr Ala Pro Asp Ala Pro Pro Asp
                805                 810                 815

Thr Thr Val Asp Gln Val Asp Asp Thr Ser Ile Val Val Arg Trp Ser
            820                 825                 830

Arg Pro Gln Ala Pro Ile Thr Gly Tyr Arg Ile Val Tyr Ser Pro Ser
        835                 840                 845

Val Glu Gly Ser Ser Thr Glu Leu Asn Leu Pro Glu Thr Ala Asn Ser
    850                 855                 860

Val Thr Leu Ser Asp Leu Gln Pro Gly Val Gln Tyr Asn Ile Thr Ile
865                 870                 875                 880

Tyr Ala Val Glu Glu Asn Gln Glu Ser Thr Pro Val Val Ile Gln Gln
                885                 890                 895

Glu Thr Thr Gly Thr Pro Arg Ser Asp Thr Val Pro Ser Pro Arg Asp
            900                 905                 910

Leu Gln Phe Val Glu Val Thr Asp Val Lys Val Thr Ile Met Trp Thr
        915                 920                 925

Pro Pro Glu Ser Ala Val Thr Gly Tyr Arg Val Asp Val Ile Pro Val
    930                 935                 940

Asn Leu Pro Gly Glu His Gly Gln Arg Leu Pro Ile Ser Arg Asn Thr
945                 950                 955                 960

Phe Ala Glu Val Thr Gly Leu Ser Pro Gly Val Thr Tyr Tyr Phe Lys
                965                 970                 975

Val Phe Ala Val Ser His Gly Arg Glu Ser Lys Pro Leu Thr Ala Gln
            980                 985                 990

Gln Thr Thr Lys Leu Asp Ala Pro Thr Asn Leu Gln Phe Val Asn Glu
        995                 1000                1005

Thr Asp Ser Thr Val Leu Val Arg Trp Thr Pro Pro Arg Ala Gln
    1010                1015                1020

Ile Thr Gly Tyr Arg Leu Thr Val Gly Leu Thr Arg Arg Gly Gln
    1025                1030                1035

Pro Arg Gln Tyr Asn Val Gly Pro Ser Val Ser Lys Tyr Pro Leu
    1040                1045                1050

Arg Asn Leu Gln Pro Ala Ser Glu Tyr Thr Val Ser Leu Val Ala
    1055                1060                1065

Ile Lys Gly Asn Gln Glu Ser Pro Lys Ala Thr Gly Val Phe Thr
    1070                1075                1080

Thr Leu Gln Pro Gly Ser Ser Ile Pro Pro Tyr Asn Thr Glu Val
    1085                1090                1095

Thr Glu Thr Thr Ile Val Ile Thr Trp Thr Pro Ala Pro Arg Ile
    1100                1105                1110

Gly Phe Lys Leu Gly Val Arg Pro Ser Gln Gly Gly Glu Ala Pro
    1115                1120                1125

Arg Glu Val Thr Ser Asp Ser Gly Ser Ile Val Val Ser Gly Leu
    1130                1135                1140

Thr Pro Gly Val Glu Tyr Val Tyr Thr Ile Gln Val Leu Arg Asp
    1145                1150                1155

Gly Gln Glu Arg Asp Ala Pro Ile Val Asn Lys Val Val Thr Pro
    1160                1165                1170

Leu Ser Pro Pro Thr Asn Leu His Leu Glu Ala Asn Pro Asp Thr
    1175                1180                1185

Gly Val Leu Thr Val Ser Trp Glu Arg Ser Thr Thr Pro Asp Ile
    1190                1195                1200

Thr Gly Tyr Arg Ile Thr Thr Thr Pro Thr Asn Gly Gln Gln Gly
    1205                1210                1215

Asn Ser Leu Glu Glu Val Val His Ala Asp Gln Ser Ser Cys Thr
    1220                1225                1230

Phe Asp Asn Leu Ser Pro Gly Leu Glu Tyr Asn Val Ser Val Tyr
    1235                1240                1245

Thr Val Lys Asp Asp Lys Glu Ser Val Pro Ile Ser Asp Thr Ile
    1250                1255                1260

Ile Pro Ala Val Pro Pro Pro Thr Asp Leu Arg Phe Thr Asn Ile
    1265                1270                1275

Gly Pro Asp Thr Met Arg Val Thr Trp Ala Pro Pro Pro Ser Ile
    1280                1285                1290

Asp Leu Thr Asn Phe Leu Val Arg Tyr Ser Pro Val Lys Asn Glu
    1295                1300                1305

Glu Asp Val Ala Glu Leu Ser Ile Ser Pro Ser Asp Asn Ala Val
    1310                1315                1320

Val Leu Thr Asn Leu Leu Pro Gly Thr Glu Tyr Val Val Ser Val
    1325                1330                1335

Ser Ser Val Tyr Glu Gln His Glu Ser Thr Pro Leu Arg Gly Arg
    1340                1345                1350

Gln Lys Thr Gly Leu Asp Ser Pro Thr Gly Ile Asp Phe Ser Asp
    1355                1360                1365

Ile Thr Ala Asn Ser Phe Thr Val His Trp Ile Ala Pro Arg Ala
    1370                1375                1380

Thr Ile Thr Gly Tyr Arg Ile Arg His His Pro Glu His Phe Ser
    1385                1390                1395

Gly Arg Pro Arg Glu Asp Arg Val Pro His Ser Arg Asn Ser Ile
    1400                1405                1410

Thr Leu Thr Asn Leu Thr Pro Gly Thr Glu Tyr Val Val Ser Ile
    1415                1420                1425

Val Ala Leu Asn Gly Arg Glu Glu Ser Pro Leu Leu Ile Gly Gln
    1430                1435                1440

Gln Ser Thr Val Ser Asp Val Pro Arg Asp Leu Glu Val Val Ala
    1445                1450                1455

Ala Thr Pro Thr Ser Leu Leu Ile Ser Trp Asp Ala Pro Ala Val
    1460                1465                1470

Thr Val Arg Tyr Tyr Arg Ile Thr Tyr Gly Glu Thr Gly Gly Asn
    1475                1480                1485

Ser Pro Val Gln Glu Phe Thr Val Pro Gly Ser Lys Ser Thr Ala
    1490                1495                1500

Thr Ile Ser Gly Leu Lys Pro Gly Val Asp Tyr Thr Ile Thr Val
    1505                1510                1515

Tyr Ala Val Thr Gly Arg Gly Asp Ser Pro Ala Ser Ser Lys Pro
    1520                1525                1530

Ile Ser Ile Asn Tyr Arg Thr Glu Ile Asp Lys Pro Ser Gln Met
    1535                1540                1545

Gln Val Thr Asp Val Gln Asp Asn Ser Ile Ser Val Lys Trp Leu

1550                1555                1560

Pro Ser Ser Ser Pro Val Thr Gly Tyr Arg Val Thr Thr Thr Pro
    1565                1570                1575

Lys Asn Gly Pro Gly Pro Thr Lys Thr Lys Thr Ala Gly Pro Asp
    1580                1585                1590

Gln Thr Glu Met Thr Ile Glu Gly Leu Gln Pro Thr Val Glu Tyr
    1595                1600                1605

Val Val Ser Val Tyr Ala Gln Asn Pro Ser Gly Glu Ser Gln Pro
    1610                1615                1620

Leu Val Gln Thr Ala Val Thr Asn Ile Asp Arg Pro Lys Gly Leu
    1625                1630                1635

Ala Phe Thr Asp Val Asp Val Asp Ser Ile Lys Ile Ala Trp Glu
    1640                1645                1650

Ser Pro Gln Gly Gln Val Ser Arg Tyr Arg Val Thr Tyr Ser Ser
    1655                1660                1665

Pro Glu Asp Gly Ile His Glu Leu Phe Pro Ala Pro Asp Gly Glu
    1670                1675                1680

Glu Asp Thr Ala Glu Leu Gln Gly Leu Arg Pro Gly Ser Glu Tyr
    1685                1690                1695

Thr Val Ser Val Val Ala Leu His Asp Asp Met Glu Ser Gln Pro
    1700                1705                1710

Leu Ile Gly Thr Gln Ser Thr Ala Ile Pro Ala Pro Thr Asp Leu
    1715                1720                1725

Lys Phe Thr Gln Val Thr Pro Thr Ser Leu Ser Ala Gln Trp Thr
    1730                1735                1740

Pro Pro Asn Val Gln Leu Thr Gly Tyr Arg Val Arg Val Thr Pro
    1745                1750                1755

Lys Glu Lys Thr Gly Pro Met Lys Glu Ile Asn Leu Ala Pro Asp
    1760                1765                1770

Ser Ser Ser Val Val Val Ser Gly Leu Met Val Ala Thr Lys Tyr
    1775                1780                1785

Glu Val Ser Val Tyr Ala Leu Lys Asp Thr Leu Thr Ser Arg Pro
    1790                1795                1800

Ala Gln Gly Val Val Thr Thr Leu Glu Asn Val Ser Pro Pro Arg
    1805                1810                1815

Arg Ala Arg Val Thr Asp Ala Thr Glu Thr Thr Ile Thr Ile Ser
    1820                1825                1830

Trp Arg Thr Lys Thr Glu Thr Ile Thr Gly Phe Gln Val Asp Ala
    1835                1840                1845

Val Pro Ala Asn Gly Gln Thr Pro Ile Gln Arg Thr Ile Lys Pro
    1850                1855                1860

Asp Val Arg Ser Tyr Thr Ile Thr Gly Leu Gln Pro Gly Thr Asp
    1865                1870                1875

Tyr Lys Ile Tyr Leu Tyr Thr Leu Asn Asp Asn Ala Arg Ser Ser
    1880                1885                1890

Pro Val Val Ile Asp Ala Ser Thr Ala Ile Asp Ala Pro Ser Asn
    1895                1900                1905

Leu Arg Phe Leu Ala Thr Thr Pro Asn Ser Leu Leu Val Ser Trp
    1910                1915                1920

Gln Pro Pro Arg Ala Arg Ile Thr Gly Tyr Ile Ile Lys Tyr Glu
    1925                1930                1935

Lys Pro Gly Ser Pro Pro Arg Glu Val Val Pro Arg Pro Arg Pro
    1940                1945                1950

Gly Val Thr Glu Ala Thr Ile Thr Gly Leu Glu Pro Gly Thr Glu
    1955                1960                1965

Tyr Thr Ile Tyr Val Ile Ala Leu Lys Asn Asn Gln Lys Ser Glu
    1970                1975                1980

Pro Leu Ile Gly Arg Lys Lys Thr Asp Glu Leu Pro Gln Leu Val
    1985                1990                1995

Thr Leu Pro His Pro Asn Leu His Gly Pro Glu Ile Leu Asp Val
    2000                2005                2010

Pro Ser Thr Val Gln Lys Thr Pro Phe Val Thr His Pro Gly Tyr
    2015                2020                2025

Asp Thr Gly Asn Gly Ile Gln Leu Pro Gly Thr Ser Gly Gln Gln
    2030                2035                2040

Pro Ser Val Gly Gln Gln Met Ile Phe Glu Glu His Gly Phe Arg
    2045                2050                2055

Arg Thr Thr Pro Pro Thr Thr Ala Thr Pro Ile Arg His Arg Pro
    2060                2065                2070

Arg Pro Tyr Pro Pro Asn Val Gly Glu Glu Ile Gln Ile Gly His
    2075                2080                2085

Ile Pro Arg Glu Asp Val Asp Tyr His Leu Tyr Pro His Gly Pro
    2090                2095                2100

Gly Leu Asn Pro Asn Ala Ser Thr Gly Gln Glu Ala Leu Ser Gln
    2105                2110                2115

Thr Thr Ile Ser Trp Ala Pro Phe Gln Asp Thr Ser Glu Tyr Ile
    2120                2125                2130

Ile Ser Cys His Pro Val Gly Thr Asp Glu Glu Pro Leu Gln Phe
    2135                2140                2145

Arg Val Pro Gly Thr Ser Thr Ser Ala Thr Leu Thr Gly Leu Thr
    2150                2155                2160

Arg Gly Ala Thr Tyr Asn Val Ile Val Glu Ala Leu Lys Asp Gln
    2165                2170                2175

Gln Arg His Lys Val Arg Glu Glu Val Val Thr Val Gly Asn Ser
    2180                2185                2190

Val Asn Glu Gly Leu Asn Gln Pro Thr Asp Asp Ser Cys Phe Asp
    2195                2200                2205

Pro Tyr Thr Val Ser His Tyr Ala Val Gly Asp Glu Trp Glu Arg
    2210                2215                2220

Met Ser Glu Ser Gly Phe Lys Leu Leu Cys Gln Cys Leu Gly Phe
    2225                2230                2235

Gly Ser Gly His Phe Arg Cys Asp Ser Ser Arg Trp Cys His Asp
    2240                2245                2250

Asn Gly Val Asn Tyr Lys Ile Gly Glu Lys Trp Asp Arg Gln Gly
    2255                2260                2265

Glu Asn Gly Gln Met Met Ser Cys Thr Cys Leu Gly Asn Gly Lys
    2270                2275                2280

Gly Glu Phe Lys Cys Asp Pro His Glu Ala Thr Cys Tyr Asp Asp
    2285                2290                2295

Gly Lys Thr Tyr His Val Gly Glu Gln Trp Gln Lys Glu Tyr Leu
    2300                2305                2310

Gly Ala Ile Cys Ser Cys Thr Cys Phe Gly Gly Gln Arg Gly Trp
    2315                2320                2325

Arg Cys Asp Asn Cys Arg Arg Pro Gly Gly Glu Pro Ser Pro Glu
    2330                2335                2340

Gly Thr Thr Gly Gln Ser Tyr Asn Gln Tyr Ser Gln Arg Tyr His
    2345                2350                2355

Gln Arg Thr Asn Thr Asn Val Asn Cys Pro Ile Glu Cys Phe Met
    2360                2365                2370

Pro Leu Asp Val Gln Ala Asp Arg Glu Asp Ser Arg Glu
    2375                2380                2385

<210>  9
<211>  154
<212>  PRT
<213>  Homo sapiens

<400>  9

<210> 10
<211> 803
<212> PRT
<213> Homo sapiens

<400> 10

Met Arg Ala Leu Trp Val Leu Gly Leu Cys Cys Val Leu Leu Thr Phe
1               5                   10                  15

Gly Ser Val Arg Ala Asp Asp Glu Val Asp Val Asp Gly Thr Val Glu
            20                  25                  30

Glu Asp Leu Gly Lys Ser Arg Glu Gly Ser Arg Thr Asp Asp Glu Val
        35                  40                  45

Val Gln Arg Glu Glu Glu Ala Ile Gln Leu Asp Gly Leu Asn Ala Ser
    50                  55                  60

Gln Ile Arg Glu Leu Arg Glu Lys Ser Glu Lys Phe Ala Phe Gln Ala
65                  70                  75                  80

Glu Val Asn Arg Met Met Lys Leu Ile Ile Asn Ser Leu Tyr Lys Asn
                85                  90                  95

Lys Glu Ile Phe Leu Arg Glu Leu Ile Ser Asn Ala Ser Asp Ala Leu
            100                 105                 110

Asp Lys Ile Arg Leu Ile Ser Leu Thr Asp Glu Asn Ala Leu Ser Gly
        115                 120                 125

Asn Glu Glu Leu Thr Val Lys Ile Lys Cys Asp Lys Glu Lys Asn Leu
    130                 135                 140

Leu His Val Thr Asp Thr Gly Val Gly Met Thr Arg Glu Glu Leu Val
145                 150                 155                 160

Lys Asn Leu Gly Thr Ile Ala Lys Ser Gly Thr Ser Glu Phe Leu Asn
                165                 170                 175

Lys Met Thr Glu Ala Gln Glu Asp Gly Gln Ser Thr Ser Glu Leu Ile
            180                 185                 190

Gly Gln Phe Gly Val Gly Phe Tyr Ser Ala Phe Leu Val Ala Asp Lys
        195                 200                 205

Val Ile Val Thr Ser Lys His Asn Asn Asp Thr Gln His Ile Trp Glu
    210                 215                 220

Ser Asp Ser Asn Glu Phe Ser Val Ile Ala Asp Pro Arg Gly Asn Thr
225                 230                 235                 240

Leu Gly Arg Gly Thr Thr Ile Thr Leu Val Leu Lys Glu Glu Ala Ser
                245                 250                 255

Asp Tyr Leu Glu Leu Asp Thr Ile Lys Asn Leu Val Lys Lys Tyr Ser
            260                 265                 270

Gln Phe Ile Asn Phe Pro Ile Tyr Val Trp Ser Ser Lys Thr Glu Thr
        275                 280                 285

Val Glu Glu Pro Met Glu Glu Glu Glu Ala Ala Lys Glu Glu Lys Glu
    290                 295                 300

Glu Ser Asp Asp Glu Ala Ala Val Glu Glu Glu Glu Glu Glu Lys Lys
305                 310                 315                 320

Pro Lys Thr Lys Lys Val Glu Lys Thr Val Trp Asp Trp Glu Leu Met
                325                 330                 335

Asn Asp Ile Lys Pro Ile Trp Gln Arg Pro Ser Lys Glu Val Glu Glu
            340                 345                 350

Asp Glu Tyr Lys Ala Phe Tyr Lys Ser Phe Ser Lys Glu Ser Asp Asp
        355                 360                 365

Pro Met Ala Tyr Ile His Phe Thr Ala Glu Gly Glu Val Thr Phe Lys
    370                 375                 380

Ser Ile Leu Phe Val Pro Thr Ser Ala Pro Arg Gly Leu Phe Asp Glu
385                 390                 395                 400

Tyr Gly Ser Lys Lys Ser Asp Tyr Ile Lys Leu Tyr Val Arg Arg Val

                405                 410                 415

Phe Ile Thr Asp Asp Phe His Asp Met Met Pro Lys Tyr Leu Asn Phe
            420                 425                 430

Val Lys Gly Val Val Asp Ser Asp Asp Leu Pro Leu Asn Val Ser Arg
        435                 440                 445

Glu Thr Leu Gln Gln His Lys Leu Leu Lys Val Ile Arg Lys Lys Leu
    450                 455                 460

Val Arg Lys Thr Leu Asp Met Ile Lys Lys Ile Ala Asp Asp Lys Tyr
465                 470                 475                 480

Asn Asp Thr Phe Trp Lys Glu Phe Gly Thr Asn Ile Lys Leu Gly Val
                485                 490                 495

Ile Glu Asp His Ser Asn Arg Thr Arg Leu Ala Lys Leu Leu Arg Phe
            500                 505                 510

Gln Ser Ser His His Pro Thr Asp Ile Thr Ser Leu Asp Gln Tyr Val
        515                 520                 525

Glu Arg Met Lys Glu Lys Gln Asp Lys Ile Tyr Phe Met Ala Gly Ser
    530                 535                 540

Ser Arg Lys Glu Ala Glu Ser Ser Pro Phe Val Glu Arg Leu Leu Lys
545                 550                 555                 560

Lys Gly Tyr Glu Val Ile Tyr Leu Thr Glu Pro Val Asp Glu Tyr Cys
                565                 570                 575

Ile Gln Ala Leu Pro Glu Phe Asp Gly Lys Arg Phe Gln Asn Val Ala
            580                 585                 590

Lys Glu Gly Val Lys Phe Asp Glu Ser Glu Lys Thr Lys Glu Ser Arg
        595                 600                 605

Glu Ala Val Glu Lys Glu Phe Glu Pro Leu Leu Asn Trp Met Lys Asp
    610                 615                 620

Lys Ala Leu Lys Asp Lys Ile Glu Lys Ala Val Val Ser Gln Arg Leu
625                 630                 635                 640

Thr Glu Ser Pro Cys Ala Leu Val Ala Ser Gln Tyr Gly Trp Ser Gly
                645                 650                 655

Asn Met Glu Arg Ile Met Lys Ala Gln Ala Tyr Gln Thr Gly Lys Asp
            660                 665                 670

Ile Ser Thr Asn Tyr Tyr Ala Ser Gln Lys Lys Thr Phe Glu Ile Asn
        675                 680                 685

Pro Arg His Pro Leu Ile Arg Asp Met Leu Arg Arg Ile Lys Glu Asp
    690                 695                 700

Glu Asp Asp Lys Thr Val Leu Asp Leu Ala Val Val Leu Phe Glu Thr
705                 710                 715                 720

Ala Thr Leu Arg Ser Gly Tyr Leu Leu Pro Asp Thr Lys Ala Tyr Gly
                725                 730                 735

Asp Arg Ile Glu Arg Met Leu Arg Leu Ser Leu Asn Ile Asp Pro Asp
            740                 745                 750

Ala Lys Val Glu Glu Glu Pro Glu Glu Glu Pro Glu Glu Thr Ala Glu
        755                 760                 765

Asp Thr Thr Glu Asp Thr Glu Gln Asp Glu Asp Glu Glu Met Asp Val
    770                 775                 780

Gly Thr Asp Glu Glu Glu Glu Thr Ala Lys Glu Ser Thr Ala Glu Lys
785                 790                 795                 800

Asp Glu Leu

<210> 11
<211> 724
<212> PRT
<213> Homo sapiens

<400> 11

Met Pro Glu Glu Val His His Gly Glu Glu Glu Val Glu Thr Phe Ala
1               5                   10                  15

Phe Gln Ala Glu Ile Ala Gln Leu Met Ser Leu Ile Ile Asn Thr Phe
            20                  25                  30

Tyr Ser Asn Lys Glu Ile Phe Leu Arg Glu Leu Ile Ser Asn Ala Ser
        35                  40                  45

Asp Ala Leu Asp Lys Ile Arg Tyr Glu Ser Leu Thr Asp Pro Ser Lys
    50                  55                  60

Leu Asp Ser Gly Lys Glu Leu Lys Ile Asp Ile Ile Pro Asn Pro Gln
65                  70                  75                  80

Glu Arg Thr Leu Thr Leu Val Asp Thr Gly Ile Gly Met Thr Lys Ala
                85                  90                  95

Asp Leu Ile Asn Asn Leu Gly Thr Ile Ala Lys Ser Gly Thr Lys Ala
            100                 105                 110

Phe Met Glu Ala Leu Gln Ala Gly Ala Asp Ile Ser Met Ile Gly Gln
        115                 120                 125

Phe Gly Val Gly Phe Tyr Ser Ala Tyr Leu Val Ala Glu Lys Val Val
    130                 135                 140

Val Ile Thr Lys His Asn Asp Asp Glu Gln Tyr Ala Trp Glu Ser Ser
145                 150                 155                 160

Ala Gly Gly Ser Phe Thr Val Arg Ala Asp His Gly Glu Pro Ile Gly
                165                 170                 175

Arg Gly Thr Lys Val Ile Leu His Leu Lys Glu Asp Gln Thr Glu Tyr
            180                 185                 190

Leu Glu Glu Arg Arg Val Lys Glu Val Val Lys Lys His Ser Gln Phe
        195                 200                 205

Ile Gly Tyr Pro Ile Thr Leu Tyr Leu Glu Lys Glu Arg Glu Lys Glu
    210                 215                 220

Ile Ser Asp Asp Glu Ala Glu Glu Glu Lys Gly Glu Lys Glu Glu Glu
225                 230                 235                 240

Asp Lys Asp Asp Glu Glu Lys Pro Lys Ile Glu Asp Val Gly Ser Asp
                245                 250                 255

Glu Glu Asp Asp Ser Gly Lys Asp Lys Lys Lys Lys Thr Lys Lys Ile
            260                 265                 270

Lys Glu Lys Tyr Ile Asp Gln Glu Glu Leu Asn Lys Thr Lys Pro Ile
        275                 280                 285

Trp Thr Arg Asn Pro Asp Asp Ile Thr Gln Glu Glu Tyr Gly Glu Phe
    290                 295                 300

Tyr Lys Ser Leu Thr Asn Asp Trp Glu Asp His Leu Ala Val Lys His
305                 310                 315                 320

Phe Ser Val Glu Gly Gln Leu Glu Phe Arg Ala Leu Leu Phe Ile Pro
                325                 330                 335

Arg Arg Ala Pro Phe Asp Leu Phe Glu Asn Lys Lys Lys Lys Asn Asn
            340                 345                 350

Ile Lys Leu Tyr Val Arg Arg Val Phe Ile Met Asp Ser Cys Asp Glu
        355                 360                 365

Leu Ile Pro Glu Tyr Leu Asn Phe Ile Arg Gly Val Val Asp Ser Glu
    370                 375                 380

Asp Leu Pro Leu Asn Ile Ser Arg Glu Met Leu Gln Gln Ser Lys Ile
385                 390                 395                 400

Leu Lys Val Ile Arg Lys Asn Ile Val Lys Lys Cys Leu Glu Leu Phe
                405                 410                 415

Ser Glu Leu Ala Glu Asp Lys Glu Asn Tyr Lys Lys Phe Tyr Glu Ala
            420                 425                 430

Phe Ser Lys Asn Leu Lys Leu Gly Ile His Glu Asp Ser Thr Asn Arg
        435                 440                 445

Arg Arg Leu Ser Glu Leu Leu Arg Tyr His Thr Ser Gln Ser Gly Asp
    450                 455                 460

Glu Met Thr Ser Leu Ser Glu Tyr Val Ser Arg Met Lys Glu Thr Gln
465                 470                 475                 480

Lys Ser Ile Tyr Tyr Ile Thr Gly Glu Ser Lys Glu Gln Val Ala Asn
                485                 490                 495

Ser Ala Phe Val Glu Arg Val Arg Lys Arg Gly Phe Glu Val Val Tyr
            500                 505                 510

Met Thr Glu Pro Ile Asp Glu Tyr Cys Val Gln Gln Leu Lys Glu Phe
        515                 520                 525

Asp Gly Lys Ser Leu Val Ser Val Thr Lys Glu Gly Leu Glu Leu Pro
    530                 535                 540

Glu Asp Glu Glu Glu Lys Lys Lys Met Glu Glu Ser Lys Ala Lys Phe
545                 550                 555                 560

Glu Asn Leu Cys Lys Leu Met Lys Glu Ile Leu Asp Lys Lys Val Glu
                565                 570                 575

Lys Val Thr Ile Ser Asn Arg Leu Val Ser Ser Pro Cys Cys Ile Val
            580                 585                 590

Thr Ser Thr Tyr Gly Trp Thr Ala Asn Met Glu Arg Ile Met Lys Ala
        595                 600                 605

Gln Ala Leu Arg Asp Asn Ser Thr Met Gly Tyr Met Met Ala Lys Lys
    610                 615                 620

His Leu Glu Ile Asn Pro Asp His Pro Ile Val Glu Thr Leu Arg Gln
625                 630                 635                 640

Lys Ala Glu Ala Asp Lys Asn Asp Lys Ala Val Lys Asp Leu Val Val
                645                 650                 655

Leu Leu Phe Glu Thr Ala Leu Leu Ser Ser Gly Phe Ser Leu Glu Asp
            660                 665                 670

Pro Gln Thr His Ser Asn Arg Ile Tyr Arg Met Ile Lys Leu Gly Leu
        675                 680                 685

Gly Ile Asp Glu Asp Glu Val Ala Ala Glu Glu Pro Asn Ala Ala Val
    690                 695                 700

Pro Asp Glu Ile Pro Pro Leu Glu Gly Asp Glu Asp Ala Ser Arg Met
705                 710                 715                 720

Glu Glu Val Asp

<210>  12
<211>  641
<212>  PRT
<213>  Homo sapiens

<400>  12

Met Ala Lys Ala Ala Ala Ile Gly Ile Asp Leu Gly Thr Thr Tyr Ser
1               5                   10                  15

Cys Val Gly Val Phe Gln His Gly Lys Val Glu Ile Ile Ala Asn Asp
            20                  25                  30

Gln Gly Asn Arg Thr Thr Pro Ser Tyr Val Ala Phe Thr Asp Thr Glu
        35                  40                  45

Arg Leu Ile Gly Asp Ala Ala Lys Asn Gln Val Ala Leu Asn Pro Gln
    50                  55                  60

Asn Thr Val Phe Asp Ala Lys Arg Leu Ile Gly Arg Lys Phe Gly Asp
65                  70                  75                  80

Pro Val Val Gln Ser Asp Met Lys His Trp Pro Phe Gln Val Ile Asn
                85                  90                  95

Asp Gly Asp Lys Pro Lys Val Gln Val Ser Tyr Lys Gly Glu Thr Lys
            100                 105                 110

Ala Phe Tyr Pro Glu Glu Ile Ser Ser Met Val Leu Thr Lys Met Lys
        115                 120                 125

Glu Ile Ala Glu Ala Tyr Leu Gly Tyr Pro Val Thr Asn Ala Val Ile
    130                 135                 140

Thr Val Pro Ala Tyr Phe Asn Asp Ser Gln Arg Gln Ala Thr Lys Asp
145                 150                 155                 160

Ala Gly Val Ile Ala Gly Leu Asn Val Leu Arg Ile Ile Asn Glu Pro
                165                 170                 175

Thr Ala Ala Ala Ile Ala Tyr Gly Leu Asp Arg Thr Gly Lys Gly Glu
            180                 185                 190

Arg Asn Val Leu Ile Phe Asp Leu Gly Gly Gly Thr Phe Asp Val Ser
        195                 200                 205

Ile Leu Thr Ile Asp Asp Gly Ile Phe Glu Val Lys Ala Thr Ala Gly
    210                 215                 220

Asp Thr His Leu Gly Gly Glu Asp Phe Asp Asn Arg Leu Val Asn His
225                 230                 235                 240

Phe Val Glu Glu Phe Lys Arg Lys His Lys Lys Asp Ile Ser Gln Asn
                245                 250                 255

Lys Arg Ala Val Arg Arg Leu Arg Thr Ala Cys Glu Arg Ala Lys Arg
            260                 265                 270

Thr Leu Ser Ser Ser Thr Gln Ala Ser Leu Glu Ile Asp Ser Leu Phe
        275                 280                 285

Glu Gly Ile Asp Phe Tyr Thr Ser Ile Thr Arg Ala Arg Phe Glu Glu
    290                 295                 300

Leu Cys Ser Asp Leu Phe Arg Ser Thr Leu Glu Pro Val Glu Lys Ala

305                 310                 315                 320

Leu Arg Asp Ala Lys Leu Asp Lys Ala Gln Ile His Asp Leu Val Leu
                325                 330                 335

Val Gly Gly Ser Thr Arg Ile Pro Lys Val Gln Lys Leu Leu Gln Asp
            340                 345                 350

Phe Phe Asn Gly Arg Asp Leu Asn Lys Ser Ile Asn Pro Asp Glu Ala
        355                 360                 365

Val Ala Tyr Gly Ala Ala Val Gln Ala Ala Ile Leu Met Gly Asp Lys
    370                 375                 380

Ser Glu Asn Val Gln Asp Leu Leu Leu Leu Asp Val Ala Pro Leu Ser
385                 390                 395                 400

Leu Gly Leu Glu Thr Ala Gly Gly Val Met Thr Ala Leu Ile Lys Arg
                405                 410                 415

Asn Ser Thr Ile Pro Thr Lys Gln Thr Gln Ile Phe Thr Thr Tyr Ser
            420                 425                 430

Asp Asn Gln Pro Gly Val Leu Ile Gln Val Tyr Glu Gly Glu Arg Ala
        435                 440                 445

Met Thr Lys Asp Asn Asn Leu Leu Gly Arg Phe Glu Leu Ser Gly Ile
    450                 455                 460

Pro Pro Ala Pro Arg Gly Val Pro Gln Ile Glu Val Thr Phe Asp Ile
465                 470                 475                 480

Asp Ala Asn Gly Ile Leu Asn Val Thr Ala Thr Asp Lys Ser Thr Gly
                485                 490                 495

Lys Ala Asn Lys Ile Thr Ile Thr Asn Asp Lys Gly Arg Leu Ser Lys
            500                 505                 510

Glu Glu Ile Glu Arg Met Val Gln Glu Ala Glu Lys Tyr Lys Ala Glu
        515                 520                 525

Asp Glu Val Gln Arg Glu Arg Val Ser Ala Lys Asn Ala Leu Glu Ser
    530                 535                 540

Tyr Ala Phe Asn Met Lys Ser Ala Val Glu Asp Glu Gly Leu Lys Gly
545                 550                 555                 560

Lys Ile Ser Glu Ala Asp Lys Lys Lys Val Leu Asp Lys Cys Gln Glu
                565                 570                 575

Val Ile Ser Trp Leu Asp Ala Asn Thr Leu Ala Glu Lys Asp Glu Phe
            580                 585                 590

Glu His Lys Arg Lys Glu Leu Glu Gln Val Cys Asn Pro Ile Ile Ser
        595                 600                 605

Gly Leu Tyr Gln Gly Ala Gly Gly Pro Gly Pro Gly Gly Phe Gly Ala
    610                 615                 620

Gln Gly Pro Lys Gly Gly Ser Gly Ser Gly Pro Thr Ile Glu Glu Val
625                 630                 635                 640

Asp

<210>  13
<211>  646
<212>  PRT
<213>  Homo sapiens

<400>  13

Met Ser Lys Gly Pro Ala Val Gly Ile Asp Leu Gly Thr Thr Tyr Ser
1               5                   10                  15

Cys Val Gly Val Phe Gln His Gly Lys Val Glu Ile Ile Ala Asn Asp
            20                  25                  30

Gln Gly Asn Arg Thr Thr Pro Ser Tyr Val Ala Phe Thr Asp Thr Glu
        35                  40                  45

Arg Leu Ile Gly Asp Ala Ala Lys Asn Gln Val Ala Met Asn Pro Thr
    50                  55                  60

Asn Thr Val Phe Asp Ala Lys Arg Leu Ile Gly Arg Arg Phe Asp Asp
65                  70                  75                  80

Ala Val Val Gln Ser Asp Met Lys His Trp Pro Phe Met Val Val Asn
                85                  90                  95

Asp Ala Gly Arg Pro Lys Val Gln Val Glu Tyr Lys Gly Glu Thr Lys
            100                 105                 110

Ser Phe Tyr Pro Glu Glu Val Ser Ser Met Val Leu Thr Lys Met Lys
        115                 120                 125

Glu Ile Ala Glu Ala Tyr Leu Gly Lys Thr Val Thr Asn Ala Val Val
    130                 135                 140

Thr Val Pro Ala Tyr Phe Asn Asp Ser Gln Arg Gln Ala Thr Lys Asp
145                 150                 155                 160

Ala Gly Thr Ile Ala Gly Leu Asn Val Leu Arg Ile Ile Asn Glu Pro
                165                 170                 175

Thr Ala Ala Ala Ile Ala Tyr Gly Leu Asp Lys Lys Val Gly Ala Glu
            180                 185                 190

Arg Asn Val Leu Ile Phe Asp Leu Gly Gly Gly Thr Phe Asp Val Ser
        195                 200                 205

Ile Leu Thr Ile Glu Asp Gly Ile Phe Glu Val Lys Ser Thr Ala Gly
    210                 215                 220

Asp Thr His Leu Gly Gly Glu Asp Phe Asp Asn Arg Met Val Asn His
225                 230                 235                 240

Phe Ile Ala Glu Phe Lys Arg Lys His Lys Lys Asp Ile Ser Glu Asn
                245                 250                 255

Lys Arg Ala Val Arg Arg Leu Arg Thr Ala Cys Glu Arg Ala Lys Arg
            260                 265                 270

Thr Leu Ser Ser Ser Thr Gln Ala Ser Ile Glu Ile Asp Ser Leu Tyr
        275                 280                 285

Glu Gly Ile Asp Phe Tyr Thr Ser Ile Thr Arg Ala Arg Phe Glu Glu
    290                 295                 300

Leu Asn Ala Asp Leu Phe Arg Gly Thr Leu Asp Pro Val Glu Lys Ala
305                 310                 315                 320

Leu Arg Asp Ala Lys Leu Asp Lys Ser Gln Ile His Asp Ile Val Leu
                325                 330                 335

Val Gly Gly Ser Thr Arg Ile Pro Lys Ile Gln Lys Leu Leu Gln Asp
            340                 345                 350

Phe Phe Asn Gly Lys Glu Leu Asn Lys Ser Ile Asn Pro Asp Glu Ala
        355                 360                 365

Val Ala Tyr Gly Ala Ala Val Gln Ala Ala Ile Leu Ser Gly Asp Lys
    370                 375                 380

Ser Glu Asn Val Gln Asp Leu Leu Leu Leu Asp Val Thr Pro Leu Ser
385                 390                 395                 400

Leu Gly Ile Glu Thr Ala Gly Gly Val Met Thr Val Leu Ile Lys Arg
                405                 410                 415

Asn Thr Thr Ile Pro Thr Lys Gln Thr Gln Thr Phe Thr Thr Tyr Ser
            420                 425                 430

Asp Asn Gln Pro Gly Val Leu Ile Gln Val Tyr Glu Gly Glu Arg Ala
        435                 440                 445

Met Thr Lys Asp Asn Asn Leu Leu Gly Lys Phe Glu Leu Thr Gly Ile
    450                 455                 460

Pro Pro Ala Pro Arg Gly Val Pro Gln Ile Glu Val Thr Phe Asp Ile
465                 470                 475                 480

Asp Ala Asn Gly Ile Leu Asn Val Ser Ala Val Asp Lys Ser Thr Gly
                485                 490                 495

Lys Glu Asn Lys Ile Thr Ile Thr Asn Asp Lys Gly Arg Leu Ser Lys
            500                 505                 510

Glu Asp Ile Glu Arg Met Val Gln Glu Ala Glu Lys Tyr Lys Ala Glu
        515                 520                 525

Asp Glu Lys Gln Arg Asp Lys Val Ser Ser Lys Asn Ser Leu Glu Ser
    530                 535                 540

Tyr Ala Phe Asn Met Lys Ala Thr Val Glu Asp Glu Lys Leu Gln Gly
545                 550                 555                 560

Lys Ile Asn Asp Glu Asp Lys Gln Lys Ile Leu Asp Lys Cys Asn Glu
                565                 570                 575

Ile Ile Asn Trp Leu Asp Lys Asn Gln Thr Ala Glu Lys Glu Glu Phe
            580                 585                 590

Glu His Gln Gln Lys Glu Leu Glu Lys Val Cys Asn Pro Ile Ile Thr
        595                 600                 605

Lys Leu Tyr Gln Ser Ala Gly Gly Met Pro Gly Gly Met Pro Gly Gly
    610                 615                 620

Phe Pro Gly Gly Gly Ala Pro Pro Ser Gly Gly Ala Ser Ser Gly Pro
625                 630                 635                 640

Thr Ile Glu Glu Val Asp
                645

<210>  14
<211>  679
<212>  PRT
<213>  Homo sapiens

<400>  14

<210>  15
<211>  108
<212>  PRT
<213>  Homo sapiens

<400>  15

Met Gly Val Gln Val Glu Thr Ile Ser Pro Gly Asp Gly Arg Thr Phe
1               5                   10                  15

Pro Lys Arg Gly Gln Thr Cys Val Val His Tyr Thr Gly Met Leu Glu
            20                  25                  30

Asp Gly Lys Lys Phe Asp Ser Ser Arg Asp Arg Asn Lys Pro Phe Lys
        35                  40                  45

Phe Met Leu Gly Lys Gln Glu Val Ile Arg Gly Trp Glu Glu Gly Val
    50                  55                  60

Ala Gln Met Ser Val Gly Gln Arg Ala Lys Leu Thr Ile Ser Pro Asp
65                  70                  75                  80

Tyr Ala Tyr Gly Ala Thr Gly His Pro Gly Ile Ile Pro Pro His Ala
                85                  90                  95

Thr Leu Val Phe Asp Val Glu Leu Leu Lys Leu Glu
            100                 105


<210>  16
<211>  459
<212>  PRT
<213>  Homo sapiens

<400>  16

Met Thr Ala Glu Glu Met Lys Ala Thr Glu Ser Gly Ala Gln Ser Ala
1               5                   10                  15

Pro Leu Pro Met Glu Gly Val Asp Ile Ser Pro Lys Gln Asp Glu Gly
            20                  25                  30

Val Leu Lys Val Ile Lys Arg Glu Gly Thr Gly Thr Glu Met Pro Met
        35                  40                  45

Ile Gly Asp Arg Val Phe Val His Tyr Thr Gly Trp Leu Leu Asp Gly
    50                  55                  60

Thr Lys Phe Asp Ser Ser Leu Asp Arg Lys Asp Lys Phe Ser Phe Asp
65                  70                  75                  80

Leu Gly Lys Gly Glu Val Ile Lys Ala Trp Asp Ile Ala Ile Ala Thr
                85                  90                  95

Met Lys Val Gly Glu Val Cys His Ile Thr Cys Lys Pro Glu Tyr Ala

            100                 105                 110

Tyr Gly Ser Ala Gly Ser Pro Pro Lys Ile Pro Pro Asn Ala Thr Leu
        115                 120                 125

Val Phe Glu Val Glu Leu Phe Glu Phe Lys Gly Glu Asp Leu Thr Glu
    130                 135                 140

Glu Glu Asp Gly Gly Ile Ile Arg Arg Ile Gln Thr Arg Gly Glu Gly
145                 150                 155                 160

Tyr Ala Lys Pro Asn Glu Gly Ala Ile Val Glu Val Ala Leu Glu Gly
                165                 170                 175

Tyr Tyr Lys Asp Lys Leu Phe Asp Gln Arg Glu Leu Arg Phe Glu Ile
            180                 185                 190

Gly Glu Gly Glu Asn Leu Asp Leu Pro Tyr Gly Leu Glu Arg Ala Ile
        195                 200                 205

Gln Arg Met Glu Lys Gly Glu His Ser Ile Val Tyr Leu Lys Pro Ser
    210                 215                 220

Tyr Ala Phe Gly Ser Val Gly Lys Glu Lys Phe Gln Ile Pro Pro Asn
225                 230                 235                 240

Ala Glu Leu Lys Tyr Glu Leu His Leu Lys Ser Phe Glu Lys Ala Lys
                245                 250                 255

Glu Ser Trp Glu Met Asn Ser Glu Glu Lys Leu Glu Gln Ser Thr Ile
            260                 265                 270

Val Lys Glu Arg Gly Thr Val Tyr Phe Lys Glu Gly Lys Tyr Lys Gln
        275                 280                 285

Ala Leu Leu Gln Tyr Lys Lys Ile Val Ser Trp Leu Glu Tyr Glu Ser
    290                 295                 300

Ser Phe Ser Asn Glu Glu Ala Gln Lys Ala Gln Ala Leu Arg Leu Ala
305                 310                 315                 320

Ser His Leu Asn Leu Ala Met Cys His Leu Lys Leu Gln Ala Phe Ser
                325                 330                 335

Ala Ala Ile Glu Ser Cys Asn Lys Ala Leu Glu Leu Asp Ser Asn Asn
            340                 345                 350

Glu Lys Gly Leu Phe Arg Arg Gly Glu Ala His Leu Ala Val Asn Asp
        355                 360                 365

Phe Glu Leu Ala Arg Ala Asp Phe Gln Lys Val Leu Gln Leu Tyr Pro
    370                 375                 380

Asn Asn Lys Ala Ala Lys Thr Gln Leu Ala Val Cys Gln Gln Arg Ile
385                 390                 395                 400

Arg Arg Gln Leu Ala Arg Glu Lys Lys Leu Tyr Ala Asn Met Phe Glu
                405                 410                 415

Arg Leu Ala Glu Glu Glu Asn Lys Ala Lys Ala Glu Ala Ser Ser Gly
            420                 425                 430

Asp His Pro Thr Asp Thr Glu Met Lys Glu Glu Gln Lys Ser Asn Thr
        435                 440                 445

Ala Gly Ser Gln Ser Gln Val Glu Thr Glu Ala
    450                 455

<210>  17
<211>  189
<212>  PRT
<213>  Homo sapiens

<400>  17

<210>  18
<211>  163
<212>  PRT
<213>  Homo sapiens

<400>  18

Met Ala Asp Glu Glu Lys Leu Pro Pro Gly Trp Glu Lys Arg Met Ser
1               5                   10                  15

Arg Ser Ser Gly Arg Val Tyr Tyr Phe Asn His Ile Thr Asn Ala Ser
            20                  25                  30

Gln Trp Glu Arg Pro Ser Gly Asn Ser Ser Ser Gly Gly Lys Asn Gly
        35                  40                  45

Gln Gly Glu Pro Ala Arg Val Arg Cys Ser His Leu Leu Val Lys His
    50                  55                  60

Ser Gln Ser Arg Arg Pro Ser Ser Trp Arg Gln Glu Lys Ile Thr Arg
65                  70                  75                  80

Thr Lys Glu Glu Ala Leu Glu Leu Ile Asn Gly Tyr Ile Gln Lys Ile
                85                  90                  95

Lys Ser Gly Glu Glu Asp Phe Glu Ser Leu Ala Ser Gln Phe Ser Asp
            100                 105                 110

Cys Ser Ser Ala Lys Ala Arg Gly Asp Leu Gly Ala Phe Ser Arg Gly
        115                 120                 125

Gln Met Gln Lys Pro Phe Glu Asp Ala Ser Phe Ala Leu Arg Thr Gly
    130                 135                 140

Glu Met Ser Gly Pro Val Phe Thr Asp Ser Gly Ile His Ile Ile Leu
145                 150                 155                 160

Arg Thr Glu

<210>  19
<211>  165
<212>  PRT
<213>  Homo sapiens

<400>  19

Met Val Asn Pro Thr Val Phe Phe Asp Ile Ala Val Asp Gly Glu Pro
1               5                   10                  15

Leu Gly Arg Val Ser Phe Glu Leu Phe Ala Asp Lys Val Pro Lys Thr
            20                  25                  30

Ala Glu Asn Phe Arg Ala Leu Ser Thr Gly Glu Lys Gly Phe Gly Tyr
        35                  40                  45

Lys Gly Ser Cys Phe His Arg Ile Ile Pro Gly Phe Met Cys Gln Gly
    50                  55                  60

Gly Asp Phe Thr Arg His Asn Gly Thr Gly Gly Lys Ser Ile Tyr Gly
65                  70                  75                  80

Glu Lys Phe Glu Asp Glu Asn Phe Ile Leu Lys His Thr Gly Pro Gly
                85                  90                  95

Ile Leu Ser Met Ala Asn Ala Gly Pro Asn Thr Asn Gly Ser Gln Phe
            100                 105                 110

Phe Ile Cys Thr Ala Lys Thr Glu Trp Leu Asp Gly Lys His Val Val
        115                 120                 125

Phe Gly Lys Val Lys Glu Gly Met Asn Ile Val Glu Ala Met Glu Arg
    130                 135                 140

Phe Gly Ser Arg Asn Gly Lys Thr Ser Lys Lys Ile Thr Ile Ala Asp
145                 150                 155                 160

Cys Gly Gln Leu Glu
                165

<210>  20
<211>  370
<212>  PRT
<213>  Homo sapiens

<400> 20

Met Ser His Pro Ser Pro Gln Ala Lys Pro Ser Asn Pro Ser Asn Pro
1               5                   10                  15

Arg Val Phe Phe Asp Val Asp Ile Gly Gly Glu Arg Val Gly Arg Ile
            20                  25                  30

Val Leu Glu Leu Phe Ala Asp Ile Val Pro Lys Thr Ala Glu Asn Phe
        35                  40                  45

Arg Ala Leu Cys Thr Gly Glu Lys Gly Ile Gly His Thr Thr Gly Lys
    50                  55                  60

Pro Leu His Phe Lys Gly Cys Pro Phe His Arg Ile Ile Lys Lys Phe
65                  70                  75                  80

Met Ile Gln Gly Gly Asp Phe Ser Asn Gln Asn Gly Thr Gly Gly Glu
                85                  90                  95

Ser Ile Tyr Gly Glu Lys Phe Glu Asp Glu Asn Phe His Tyr Lys His
            100                 105                 110

Asp Arg Glu Gly Leu Leu Ser Met Ala Asn Ala Gly Arg Asn Thr Asn
        115                 120                 125

Gly Ser Gln Phe Phe Ile Thr Thr Val Pro Thr Pro His Leu Asp Gly
    130                 135                 140

Lys His Val Val Phe Gly Gln Val Ile Lys Gly Ile Gly Val Ala Arg
145                 150                 155                 160

Ile Leu Glu Asn Val Glu Val Lys Gly Glu Lys Pro Ala Lys Leu Cys
                165                 170                 175

Val Ile Ala Glu Cys Gly Glu Leu Lys Glu Gly Asp Asp Gly Gly Ile
            180                 185                 190

Phe Pro Lys Asp Gly Ser Gly Asp Ser His Pro Asp Phe Pro Glu Asp
        195                 200                 205

Ala Asp Ile Asp Leu Lys Asp Val Asp Lys Ile Leu Leu Ile Thr Glu
    210                 215                 220

Asp Leu Lys Asn Ile Gly Asn Thr Phe Phe Lys Ser Gln Asn Trp Glu
225                 230                 235                 240

Met Ala Ile Lys Lys Tyr Ala Glu Val Leu Arg Tyr Val Asp Ser Ser
                245                 250                 255

Lys Ala Val Ile Glu Thr Ala Asp Arg Ala Lys Leu Gln Pro Ile Ala
            260                 265                 270

Leu Ser Cys Val Leu Asn Ile Gly Ala Cys Lys Leu Lys Met Ser Asn
        275                 280                 285

Trp Gln Gly Ala Ile Asp Ser Cys Leu Glu Ala Leu Glu Leu Asp Pro
    290                 295                 300

Ser Asn Thr Lys Ala Leu Tyr Arg Arg Ala Gln Gly Trp Gln Gly Leu
305                 310                 315                 320

Lys Glu Tyr Asp Gln Ala Leu Ala Asp Leu Lys Lys Ala Gln Gly Ile
                325                 330                 335

Ala Pro Glu Asp Lys Ala Ile Gln Ala Glu Leu Leu Lys Val Lys Gln
            340                 345                 350

Lys Ile Lys Ala Gln Lys Asp Lys Glu Lys Ala Val Tyr Ala Lys Met
        355                 360                 365

Phe Ala
    370

<210>  21
<211>  247
<212>  PRT
<213>  Homo sapiens

<400>  21

<210>  22
<211>  255
<212>  PRT
<213>  Homo sapiens

<400>  22

Met Asp Asp Arg Glu Asp Leu Val Tyr Gln Ala Lys Leu Ala Glu Gln
1               5                   10                  15

Ala Glu Arg Tyr Asp Glu Met Val Glu Ser Met Lys Lys Val Ala Gly
            20                  25                  30

Met Asp Val Glu Leu Thr Val Glu Glu Arg Asn Leu Leu Ser Val Ala
        35                  40                  45

Tyr Lys Asn Val Ile Gly Ala Arg Arg Ala Ser Trp Arg Ile Ile Ser
    50                  55                  60

Ser Ile Glu Gln Lys Glu Glu Asn Lys Gly Gly Glu Asp Lys Leu Lys
65                  70                  75                  80

Met Ile Arg Glu Tyr Arg Gln Met Val Glu Thr Glu Leu Lys Leu Ile
                85                  90                  95

Cys Cys Asp Ile Leu Asp Val Leu Asp Lys His Leu Ile Pro Ala Ala
            100                 105                 110

Asn Thr Gly Glu Ser Lys Val Phe Tyr Tyr Lys Met Lys Gly Asp Tyr
        115                 120                 125

His Arg Tyr Leu Ala Glu Phe Ala Thr Gly Asn Asp Arg Lys Glu Ala
    130                 135                 140

Ala Glu Asn Ser Leu Val Ala Tyr Lys Ala Ala Ser Asp Ile Ala Met
145                 150                 155                 160

Thr Glu Leu Pro Pro Thr His Pro Ile Arg Leu Gly Leu Ala Leu Asn
                165                 170                 175

Phe Ser Val Phe Tyr Tyr Glu Ile Leu Asn Ser Pro Asp Arg Ala Cys
            180                 185                 190

Arg Leu Ala Lys Ala Ala Phe Asp Asp Ala Ile Ala Glu Leu Asp Thr
        195                 200                 205

Leu Ser Glu Glu Ser Tyr Lys Asp Ser Thr Leu Ile Met Gln Leu Leu
    210                 215                 220

Arg Asp Asn Leu Thr Leu Trp Thr Ser Asp Met Gln Gly Asp Gly Glu
225                 230                 235                 240

Glu Gln Asn Lys Glu Ala Leu Gln Asp Val Glu Asp Glu Asn Gln
                245                 250                 255

<210>  23
<211>  245
<212>  PRT
<213>  Homo sapiens

<400>  23

Met Glu Lys Thr Glu Leu Ile Gln Lys Ala Lys Leu Ala Glu Gln Ala
1               5                   10                  15

Glu Arg Tyr Asp Asp Met Ala Thr Cys Met Lys Ala Val Thr Glu Gln
            20                  25                  30

Gly Ala Glu Leu Ser Asn Glu Glu Arg Asn Leu Leu Ser Val Ala Tyr
        35                  40                  45

Lys Asn Val Val Gly Gly Arg Arg Ser Ala Trp Arg Val Ile Ser Ser
    50                  55                  60

Ile Glu Gln Lys Thr Asp Thr Ser Asp Lys Lys Leu Gln Leu Ile Lys
65                  70                  75                  80

Asp Tyr Arg Glu Lys Val Glu Ser Glu Leu Arg Ser Ile Cys Thr Thr
                85                  90                  95

Val Leu Glu Leu Leu Asp Lys Tyr Leu Ile Ala Asn Ala Thr Asn Pro
            100                 105                 110

Glu Ser Lys Val Phe Tyr Leu Lys Met Lys Gly Asp Tyr Phe Arg Tyr
        115                 120                 125

Leu Ala Glu Val Ala Cys Gly Asp Asp Arg Lys Gln Thr Ile Asp Asn
    130                 135                 140

Ser Gln Gly Ala Tyr Gln Glu Ala Phe Asp Ile Ser Lys Lys Glu Met
145                 150                 155                 160

Gln Pro Thr His Pro Ile Arg Leu Gly Leu Ala Leu Asn Phe Ser Val
                165                 170                 175

Phe Tyr Tyr Glu Ile Leu Asn Asn Pro Glu Leu Ala Cys Thr Leu Ala
            180                 185                 190

Lys Thr Ala Phe Asp Glu Ala Ile Ala Glu Leu Asp Thr Leu Asn Glu
        195                 200                 205

Asp Ser Tyr Lys Asp Ser Thr Leu Ile Met Gln Leu Leu Arg Asp Asn
    210                 215                 220

Leu Thr Leu Trp Thr Ser Asp Ser Ala Gly Glu Glu Cys Asp Ala Ala
225                 230                 235                 240

Glu Gly Ala Glu Asn
                245

<210>  24
<211>  205
<212>  PRT

<213>  Homo sapiens

<400>  24

<210>  25
<211>  216
<212>  PRT
<213>  Homo sapiens

<400>  25

<210>  26
<211>  340
<212>  PRT
<213>  Homo sapiens

<400>  26

Met Gly Lys Asp Tyr Tyr Gln Thr Leu Gly Leu Ala Arg Gly Ala Ser
1               5                   10                  15

Asp Glu Glu Ile Lys Arg Ala Tyr Arg Arg Gln Ala Leu Arg Tyr His
            20                  25                  30

Pro Asp Lys Asn Lys Glu Pro Gly Ala Glu Glu Lys Phe Lys Glu Ile
        35                  40                  45

Ala Glu Ala Tyr Asp Val Leu Ser Asp Pro Arg Lys Arg Glu Ile Phe
    50                  55                  60

Asp Arg Tyr Gly Glu Glu Gly Leu Lys Gly Ser Gly Pro Ser Gly Gly
65                  70                  75                  80

Ser Gly Gly Gly Ala Asn Gly Thr Ser Phe Ser Tyr Thr Phe His Gly
                85                  90                  95

Asp Pro His Ala Met Phe Ala Glu Phe Phe Gly Gly Arg Asn Pro Phe
            100                 105                 110

Asp Thr Phe Phe Gly Gln Arg Asn Gly Glu Glu Gly Met Asp Ile Asp
        115                 120                 125

Asp Pro Phe Ser Gly Phe Pro Met Gly Met Gly Gly Phe Thr Asn Val
    130                 135                 140

Asn Phe Gly Arg Ser Arg Ser Ala Gln Glu Pro Ala Arg Lys Lys Gln
145                 150                 155                 160

Asp Pro Pro Val Thr His Asp Leu Arg Val Ser Leu Glu Glu Ile Tyr
                165                 170                 175

Ser Gly Cys Thr Lys Lys Met Lys Ile Ser His Lys Arg Leu Asn Pro
            180                 185                 190

Asp Gly Lys Ser Ile Arg Asn Glu Asp Lys Ile Leu Thr Ile Glu Val
        195                 200                 205

Lys Lys Gly Trp Lys Glu Gly Thr Lys Ile Thr Phe Pro Lys Glu Gly
    210                 215                 220

Asp Gln Thr Ser Asn Asn Ile Pro Ala Asp Ile Val Phe Val Leu Lys
225                 230                 235                 240

Asp Lys Pro His Asn Ile Phe Lys Arg Asp Gly Ser Asp Val Ile Tyr
                245                 250                 255

Pro Ala Arg Ile Ser Leu Arg Glu Ala Leu Cys Gly Cys Thr Val Asn
            260                 265                 270

Val Pro Thr Leu Asp Gly Arg Thr Ile Pro Val Val Phe Lys Asp Val
        275                 280                 285

Ile Arg Pro Gly Met Arg Arg Lys Val Pro Gly Glu Gly Leu Pro Leu
    290                 295                 300

Pro Lys Thr Pro Glu Lys Arg Gly Asp Leu Ile Ile Glu Phe Glu Val
305                 310                 315                 320

Ile Phe Pro Glu Arg Ile Pro Gln Thr Ser Arg Thr Val Leu Glu Gln
                325                 330                 335

Val Leu Pro Ile
            340

<210>  27
<211>  198
<212>  PRT
<213>  Homo sapiens

<400>  27

<210>  28
<211>  449
<212>  PRT
<213>  Homo sapiens

<400>  28

Met Met Lys Thr Leu Leu Leu Phe Val Gly Leu Leu Leu Thr Trp Glu
1               5                   10                  15

Ser Gly Gln Val Leu Gly Asp Gln Thr Val Ser Asp Asn Glu Leu Gln
            20                  25                  30

Glu Met Ser Asn Gln Gly Ser Lys Tyr Val Asn Lys Glu Ile Gln Asn
        35                  40                  45

Ala Val Asn Gly Val Lys Gln Ile Lys Thr Leu Ile Glu Lys Thr Asn
    50                  55                  60

Glu Glu Arg Lys Thr Leu Leu Ser Asn Leu Glu Glu Ala Lys Lys Lys
65                  70                  75                  80

Lys Glu Asp Ala Leu Asn Glu Thr Arg Glu Ser Glu Thr Lys Leu Lys
                85                  90                  95

Glu Leu Pro Gly Val Cys Asn Glu Thr Met Met Ala Leu Trp Glu Glu
            100                 105                 110

Cys Lys Pro Cys Leu Lys Gln Thr Cys Met Lys Phe Tyr Ala Arg Val
        115                 120                 125

Cys Arg Ser Gly Ser Gly Leu Val Gly Arg Gln Leu Glu Glu Phe Leu
    130                 135                 140

Asn Gln Ser Ser Pro Phe Tyr Phe Trp Met Asn Gly Asp Arg Ile Asp
145                 150                 155                 160

Ser Leu Leu Glu Asn Asp Arg Gln Gln Thr His Met Leu Asp Val Met
                165                 170                 175

Gln Asp His Phe Ser Arg Ala Ser Ser Ile Ile Asp Glu Leu Phe Gln
            180                 185                 190

Asp Arg Phe Phe Thr Arg Glu Pro Gln Asp Thr Tyr His Tyr Leu Pro
        195                 200                 205

Phe Ser Leu Pro His Arg Arg Pro His Phe Phe Phe Pro Lys Ser Arg
    210                 215                 220

Ile Val Arg Ser Leu Met Pro Phe Ser Pro Tyr Glu Pro Leu Asn Phe
225                 230                 235                 240

His Ala Met Phe Gln Pro Phe Leu Glu Met Ile His Glu Ala Gln Gln
                245                 250                 255

Ala Met Asp Ile His Phe His Ser Pro Ala Phe Gln His Pro Pro Thr
            260                 265                 270

Glu Phe Ile Arg Glu Gly Asp Asp Asp Arg Thr Val Cys Arg Glu Ile
        275                 280                 285

Arg His Asn Ser Thr Gly Cys Leu Arg Met Lys Asp Gln Cys Asp Lys
    290                 295                 300

Cys Arg Glu Ile Leu Ser Val Asp Cys Ser Thr Asn Asn Pro Ser Gln
305                 310                 315                 320

Ala Lys Leu Arg Arg Glu Leu Asp Glu Ser Leu Gln Val Ala Glu Arg
                325                 330                 335

Leu Thr Arg Lys Tyr Asn Glu Leu Leu Lys Ser Tyr Gln Trp Lys Met
            340                 345                 350

Leu Asn Thr Ser Ser Leu Leu Glu Gln Leu Asn Glu Gln Phe Asn Trp
        355                 360                 365

Val Ser Arg Leu Ala Asn Leu Thr Gln Gly Glu Asp Gln Tyr Tyr Leu
    370                 375                 380

Arg Val Thr Thr Val Ala Ser His Thr Ser Asp Ser Asp Val Pro Ser
385                 390                 395                 400

Gly Val Thr Glu Val Val Val Lys Leu Phe Asp Ser Asp Pro Ile Thr
                405                 410                 415

Val Thr Val Pro Val Glu Val Ser Arg Lys Asn Pro Lys Phe Met Glu
            420                 425                 430

Thr Val Ala Glu Lys Ala Leu Gln Glu Tyr Arg Lys Lys His Arg Glu
        435                 440                 445

Glu

<210>  29
<211>  999
<212>  PRT
<213>  Homo sapiens

<400>  29

Met Ala Asp Lys Val Arg Arg Gln Arg Pro Arg Arg Arg Val Cys Trp
1               5                   10                  15

Ala Leu Val Ala Val Leu Leu Ala Asp Leu Leu Ala Leu Ser Asp Thr
            20                  25                  30

Leu Ala Val Met Ser Val Asp Leu Gly Ser Glu Ser Met Lys Val Ala
        35                  40                  45

Ile Val Lys Pro Gly Val Pro Met Glu Ile Val Leu Asn Lys Glu Ser
    50                  55                  60

Arg Arg Lys Thr Pro Val Ile Val Thr Leu Lys Glu Asn Glu Arg Phe
65                  70                  75                  80

Phe Gly Asp Ser Ala Ala Ser Met Ala Ile Lys Asn Pro Lys Ala Thr
                85                  90                  95

Leu Arg Tyr Phe Gln His Leu Leu Gly Lys Gln Ala Asp Asn Pro His
            100                 105                 110

Val Ala Leu Tyr Gln Ala Arg Phe Pro Glu His Glu Leu Thr Phe Asp
        115                 120                 125

Pro Gln Arg Gln Thr Val His Phe Gln Ile Ser Ser Gln Leu Gln Phe
    130                 135                 140

Ser Pro Glu Glu Val Leu Gly Met Val Leu Asn Tyr Ser Arg Ser Leu
145                 150                 155                 160

Ala Glu Asp Phe Ala Glu Gln Pro Ile Lys Asp Ala Val Ile Thr Val
                165                 170                 175

Pro Val Phe Phe Asn Gln Ala Glu Arg Arg Ala Val Leu Gln Ala Ala
            180                 185                 190

Arg Met Ala Gly Leu Lys Val Leu Gln Leu Ile Asn Asp Asn Thr Ala
        195                 200                 205

Thr Ala Leu Ser Tyr Gly Val Phe Arg Arg Lys Asp Ile Asn Thr Thr
    210                 215                 220

Ala Gln Asn Ile Met Phe Tyr Asp Met Gly Ser Gly Ser Thr Val Cys
225                 230                 235                 240

Thr Ile Val Thr Tyr Gln Met Val Lys Thr Lys Glu Ala Gly Met Gln
                245                 250                 255

Pro Gln Leu Gln Ile Arg Gly Val Gly Phe Asp Arg Thr Leu Gly Gly
            260                 265                 270

Leu Glu Met Glu Leu Arg Leu Arg Glu Arg Leu Ala Gly Leu Phe Asn
        275                 280                 285

Glu Gln Arg Lys Gly Gln Arg Ala Lys Asp Val Arg Glu Asn Pro Arg
    290                 295                 300

Ala Met Ala Lys Leu Leu Arg Glu Ala Asn Arg Leu Lys Thr Val Leu
305                 310                 315                 320

Ser Ala Asn Ala Asp His Met Ala Gln Ile Glu Gly Leu Met Asp Asp
                325                 330                 335

Val Asp Phe Lys Ala Lys Val Thr Arg Val Glu Phe Glu Glu Leu Cys
            340                 345                 350

Ala Asp Leu Phe Glu Arg Val Pro Gly Pro Val Gln Gln Ala Leu Gln
        355                 360                 365

Ser Ala Glu Met Ser Leu Asp Glu Ile Glu Gln Val Ile Leu Val Gly
    370                 375                 380

Gly Ala Thr Arg Val Pro Arg Val Gln Glu Val Leu Leu Lys Ala Val
385                 390                 395                 400

Gly Lys Glu Glu Leu Gly Lys Asn Ile Asn Ala Asp Glu Ala Ala Ala

                405                 410                 415

Met Gly Ala Val Tyr Gln Ala Ala Ala Leu Ser Lys Ala Phe Lys Val
            420                 425                 430

Lys Pro Phe Val Val Arg Asp Ala Val Val Tyr Pro Ile Leu Val Glu
        435                 440                 445

Phe Thr Arg Glu Val Glu Glu Glu Pro Gly Ile His Ser Leu Lys His
    450                 455                 460

Asn Lys Arg Val Leu Phe Ser Arg Met Gly Pro Tyr Pro Gln Arg Lys
465                 470                 475                 480

Val Ile Thr Phe Asn Arg Tyr Ser His Asp Phe Asn Phe His Ile Asn
                485                 490                 495

Tyr Gly Asp Leu Gly Phe Leu Gly Pro Glu Asp Leu Arg Val Phe Gly
            500                 505                 510

Ser Gln Asn Leu Thr Thr Val Lys Leu Lys Gly Val Gly Asp Ser Phe
        515                 520                 525

Lys Lys Tyr Pro Asp Tyr Glu Ser Lys Gly Ile Lys Ala His Phe Asn
    530                 535                 540

Leu Asp Glu Ser Gly Val Leu Ser Leu Asp Arg Val Glu Ser Val Phe
545                 550                 555                 560

Glu Thr Leu Val Glu Asp Ser Ala Glu Glu Glu Ser Thr Leu Thr Lys
                565                 570                 575

Leu Gly Asn Thr Ile Ser Ser Leu Phe Gly Gly Gly Thr Thr Pro Asp
            580                 585                 590

Ala Lys Glu Asn Gly Thr Asp Thr Val Gln Glu Glu Glu Glu Ser Pro
        595                 600                 605

Ala Glu Gly Ser Lys Asp Glu Pro Gly Glu Gln Val Glu Leu Lys Glu
    610                 615                 620

Glu Ala Glu Ala Pro Val Glu Asp Gly Ser Gln Pro Pro Pro Pro Glu
625                 630                 635                 640

Pro Lys Gly Asp Ala Thr Pro Glu Gly Glu Lys Ala Thr Glu Lys Glu
                645                 650                 655

Asn Gly Asp Lys Ser Glu Ala Gln Lys Pro Ser Glu Lys Ala Glu Ala
        660                 665                 670

Gly Pro Glu Gly Val Ala Pro Ala Pro Glu Gly Glu Lys Lys Gln Lys
    675                 680                 685

Pro Ala Arg Lys Arg Arg Met Val Glu Glu Ile Gly Val Glu Leu Val
    690                 695                 700

Val Leu Asp Leu Pro Asp Leu Pro Glu Asp Lys Leu Ala Gln Ser Val
705                 710                 715                 720

Gln Lys Leu Gln Asp Leu Thr Leu Arg Asp Leu Glu Lys Gln Glu Arg
                725                 730                 735

Glu Lys Ala Ala Asn Ser Leu Glu Ala Phe Ile Phe Glu Thr Gln Asp
        740                 745                 750

Lys Leu Tyr Gln Pro Glu Tyr Gln Glu Val Ser Thr Glu Glu Gln Arg
    755                 760                 765

Glu Glu Ile Ser Gly Lys Leu Ser Ala Ala Ser Thr Trp Leu Glu Asp
    770                 775                 780

Glu Gly Val Gly Ala Thr Thr Val Met Leu Lys Glu Lys Leu Ala Glu
785                 790                 795                 800

Leu Arg Lys Leu Cys Gln Gly Leu Phe Phe Arg Val Glu Glu Arg Lys
                805                 810                 815

Lys Trp Pro Glu Arg Leu Ser Ala Leu Asp Asn Leu Leu Asn His Ser
        820                 825                 830

Ser Met Phe Leu Lys Gly Ala Arg Leu Ile Pro Glu Met Asp Gln Ile
    835                 840                 845

Phe Thr Glu Val Glu Met Thr Thr Leu Glu Lys Val Ile Asn Glu Thr
    850                 855                 860

Trp Ala Trp Lys Asn Ala Thr Leu Ala Glu Gln Ala Lys Leu Pro Ala
865                 870                 875                 880

Thr Glu Lys Pro Val Leu Leu Ser Lys Asp Ile Glu Ala Lys Met Met
                885                 890                 895

Ala Leu Asp Arg Glu Val Gln Tyr Leu Leu Asn Lys Ala Lys Phe Thr
        900                 905                 910

Lys Pro Arg Pro Arg Pro Lys Asp Lys Asn Gly Thr Arg Ala Glu Pro
        915                 920                 925

Pro Leu Asn Ala Ser Ala Ser Asp Gln Gly Glu Lys Val Ile Pro Pro
    930                 935                 940

Ala Gly Gln Thr Glu Asp Ala Glu Pro Ile Ser Glu Pro Glu Lys Val
945                 950                 955                 960

Glu Thr Gly Ser Glu Pro Gly Asp Thr Glu Pro Leu Glu Leu Gly Gly
                965                 970                 975

Pro Gly Ala Glu Pro Glu Gln Lys Glu Gln Ser Thr Gly Gln Lys Arg
            980                 985                 990

Pro Leu Lys Asn Asp Glu Leu
        995

<210>  30
<211>  350
<212>  PRT
<213>  Homo sapiens

<400>  30

Met Leu Leu Ser Val Pro Leu Leu Leu Gly Leu Leu Gly Leu Ala Val
1               5                   10                  15

Ala Glu Pro Ala Val Tyr Phe Lys Glu Gln Phe Leu Asp Gly Asp Gly
            20                  25                  30

Trp Thr Ser Arg Trp Ile Glu Ser Lys His Lys Ser Asp Phe Gly Lys
        35                  40                  45

Phe Val Leu Ser Ser Gly Lys Phe Tyr Gly Asp Glu Glu Lys Asp Lys
    50                  55                  60

Gly Leu Gln Thr Ser Gln Asp Ala Arg Phe Tyr Ala Leu Ser Ala Ser
65                  70                  75                  80

Phe Glu Pro Phe Ser Asn Lys Gly Gln Thr Leu Val Val Gln Phe Thr
                85                  90                  95

Val Lys His Glu Gln Asn Ile Asp Cys Gly Gly Gly Tyr Val Lys Leu
            100                 105                 110

Phe Pro Asn Ser Leu Asp Gln Thr Asp Met His Gly Asp Ser Glu Tyr
        115                 120                 125

Asn Ile Met Phe Gly Pro Asp Ile Cys Gly Pro Gly Thr Lys Lys Val
    130                 135                 140

His Val Ile Phe Asn Tyr Lys Gly Lys Asn Val Leu Ile Asn Lys Asp
145                 150                 155                 160

Ile Arg Cys Lys Asp Asp Glu Phe Thr His Leu Tyr Thr Leu Ile Val
                165                 170                 175

Arg Pro Asp Asn Thr Tyr Glu Val Lys Ile Asp Asn Ser Gln Val Glu
            180                 185                 190

Ser Gly Ser Leu Glu Asp Asp Trp Asp Phe Leu Pro Pro Lys Lys Ile
        195                 200                 205

Lys Asp Pro Asp Ala Ser Lys Pro Glu Asp Trp Asp Glu Arg Ala Lys
    210                 215                 220

Ile Asp Asp Pro Thr Asp Ser Lys Pro Glu Asp Trp Asp Lys Pro Glu
225                 230                 235                 240

His Ile Pro Asp Pro Asp Ala Lys Lys Pro Glu Asp Trp Asp Glu Glu
                245                 250                 255

Met Asp Gly Glu Trp Glu Pro Pro Val Ile Gln Asn Pro Glu Tyr Lys
            260                 265                 270

Gly Glu Trp Lys Pro Arg Gln Ile Asp Asn Pro Asp Tyr Lys Gly Thr
        275                 280                 285

Trp Ile His Pro Glu Ile Asp Asn Pro Glu Tyr Ser Pro Asp Pro Ser
    290                 295                 300

Ile Tyr Ala Tyr Asp Asn Phe Gly Val Leu Gly Leu Asp Leu Trp Gln
305                 310                 315                 320

Val Lys Ser Gly Thr Ile Phe Asp Asn Phe Leu Ile Thr Asn Asp Glu
                325                 330                 335

Ala Tyr Ala Glu Glu Phe Gly Asn Glu Thr Trp Gly Val Thr
            340                 345                 350

<210>  31
<211>  858
<212>  PRT
<213>  Homo sapiens

<400>  31

Met Ser Val Val Gly Leu Asp Val Gly Ser Gln Ser Cys Tyr Ile Ala
1               5                   10                  15

Val Ala Arg Ala Gly Gly Ile Glu Thr Ile Ala Asn Glu Phe Ser Asp
            20                  25                  30

Arg Cys Thr Pro Ser Val Ile Ser Phe Gly Ser Lys Asn Arg Thr Ile
        35                  40                  45

Gly Val Ala Ala Lys Asn Gln Gln Ile Thr His Ala Asn Asn Thr Val
    50                  55                  60

Ser Asn Phe Lys Arg Phe His Gly Arg Ala Phe Asn Asp Pro Phe Ile
65                  70                  75                  80

Gln Lys Glu Lys Glu Asn Leu Ser Tyr Asp Leu Val Pro Leu Lys Asn
                85                  90                  95

Gly Gly Val Gly Ile Lys Val Met Tyr Met Gly Glu Glu His Leu Phe
            100                 105                 110

Ser Val Glu Gln Ile Thr Ala Met Leu Leu Thr Lys Leu Lys Glu Thr
        115                 120                 125

Ala Glu Asn Ser Leu Lys Lys Pro Val Thr Asp Cys Val Ile Ser Val
    130                 135                 140

Pro Ser Phe Phe Thr Asp Ala Glu Arg Arg Ser Val Leu Asp Ala Ala
145                 150                 155                 160

Gln Ile Val Gly Leu Asn Cys Leu Arg Leu Met Asn Asp Met Thr Ala
                165                 170                 175

Val Ala Leu Asn Tyr Gly Ile Tyr Lys Gln Asp Leu Pro Ser Leu Asp
            180                 185                 190

Glu Lys Pro Arg Ile Val Val Phe Val Asp Met Gly His Ser Ala Phe
        195                 200                 205

Gln Val Ser Ala Cys Ala Phe Asn Lys Gly Lys Leu Lys Val Leu Gly
    210                 215                 220

Thr Ala Phe Asp Pro Phe Leu Gly Gly Lys Asn Phe Asp Glu Lys Leu
225                 230                 235                 240

Val Glu His Phe Cys Ala Glu Phe Lys Thr Lys Tyr Lys Leu Asp Ala
                245                 250                 255

Lys Ser Lys Ile Arg Ala Leu Leu Arg Leu Tyr Gln Glu Cys Glu Lys
            260                 265                 270

Leu Lys Lys Leu Met Ser Ser Asn Ser Thr Asp Leu Pro Leu Asn Ile
        275                 280                 285

Glu Cys Phe Met Asn Asp Lys Asp Val Ser Gly Lys Met Asn Arg Ser
    290                 295                 300

Gln Phe Glu Glu Leu Cys Ala Glu Leu Leu Gln Lys Ile Glu Val Pro
305                 310                 315                 320

Leu Tyr Ser Leu Leu Glu Gln Thr His Leu Lys Val Glu Asp Val Ser
                325                 330                 335

Ala Val Glu Ile Val Gly Gly Ala Thr Arg Ile Pro Ala Val Lys Glu
            340                 345                 350

Arg Ile Ala Lys Phe Phe Gly Lys Asp Ile Ser Thr Thr Leu Asn Ala
        355                 360                 365

Asp Glu Ala Val Ala Arg Gly Cys Ala Leu Gln Cys Ala Ile Leu Ser
    370                 375                 380

Pro Ala Phe Lys Val Arg Glu Phe Ser Val Thr Asp Ala Val Pro Phe
385                 390                 395                 400

Pro Ile Ser Leu Ile Trp Asn His Asp Ser Glu Asp Thr Glu Gly Val
                405                 410                 415

His Glu Val Phe Ser Arg Asn His Ala Ala Pro Phe Ser Lys Val Leu
            420                 425                 430

Thr Phe Leu Arg Arg Gly Pro Phe Glu Leu Glu Ala Phe Tyr Ser Asp
        435                 440                 445

Pro Gln Gly Val Pro Tyr Pro Glu Ala Lys Ile Gly Arg Phe Val Val
    450                 455                 460

Gln Asn Val Ser Ala Gln Lys Asp Gly Glu Lys Ser Arg Val Lys Val
465                 470                 475                 480

Lys Val Arg Val Asn Thr His Gly Ile Phe Thr Ile Ser Thr Ala Ser
                485                 490                 495

Met Val Glu Lys Val Pro Thr Glu Glu Asn Glu Met Ser Ser Glu Ala

            500                 505                 510

Asp Met Glu Cys Leu Asn Gln Arg Pro Pro Glu Asn Pro Asp Thr Asp
        515                 520                 525

Lys Asn Val Gln Gln Asp Asn Ser Glu Ala Gly Thr Gln Pro Gln Val
    530                 535                 540

Gln Thr Asp Ala Gln Gln Thr Ser Gln Ser Pro Pro Ser Pro Glu Leu
545                 550                 555                 560

Thr Ser Glu Glu Asn Lys Ile Pro Asp Ala Asp Lys Ala Asn Glu Lys
                565                 570                 575

Lys Val Asp Gln Pro Pro Glu Ala Lys Lys Pro Lys Ile Lys Val Val
            580                 585                 590

Asn Val Glu Leu Pro Ile Glu Ala Asn Leu Val Trp Gln Leu Gly Lys
        595                 600                 605

Asp Leu Leu Asn Met Tyr Ile Glu Thr Glu Gly Lys Met Ile Met Gln
    610                 615                 620

Asp Lys Leu Glu Lys Glu Arg Asn Asp Ala Lys Asn Ala Val Glu Glu
625                 630                 635                 640

Tyr Val Tyr Glu Phe Arg Asp Lys Leu Cys Gly Pro Tyr Glu Lys Phe
                645                 650                 655

Ile Cys Glu Gln Asp His Gln Asn Phe Leu Arg Leu Leu Thr Glu Thr
            660                 665                 670

Glu Asp Trp Leu Tyr Glu Glu Gly Glu Asp Gln Ala Lys Gln Ala Tyr
        675                 680                 685

Val Asp Lys Leu Glu Glu Leu Met Lys Ile Gly Thr Pro Val Lys Val
    690                 695                 700

Arg Phe Gln Glu Ala Glu Glu Arg Pro Lys Met Phe Glu Glu Leu Gly
705                 710                 715                 720

Gln Arg Leu Gln His Tyr Ala Lys Ile Ala Ala Asp Phe Arg Asn Lys
                725                 730                 735

Asp Glu Lys Tyr Asn His Ile Asp Glu Ser Glu Met Lys Lys Val Glu
            740                 745                 750

Lys Ser Val Asn Glu Val Met Glu Trp Met Asn Asn Val Met Asn Ala
        755                 760                 765

Gln Ala Lys Lys Ser Leu Asp Gln Asp Pro Val Val Arg Ala Gln Glu
    770                 775                 780

Ile Lys Thr Lys Ile Lys Glu Leu Asn Asn Thr Cys Glu Pro Val Val
785                 790                 795                 800

Thr Gln Pro Lys Pro Lys Ile Glu Ser Pro Lys Leu Glu Arg Thr Pro
                805                 810                 815

Asn Gly Pro Asn Ile Asp Lys Lys Glu Glu Asp Leu Glu Asp Lys Asn
            820                 825                 830

Asn Phe Gly Ala Glu Pro Pro His Gln Asn Gly Glu Cys Tyr Pro Asn
        835                 840                 845

Glu Lys Asn Ser Val Asn Met Asp Leu Asp
    850                 855

<210>  32
<211>  303
<212>  PRT
<213>  Homo sapiens

<400>  32

<210>  33
<211>  173
<212>  PRT
<213>  Homo sapiens

<400>  33

Met Asp Val Thr Ile Gln His Pro Trp Phe Lys Arg Thr Leu Gly Pro
1               5                   10                  15

Phe Tyr Pro Ser Arg Leu Phe Asp Gln Phe Phe Gly Glu Gly Leu Phe
            20                  25                  30

Glu Tyr Asp Leu Leu Pro Phe Leu Ser Ser Thr Ile Ser Pro Tyr Tyr
        35                  40                  45

Arg Gln Ser Leu Phe Arg Thr Val Leu Asp Ser Gly Ile Ser Glu Val
    50                  55                  60

Arg Ser Asp Arg Asp Lys Phe Val Ile Phe Leu Asp Val Lys His Phe
65                  70                  75                  80

Ser Pro Glu Asp Leu Thr Val Lys Val Gln Asp Asp Phe Val Glu Ile
                85                  90                  95

His Gly Lys His Asn Glu Arg Gln Asp Asp His Gly Tyr Ile Ser Arg
            100                 105                 110

Glu Phe His Arg Arg Tyr Arg Leu Pro Ser Asn Val Asp Gln Ser Ala
        115                 120                 125

Leu Ser Cys Ser Leu Ser Ala Asp Gly Met Leu Thr Phe Cys Gly Pro
    130                 135                 140

Lys Ile Gln Thr Gly Leu Asp Ala Thr His Ala Glu Arg Ala Ile Pro
145                 150                 155                 160

Val Ser Arg Glu Glu Lys Pro Thr Ser Ala Pro Ser Ser
                165                 170